# EUROPEAN PATENT APPLICATION

(11) **EP 3 950 025 A1**
(43) Date of publication of application: **09.02.2022**
(21) Application number: 20785100.7
(22) Date of filing: 26.03.2020
(51) Int. Cl.: A61M 5/178, A61M 5/32, C12M 1/00, C12M 1/26, C12M 3/00, A61L 31/14, A61M 37/00, A61L 27/38, A61L 27/56, A61L 27/58

(54) **CELL TRANSPLANTATION DEVICE AND CELL TRANSPLANTATION SYSTEM**

(30) Priority: 29.03.2019 JP 2019068169; 29.03.2019 JP 2019068170; 29.03.2019 JP 2019068172; 29.03.2019 JP 2019068173; 29.03.2019 JP 2019068174
(71) Applicant: Toppan Printing Co., Ltd., Tokyo 110-0016 (JP)
(72) Inventor: KODAMA Yoshihiro, Tokyo 110-0016 (JP); OKAMOTO Shinichi, Tokyo 110-0016 (JP); IMAI Keiichi, Tokyo 110-0016 (JP); KOIKE Motonori, Tokyo 110-0016 (JP)
(74) Representative: TBK
(86) International application number: PCT/JP2020/013759
(87) International publication number: WO 2020/203674

(57) **Abstract**

A cell transplantation device is used to place a graft containing cells into a target region in a living body. The cell transplantation device includes a needle extending in one direction, and a filter unit made of a fibrous member. The needle has a flow channel defined therein and an opening at the tip of the needle. The filter unit is disposed at an intermediate position in the flow channel and configured to retain a graft between the filter unit and the opening when a liquid material containing the graft enters the flow channel through the opening.

## Description

### [Technical Field]

The present disclosure relates to a device and a system used for transferring objects such as groups of cells into a living body.

### [Background Art]

Techniques have been developed for transplanting a graft which is obtained by culturing cells collected from a living body into an intradermal or subcutaneous site in a living body. For example, regeneration of hair has been performed by purifying groups of cells that contribute to formation of hair follicles, which are organs that produce hair, and transplanting the groups of cells into intradermal sites.

Hair follicles are formed by interactions between epithelial cells and mesenchymal cells. For good hair regeneration, it is desirable that the transplanted groups of cells generate hair follicles having a normal tissue structure and capable of a normal hair growth cycle. Therefore, various research and development projects have been performed for methods of producing groups of cells that can produce such hair follicles (for example, see PTLs 1 to 3).

### [Citation List]

### [Patent Literature]

PTL 1: WO2017/073625
PTL 2: WO2012/108069
PTL 3: JP 2008-29331 A

### [Summary of the Invention]

### [Technical Problem]

For smooth transplantation of a graft, it is desirable to smoothly transfer the graft stored in a container such as a culture vessel to a transplant target region in the living body. For example, in a transplantation method in which an incision is made in the skin with a scalpel, and then groups of cells in a culture vessel are picked up one by one with tweezers or the like and transferred to an intradermal site, it is required to exchange the tools used and frequently repeat operations of transferring groups of cells, which may hinder smooth transplantation.

The present disclosure has been made to provide a cell transplantation device and a cell transplantation system capable of smooth transplantation of cells.

### [Solution to Problem]

A cell transplantation device for solving the above problem is a cell transplantation device configured to place a graft containing cells into a target region in a living body, the cell transplantation device including: a needle extending in one direction, the needle having a flow channel defined therein and an opening at a tip of the needle; and a filter unit made of a fibrous member, the filter unit being disposed at an intermediate position in the flow channel and configured to retain the graft between the filter unit and the opening when a liquid material containing the graft enters the flow channel through the opening.

With this configuration, the cell transplantation device collects the graft into the needle through the opening at the tip of the needle, and, after the needle is inserted into the transplantation site, ejects the graft through the opening of the needle. According to the above device, since the grafts can be collected from the container and placed in the transplant target region continuously using a single device, smooth transplantation of cells can be performed. For further improvement in the efficiency of transplantation, there is a need for a structure of a needle capable of more smoothly collecting and ejecting grafts in such a cell transplantation device. In this regard, according to the above configuration, the graft can be retained at a position close to the opening regardless of the amount of the liquid aspirated together with the graft, the period of suction, and the like. Accordingly, even when the amount of the liquid aspirated and the period of suction increase, it is possible to prevent the amount of the liquid ejected together with the graft from excessively increasing, and to smoothly perform collection and ejection of the grafts.

A cell transplantation device for solving the above problem is a cell transplantation device configured to place a graft containing cells into a target region in a living body, the cell transplantation device including: a needle extending in one direction, the needle having a flow channel defined therein and an opening at a tip of the needle, wherein a region surrounded by a side wall of the flow channel from an intermediate position in the flow channel to the opening serves as a storage portion that stores a liquid material entering through the opening while allowing the graft contained in the liquid material to be retained in the region, and a length of the storage portion in the extending direction of the needle is larger than a maximum width of the storage portion in a direction perpendicular to the extending direction of the needle.

With this configuration, since the grafts can be collected from the container and placed in the transplant target region continuously using a single device, smooth transplantation of cells can be performed. Further, with this configuration, the graft can be stored in the storage portion in a state in which the liquid contained in the liquid material together with the graft surrounds the graft in the storage portion, and a space between the graft and the tip of the storage portion is filled with the protective liquid. Therefore, it is possible to prevent the cells from drying out during transfer to the transplant target region.

A cell transplantation device for solving the above problem is a cell transplantation device configured to place a graft containing cells into a target region in a living body, the cell transplantation device including a plurality of needles extending in a first direction, wherein the plurality of needles include the needle having a tip whose position is not aligned with that of others in the first direction, and a plurality of transplantation needles each having an opening in a tip region, the opening being provided as an inlet port through which the graft is taken into the needle and an outlet port through which the graft is ejected toward the target region.

With this configuration, since the grafts can be collected from the container and placed in the transplant target region continuously using a single device, smooth transplantation of cells can be performed. Further, since the cell transplantation device includes the plurality of transplantation needles, a plurality of grafts can be collectively transplanted. Accordingly, the time required for transplantation can be reduced, and the efficiency of transplantation can be further improved.

A cell transplantation system for solving the above problem is a cell transplantation system including: a cell transplantation device configured to place a graft containing cells into a target region in a living body, the cell transplantation device including a needle extending in one direction, the needle being configured to collect the graft through an opening formed at a tip of the needle and store the graft in the needle; and a detector that detects when the graft is held by the needle.

With this configuration, since the grafts can be collected from the container and placed in the transplant target region continuously using a single device, smooth transplantation of cells can be performed. Further, with this configuration, since the detector can detect when the graft is held by the needle, the holding state of the graft, that is, whether the graft is held or not, is easily recognized compared with a case of visual inspection, and the accuracy in the inspection of the holding state can be improved. Accordingly, it is possible to improve the efficiency in collection of the graft into the cell transplantation device, and more smoothly perform cell transplantation using the cell transplantation device.

### [Advantageous Effects of the Invention]

According to the present invention, smooth transplantation of cells can be achieved.

### [Brief Description of the Drawings]

Fig. 1 is a diagram illustrating a side structure of a cell transplantation device of a first embodiment, in which the cell transplantation device has a single needle.
Fig. 2 is a diagram illustrating a side structure of the cell transplantation device of the first embodiment, in which the cell transplantation device has a plurality of needles.
Fig. 3 is a diagram illustrating a configuration of the cell transplantation device of the first embodiment, in which the cell transplantation device has a suction-ejection unit.
Fig. 4 is a diagram illustrating a cross-sectional structure of a needle in the cell transplantation device of the first embodiment.
Fig. 5 is a diagram illustrating a planar structure and a side structure of a first example of a first tube on which a filter unit is provided in the cell transplantation device of the first embodiment.
Fig. 6 is a diagram illustrating a planar structure and a side structure of a second example of the first tube on which a filter unit is provided in the cell transplantation device of the first embodiment.
Fig. 7 is a diagram illustrating a planar structure and a side structure of a third example of the first tube on which a filter unit is provided in the cell transplantation device of the first embodiment.
Fig. 8 is a diagram illustrating a step of assembling a needle and a filter unit in the cell transplantation device of the first embodiment.
Fig. 9 is a diagram illustrating a step of assembling the needle and the filter unit in the cell transplantation device of the first embodiment.
Fig. 10 is a diagram illustrating a cross-sectional structure of the cell transplantation device of the first embodiment.
Fig. 11 is a diagram illustrating arrangement of a cell transplantation device and a tray that holds a graft according to a transplantation method using the cell transplantation device of the first embodiment.
Fig. 12 is a diagram illustrating a step of collecting a graft according to the transplantation method using the cell transplantation device of the first embodiment.
Fig. 13 is a diagram illustrating a step of collecting a graft according to the transplantation method using the cell transplantation device of the first embodiment.
Fig. 14 is a diagram illustrating a step of puncturing the needle into a transplantation site according to the transplantation method using the cell transplantation device of the first embodiment.
Fig. 15 is a diagram illustrating a step of placing a graft according to the transplantation method using the cell transplantation device of the first embodiment.
Fig. 16 is a diagram illustrating arrangement of a cell transplantation device and a tray, the cell transplantation device having a plurality of needles, in the transplantation method using the cell transplantation device of the first embodiment.
Fig. 17 is a diagram illustrating a cross-sectional structure of a needle in a second embodiment of the cell transplantation device.
Fig. 18 is a diagram illustrating a cross-sectional structure of a needle in a modification of the cell transplantation device of the second embodiment.
Fig. 19 is a diagram illustrating a cross-sectional structure of a needle in a modification of the cell transplantation device of the second embodiment.
Fig. 20 is a diagram illustrating a side structure of a cell transplantation device of a third embodiment, in which the cell transplantation device has a single needle.
Fig. 21 is a diagram illustrating a side structure of the cell transplantation device of the third embodiment, in which the cell transplantation device has a plurality of needles.
Fig. 22 is a diagram illustrating a cross-sectional structure of a cell transplantation device in a first example of the cell transplantation device of the third embodiment.
Fig. 23 is a diagram illustrating a planar structure and a side structure of a first tube on which a filter unit is provided in the first example of the cell transplantation device of the third embodiment.
Fig. 24 is a diagram illustrating a cross-sectional structure of a needle in a first example of the cell transplantation device of the third embodiment.
Fig. 25 is a diagram illustrating a cross-sectional structure of a cell transplantation device in a second example of the cell transplantation device of the third embodiment.
Fig. 26 is a diagram illustrating arrangement of a cell transplantation device and a tray that holds a graft according to a transplantation method using the cell transplantation device of the third embodiment.
Fig. 27 is a diagram illustrating a step of collecting a graft according to the transplantation method using the cell transplantation device of the third embodiment.
Fig. 28 is a diagram illustrating a step of collecting a graft according to the transplantation method using the cell transplantation device of the third embodiment.
Fig. 29 is a diagram illustrating a step of puncturing the needle into a transplantation site according to the transplantation method using the cell transplantation device of the third embodiment.
Fig. 30 is a diagram illustrating a step of placing a graft according to the transplantation method using the cell transplantation device of the third embodiment.
Fig. 31 is a diagram illustrating arrangement of a cell transplantation device and a tray, the cell transplantation device having a plurality of needles, in the transplantation method using the cell transplantation device of the third embodiment.
Fig. 32 is a diagram illustrating a side structure of a cell transplantation device of a fourth embodiment.
Fig. 33 is a diagram illustrating an example of a planar structure of the cell transplantation device of the fourth embodiment.
Fig. 34 is a diagram illustrating an example of a planar structure of the cell transplantation device of the fourth embodiment.
Fig. 35 is a diagram illustrating an example of a cross-sectional structure of a transplantation needle provided in the cell transplantation device of the fourth embodiment.
Fig. 36 is a diagram illustrating a graft collected into a transplantation needle of the fourth embodiment.
Fig. 37 is a diagram illustrating a side structure of a cell transplantation device of a fifth embodiment.
Fig. 38 is a diagram illustrating a side structure of a cell transplantation device in a modification of the cell transplantation device of the fifth embodiment.
Fig. 39 is a diagram illustrating an overall structure of a cell transplantation system of a sixth embodiment.
Fig. 40 is a diagram illustrating a side structure of the cell transplantation device of the sixth embodiment, in which the cell transplantation device has a single needle.
Fig. 41 is a diagram illustrating a side structure of the cell transplantation device of the sixth embodiment, in which the cell transplantation device has a plurality of needles.
Fig. 42 is a diagram illustrating a configuration of the cell transplantation device of the sixth embodiment, in which the cell transplantation device has a suction-ejection unit.
Fig. 43 is a diagram illustrating a cross-sectional structure of a needle in the cell transplantation device of the sixth embodiment.
Fig. 44 is a diagram illustrating arrangement of a cell transplantation device and a tray that holds a graft according to a transplantation method using the cell transplantation device of the sixth embodiment.
Fig. 45 is a diagram illustrating a step of collecting a graft according to the transplantation method using the cell transplantation device of the sixth embodiment.
Fig. 46 is a diagram illustrating a step of collecting a graft according to the transplantation method using the cell transplantation device of the sixth embodiment.
Fig. 47 is a diagram illustrating a cross-sectional structure of a needle holding a graft in the cell transplantation device of the sixth embodiment.
Fig. 48 is a flowchart illustrating a procedure performed in the cell transplantation system of the sixth embodiment.
Fig. 49 is a diagram illustrating a step of puncturing the needle into a transplantation site according to the transplantation method using the cell transplantation device of the sixth embodiment.
Fig. 50 is a diagram illustrating a step of placing a graft according to the transplantation method using the cell transplantation device of the sixth embodiment.
Fig. 51 is a diagram illustrating arrangement of a cell transplantation device and a tray, the cell transplantation device having a plurality of needles, in the transplantation method using the cell transplantation device of the sixth embodiment.
Fig. 52 is a diagram illustrating a configuration of a cell transplantation device of a seventh embodiment focusing on a needle.
Fig. 53 is a diagram illustrating a step of collecting a graft according to a transplantation method using the cell transplantation device of the seventh embodiment.
Fig. 54 is a diagram illustrating a step of collecting a graft according to the transplantation method using the cell transplantation device of the seventh embodiment.
Fig. 55 is a diagram illustrating a step of collecting a graft according to the transplantation method using the cell transplantation device of the seventh embodiment.
Fig. 56 is a diagram illustrating a cross-sectional structure of a needle holding a graft in the cell transplantation device of the seventh embodiment.
Fig. 57 is a diagram illustrating a configuration of a cell transplantation device of an eighth embodiment focusing on a needle.
Fig. 58 is a diagram illustrating a step of collecting a graft according to a transplantation method using the cell transplantation device of the eighth embodiment.
Fig. 59 is a diagram illustrating a step of collecting a graft according to the transplantation method using the cell transplantation device of the eighth embodiment.
Fig. 60 is a diagram illustrating an overall configuration of a cell transplantation device of a ninth embodiment.
Fig. 61 is a cross-sectional structure of a needle and an outer cylinder included in the cell transplantation device of the ninth embodiment.
Fig. 62 is a diagram illustrating an example of a cross-sectional structure of the cell transplantation device of the ninth embodiment.
Fig. 63 is a diagram illustrating a cross-sectional structure of the cell transplantation device of the ninth embodiment, in which the cell transplantation device has a plurality of needles.
Fig. 64 is a diagram illustrating arrangement of a cell transplantation device and a tray that holds a graft according to a transplantation method using the cell transplantation device of the ninth embodiment.
Fig. 65 is a diagram illustrating another example of arrangement of a cell transplantation device and a tray that holds a graft according to a transplantation method using the cell transplantation device of the ninth embodiment.
Fig. 66 is a diagram illustrating another example of arrangement of a cell transplantation device and a tray that holds a graft according to a transplantation method using the cell transplantation device of the ninth embodiment.
Fig. 67 is a diagram illustrating a step of collecting a graft according to the transplantation method using the cell transplantation device of the ninth embodiment.
Fig. 68 is a diagram illustrating a step of collecting a graft according to the transplantation method using the cell transplantation device of the ninth embodiment.
Fig. 69 is a diagram illustrating an example of displacement of an outer cylinder according to the transplantation method using the cell transplantation device of the ninth embodiment.
Fig. 70 is a diagram illustrating an example of displacement of an outer cylinder according to the transplantation method using the cell transplantation device of the ninth embodiment.
Fig. 71 is a diagram illustrating a step of puncturing the needle into a transplantation site according to the transplantation method using the cell transplantation device of the ninth embodiment.
Fig. 72 is a diagram illustrating a step of placing a graft according to the transplantation method using the cell transplantation device of the ninth embodiment.
Fig. 73 is a diagram illustrating arrangement of a cell transplantation device and a tray, the cell transplantation device having a plurality of needles, in the transplantation method using the cell transplantation device of the ninth embodiment.
Fig. 74 is a cross-sectional structure of a needle and an outer cylinder included in a cell transplantation device of a modification of the ninth embodiment.
Fig. 75 is a side structure of a needle included in a cell transplantation device of a modification of the ninth embodiment.
Fig. 76 is a cross-sectional structure of a needle and an outer cylinder included in a cell transplantation device of a modification of the ninth embodiment.
Fig. 77 is a cross-sectional structure of a needle and an outer cylinder included in a cell transplantation device of a modification of the ninth embodiment.
Fig. 78 is a cross-sectional structure of a needle and an outer cylinder included in a cell transplantation device of a modification of the ninth embodiment together with a tray.

### [Description of the Embodiments]

### (Grafts)

In the following embodiments, a cell transplantation device is used for transplantation of a graft into a living body. A transplant target region may be at least one of an intradermal layer and a subcutaneous layer, or tissues in organs or the like. The graft may include groups of cells. The following description will be given of groups of cells, which are objects to be transplanted.

The groups of cells to be transplanted each include a plurality of cells. A group of cells may be an aggregation of a plurality of cells that are aggregated, or may be an aggregation of a plurality of cells that are joined by intercellular junctions. Alternatively, a group of cells may be composed of a plurality of dispersed cells. Further, a group of cells may include undifferentiated cells or differentiated cells, or may include both undifferentiated cells and differentiated cells. The groups of cells include, for example, masses of cells (spheroids), germs, tissues, organs, organoids, and mini organs.

These groups of cells have an ability to control tissue formation in a living body when placed in the target region. Examples of such groups of cells include cell aggregates containing stem cells such as skin-derived stem cells. For example, the groups of cells to be transplanted may be placed in the intradermal or subcutaneous layer to contribute to hair growth or hair restoration. The groups of cells have an ability to function as a hair follicle organ, an ability to differentiate into a hair follicle organ, an ability to induce or promote formation of a hair follicle organ, or an ability to induce or promote formation of hair in a hair follicle. Further, the groups of cells may include cells that contribute to control of hair color, such as pigment cells or stem cells that differentiate into pigment cells. Further, the groups of cells may also include vascular cells.

Specifically, an example of the groups of cells to be transplanted in each embodiment is a hair follicle germ, which is a primitive hair follicle organ. The hair follicle germ includes mesenchymal cells and epithelial cells. In the hair follicle organ, dermal papilla cells, which are mesenchymal cells, induce differentiation of hair follicle epithelial stem cells to form a hair bulb, and hair matrix cells in the hair bulb repeat division to form a hair. The hair follicle germ is a group of cells that differentiates into such hair follicle organs.

The hair follicle germ is formed, for example, by culturing a mixture of mesenchymal cells derived from mesenchymal tissues such as dermal papilla and epithelial cells derived from epithelial tissues located in a bulge region or a hair bulb base under predetermined conditions. It should be noted that the process of forming hair follicle germs is not limited to the above example. In addition, the mesenchymal cells and epithelial cells used for formation of hair follicle germs may also be derived from any tissue. These cells may be derived from a hair follicle organ, may be derived from an organ different from the hair follicle organ, or may be derived from a pluripotent stem cell.

Further, the graft may include, together with the groups of cells, a member that assists in transplantation of the groups of cells.

### (First Embodiment)

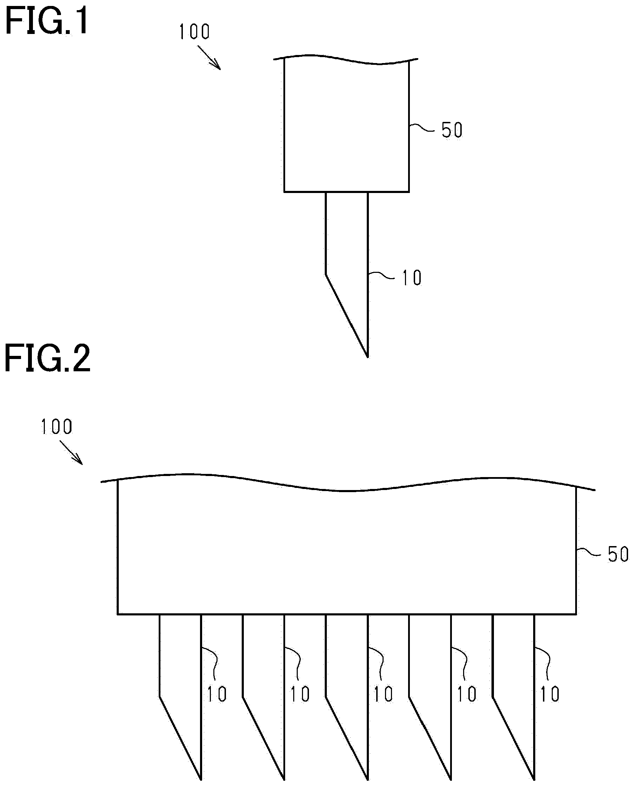

With reference to Figs. 1 to 16, a cell transplantation device of a first embodiment will be described.

### [Overall Configuration of Cell Transplantation Device]

As shown in Fig. 1, a cell transplantation device 100 includes a needle 10, and a support 50 that supports the needle 10. The needle 10 has a tubular shape extending in one direction, in other words, a hollow needle shape. The outer shape of the needle 10 is not specifically limited as long as it has a tip that can puncture a transplant target site in a living body. For example, the tip of the needle 10 may be sharpened by truncating a cylindrical shape obliquely relative to the extending direction.

The support 50 supports the needle 10, surrounding the outer periphery of a portion of the needle 10 which is continuous from the tip of the needle 10. The tip and a region near the tip of the needle 10 protrude from the support 50 in the extending direction of the needle 10. For example, the needle 10 extends through a hole formed in the support 50 whereby it is assembled to the support 50. The outer shape of the support 50 is not specifically limited. A portion of the needle 10 protruding from the support 50 includes at least a portion that penetrates a target region in the living body at the time of transplantation.

The number of needles 10 of the cell transplantation device 100 is not specifically limited. The cell transplantation device 100 may include a single needle 10 as shown in Fig. 1 or a plurality of needles 10 as shown in Fig. 2. When the cell transplantation device 100 includes a plurality of needles 10, the arrangement of the plurality of needles 10 is not specifically limited. The plurality of needles 10 may be regularly arranged or irregularly arranged. Further, when the cell transplantation device 100 includes a plurality of needles 10, it is preferred that all the needles 10 are collectively supported by a single support 50. That is, the plurality of needles 10 are preferably supported by a single support 50 to provide the cell transplantation device 100 having the integrally formed plurality of needles 10.

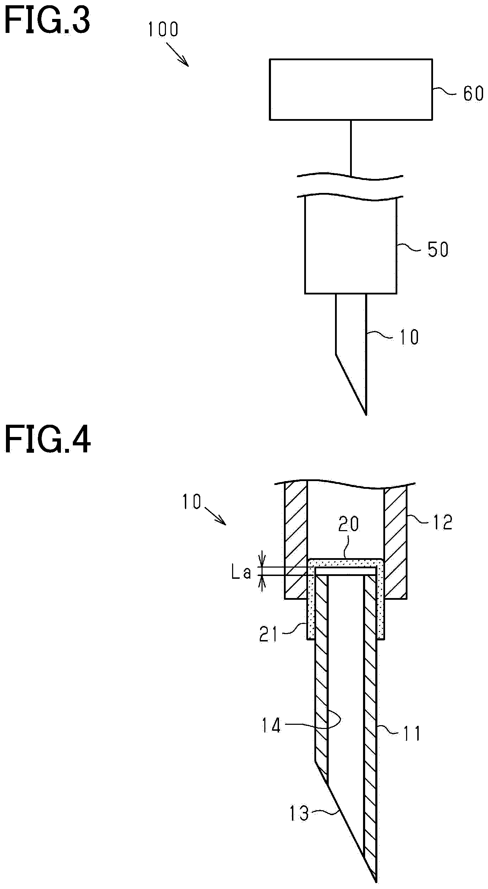

As shown in Fig. 3, the cell transplantation device 100 may include a suction-ejection unit 60 that assists in collecting and ejecting a graft into and from the needle 10. The suction-ejection unit 60 is configured to apply suction to a graft located outside the needle 10 so that the graft is taken into the needle 10 through an opening at the tip of the needle 10, and apply pressure to a graft located inside the needle 10 so that the graft is ejected from the needle 10 through the opening of the needle 10.

The suction-ejection unit 60 is not specifically limited as long as it has a mechanism capable of suction and ejection of a graft, and is connected to the needle 10 in a manner capable of such suction and ejection. For example, the suction-ejection unit 60 may be configured to aspirate a graft together with its protective liquid by sucking in air from the needle 10, and eject the graft together with the protective liquid by injecting air into the needle 10.

Although Fig. 3 shows a configuration of the cell transplantation device 100 having a single needle 10, a cell transplantation device 100 having a plurality of needles 10 may also have the suction-ejection unit 60.

### [Detailed Configuration of Needle]

As shown in Fig. 4, the needle 10 includes a first tube 11 having a tip of the needle 10 and a second tube 12 having a flow channel cross-sectional area larger than that of the first tube 11. The first tube 11 and the second tube 12 each extend in an extending direction of the needle 10. The tip of the first tube 11 constitutes the tip of the needle 10, and has an opening 13. The second tube 12 is connected to a proximal end of the first tube 11. The internal flow channel 14 defined in the needle 10 extends in the extending direction of the needle 10, and communicates with the opening 13. In other words, the internal flow channel 14 is open at the tip of the needle 10.

The flow channel cross-sectional area of the first tube 11 is an area of a region surrounded by the inner peripheral surface of the first tube 11 in a cross-section perpendicular to the extending direction of the needle 10, and the flow channel cross-sectional area of the second tube 12 is an area of a region surrounded by the inner peripheral surface of the second tube 12 in a cross-section perpendicular to the extending direction of the needle 10. The flow channel cross-sectional area of the first tube 11 is constant, and the flow channel cross-sectional area of the second tube 12 is also constant.

For example, the first tube 11 excluding the opening 13 and the second tube 12 are each formed in a hollow cylindrical shape having a constant inner diameter, and the inner diameter of the second tube 12 is larger than the inner diameter of the first tube 11. An end of the first tube 11 is inserted in an end of the second tube 12 at the connection between the first tube 11 and the second tube 12, and the inner diameter of the second tube 12 is substantially equal to or slightly larger than the outer diameter of the first tube 11. The first tube 11 and the second tube 12 are made of a metal or a resin.

The cell transplantation device 100 includes a filter unit 20, which is disposed in the needle 10 near the position where the flow channel cross-sectional area varies, that is, near the connection between the first tube 11 and the second tube 12. The filter unit 20 intersects the internal flow channel 14 at an intermediate position in the internal flow channel 14. The filter unit 20 allows a protective liquid to pass through from the tip of the needle 10 toward the proximal end, and prevents the passage of a graft.

The filter unit 20 is made of a fibrous member 21. Due to the fibrous member 21 intersecting the internal flow channel 14, the filter unit 20 prevents passage of a graft. The number, material, and arrangement of the fibrous member 21 constituting the filter unit 20 are not specifically limited as long as the filter unit 20 can prevent passage of the graft.

In the first embodiment, the fibrous member 21 is disposed to straddle over the proximal end of the first tube 11. Specifically, both end portions of the fibrous member 21 are disposed on the outer peripheral surface of the first tube 11, whereby the fibrous member 21 extends across the end face of the first tube 11. That is, a portion of the filter unit 20 intersecting the internal flow channel 14 is located inside the second tube 12 and prevents a graft from flowing from the first tube 11 into the second tube 12.

The filter unit 20 may or may not be in contact with the end face on the proximal end of the first tube 11. When the filter unit 20 is not in contact with the end face of the first tube 11, the protective liquid tends to easily flow from one side to the other side of the filter unit 20. However, when a distance from the end face of the first tube 11 to the filter unit 20 is too large, the entire group of cells in the graft may enter the second tube 12 from the first tube 11. In this case, when the group of cells are urged toward the opening 13 for transplantation, it may be caught by the edge of the first tube 11. Therefore, when the group of cells is an aggregation of cells, a distance La between the filter unit 20 and the end face on the proximal end of the first tube 11 in the extending direction of the needle 10 is preferably not larger than the diameter of a group of cells. The distance La is, in other words, a distance between the filter unit 20 and the position where the flow channel cross-sectional area varies in the extending direction of the needle 10.

In transplantation of aggregations of cells such as cell aggregates, which are groups of cells that contribute to hair growth or hair restoration, the number of cells included in a group of cells is approximately from 1 × 10³cells to 4 × 10⁴cells. In this case, the diameter of a sphere that approximates a group of cells, that is, the diameter of a minimum circumscribed sphere of a group of cells, is approximately from 100 µm to 1000 µm. Accordingly, the distance La is preferably 100 µm or less.

Further, in the extending direction of the needle 10, a length from the proximal end of the opening 13 to the filter unit 20 may be larger than the diameter of a group of cells. Accordingly, the length from the proximal end of the opening 13 to the filter unit 20 is preferably 1 mm or more. Furthermore, for reducing the amount of the protective liquid ejected together with the graft in ejection of the graft from the opening 13, the length from the proximal end of the opening 13 to the filter unit 20 is preferably 50 mm or less.

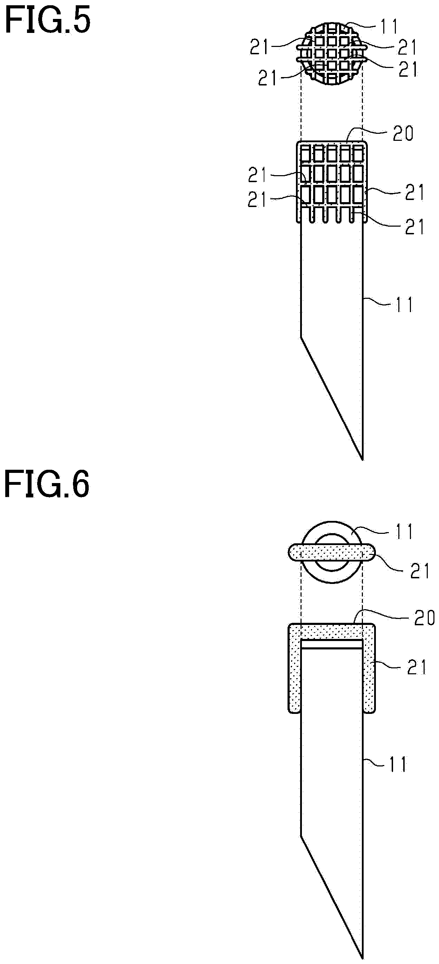

In the following description, specific examples of the arrangement of the fibrous member 21 will be described. Figs. 5 to 7 illustrate the proximal end of the first tube 11 on which the filter unit 20 is disposed, as viewed in a direction parallel to the extending direction of the needle 10 and a direction perpendicular to the extending direction of the needle 10.

In a first example shown in Fig. 5, the filter unit 20 has a mesh form in which a plurality of fibrous members 21 intersect each other. The needle 10 in which the filter unit 20 of the first example is disposed is formed by, for example, preparing a small piece of a mesh in advance in which a plurality of fibrous members 21 intersect each other, adhering this small piece to cover the proximal end of the first tube 11, and joining the first tube 11 to the second tube 12. According to this method, the filter unit 20 can be easily arranged compared with a method in which the fibrous members 21 are arranged one by one.

The filter unit 20 of the first example, when viewed in a direction parallel to the extending direction of the needle 10, includes a region where the fibrous member 21 is located and a region where a gap between the adjacent fibrous members 21 is located. The size of the gap can be regulated by adjusting the diameter, number, and intersection form of the fibrous members 21 to thereby adjust the passage performance of the filter unit 20, that is, difficulty of passing a graft and ease of passing a protective liquid. According to the first example, good passage performance can be easily obtained by setting the detailed passage performance of the filter unit 20.

In a second example shown in Fig. 6, the filter unit 20 is formed of a single fibrous member 21. The needle 10 in which the filter unit 20 of the second example is disposed is formed by, for example, hanging the fibrous member 21 across the proximal end of the first tube 11, fixing the fibrous member 21 thereto, and then joining the first tube 11 to the second tube 12. The fibrous member 21 can be fixed to the first tube 11 simply with an adhesive. Alternatively, two notches may be formed on the edge of the first tube 11 so that the fibrous member 21 can be hooked in these notches and temporarily fixed to the first tube 11 before being fixed to the first tube 11 with an adhesive. Due to the temporary fixation of the fibrous members 21, the fibrous members 21 can be easily arranged. According to the second example, the passage performance of the filter unit 20 can be regulated by adjusting the diameter and arrangement position of the fibrous member 21.

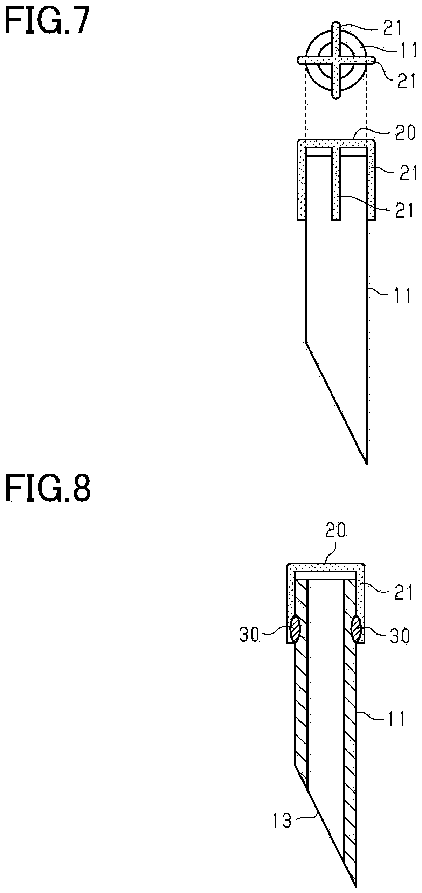

In a third example shown in Fig. 7, the filter unit 20 is formed of two fibrous members 21. The two fibrous members 21 intersect each other at a position facing the opening on the proximal end of the first tube 11. The needle 10 in which the filter unit 20 of the third example is disposed is formed by, for example, hanging two fibrous members 21 across the proximal end of the first tube 11, fixing the fibrous members 21 thereto, and then joining the first tube 11 to the second tube 12. The fibrous members 21 can be fixed to the first tube 11 simply with an adhesive. Alternatively, four notches may be formed on the edge of the first tube 11 so that two fibrous members 21 intersecting each other can be hooked in these notches and temporarily fixed thereto before being fixed to the first tube 11 with an adhesive. Due to the temporary fixation of the fibrous members 21, the fibrous members 21 can be easily arranged. According to the third example, the passage performance of the filter unit 20 can be regulated by adjusting the diameter and arrangement position of the fibrous members 21.

In plan view in the extending direction of the needle 10, an area of a portion of the opening on the proximal end of the first tube 11 which overlaps the fibrous member 21 is defined as a shielding area S. Regardless of the number and arrangement of the fibrous members 21, the ratio of the shielding area S to the area of the opening on the proximal end of the first tube 11 is preferably 20% or more and 85% or less. That is, in the case where the first tube 11 has a constant flow channel cross-sectional area, the ratio of the shielding area S to the flow channel cross-sectional area of the first tube 11 is preferably 20% or more and 85% or less.

When the above ratio of the shielding area S is 20% or more, an effect of preventing the graft from passing through the filter unit 20 can be sufficiently achieved. When the above ratio of the shielding area S is 85% or less, the filter unit 20 facilitates passage of the protective liquid, reducing a pressure loss of the protective liquid. Accordingly, suction and ejection can be smoothly performed. Further, even when the shielding area S is large, a pressure loss of the protective liquid when passing through the filter unit 20 can be reduced by increasing the distance La between the filter unit 20 and the first tube 11.

The material of the fibrous member 21 is not specifically limited as long as it is a threadlike elongated structure. The fibrous member 21 is not limited to a typical fiber, but may also be a structure processed into a wire shape or a tube shape. Examples of fibers that can be used include: plant fibers such as cotton, hemp, flax, ramie, and jute; animal fibers such as wool and silk; mineral fibers; regenerated fiber such as rayon, polynosic, and Cupro fibers; semi-synthetic fibers such as acetate, triacetate, and promix; synthetic fibers such as Nylon, polyester, acrylic, Vinylon, polyethylene, polypropylene, and polyurethane; glass fibers; metal fibers; and carbon fibers. In addition, each fibrous member 21 may have a configuration in which a plurality of fibers are twisted.

In particular, when the graft is susceptible to physical impact, it is preferred to decrease the impact that the graft which flows into the internal flow channel 14 via the opening 13 receives when it comes into contact with the filter unit 20. Examples of the cases where the graft is susceptible to physical impact include, for example, the cases where cells are easily damaged by impact, and where an aggregation of cells is easily broken due to weak bonding between the cells.

In order to reduce the above impact, the fibrous member 21 is preferably made of a fiber having a nominal water retention value of 7.0% or more. When the fibrous member 21 is made of a fiber having the above nominal water retention value, liquid such as the protective liquid can be drawn to the same level as that of the filter unit 20 to cause the fibrous member 21 to retain the liquid and increase the flexibility of the fibrous member 21 before the graft is collected into the needle 10. Due to the increased flexibility of the fibrous member 21, the impact that the graft receives when it comes into contact with the filter unit 20 decreases. Therefore, physical impact on the graft can be reduced.

### [Assembly of Needle]

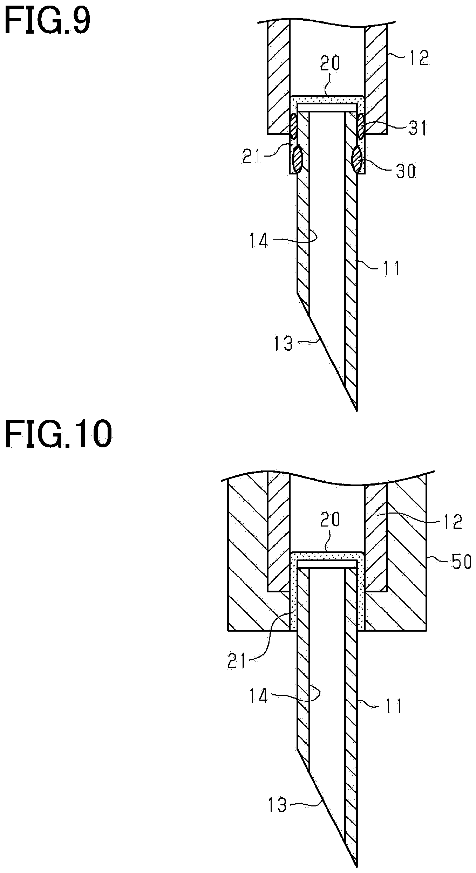

A procedure of assembling the needle 10 and the filter unit 20 will be described below. In Figs. 8 and 9, an adhesive portion used for fixing the members is emphasized.

As shown in Fig. 8, first, the filter unit 20 is formed by fixing the fibrous member 21 to the proximal end of the first tube 11. Specifically, end portions of the fibrous member 21 are bonded to the outer peripheral surface of the first tube 11 via the adhesive portion 30. The adhesive portion 30 may be formed on part of the circumference of the outer peripheral surface of the first tube 11, and configured to fix the fibrous member 21 in position.

Then, as shown in Fig. 9, the proximal end of the first tube 11 together with the filter unit 20 disposed thereon is inserted into an end of the second tube 12 so that the outer peripheral surface of the first tube 11 and the fibrous member 21 located on the outer peripheral surface are bonded to the inner peripheral surface of the second tube 12 via an adhesive portion 31. The adhesive portion 31 is formed on the entire circumference of the outer peripheral surface of the first tube 11 and the inner peripheral surface of the second tube 12. The end portions of the fibrous member 21 may be exposed from the second tube 12, or may be covered with the second tube 12. The adhesive portions 30 and 31 may be an adhesive made of a known adhesive resin.

Thus, the second tube 12 is fixed to the first tube 11 to form the needle 10, and the needle 10 and the filter unit 20 are assembled to each other. According to the above production method, since the filter unit 20 can be positioned in the internal flow channel 14 while the first tube 11 is joined to the second tube 12, the needle 10 in which the filter unit 20 is disposed can be easily formed. The procedure of assembling the needle 10 and the filter unit 20, and the method of fixing the first tube 11, the second tube 12, and the fibrous member 21 to each other are not limited to those described above. Any method by which the filter unit 20 can be disposed at an intermediate position in the internal flow channel 14 can be used.

Fig. 10 shows a cross-sectional structure of the cell transplantation device 100 having a single needle 10, illustrating that the single needle 10 is assembled to the support 50. A portion of the first tube 11 including at least the opening 13, which is a portion to be advanced into a target region in transplantation, may be exposed from the support 50. The entire second tube 12 may be covered with the support 50, or an end of the second tube 12 may be exposed from the support 50. Further, when an end portion of the fibrous member 21 is exposed from the second tube 12, the end portion of the fibrous member 21 may be covered with the support 50, or may be exposed from the support 50. When both the second tube 12 and the end portion of the fibrous member 21 are covered with the support 50, it is possible to prevent a level difference from being formed at the connection between the first tube 11 and the second tube 12, and prevent an external object from being caught by the edge of the fibrous member 21.

### [Transplantation Method]

A method of transplanting a graft using the cell transplantation device 100 will be described below.

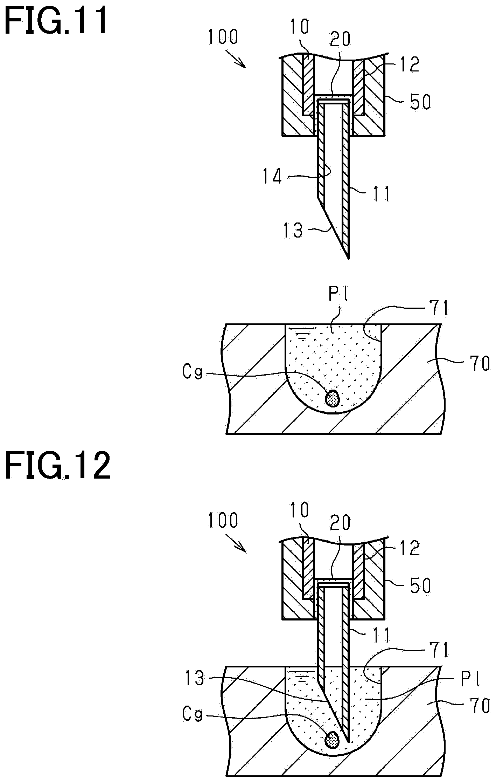

As shown in Fig. 11, a graft Cg together with a protective liquid Pl is held in a tray 70 such as a culture vessel before transplantation. For example, as shown in Fig. 11, the graft Cg and the protective liquid Pl are stored in a recess 71 of the tray 70. The graft Cg and the protective liquid Pl may not necessarily be held in the recess 71, and, for example, the graft Cg and the protective liquid Pl may be placed on a flat surface of the tray 70.

The protective liquid Pl may be any liquid that is unlikely to hinder the viability of cells, and is preferably a liquid that has a minimal influence on a living body when injected into the living body. For example, the protective liquid Pl may be physiological saline, liquid that protects the skin such as petrolatum or lotion, or a mixture of these liquids. The protective liquid Pl may contain additive components such as a nutrient composition. Further, when the tray 70 in which the graft Cg is cultured is a culture vessel, the protective liquid Pl may be a culture medium for culturing cells or a liquid exchanged from the culture medium. The liquid material which contains the graft Cg and the protective liquid Pl may be a low viscosity fluid or a high viscosity fluid. Further, the above liquid material may be a sol or a gel. The above liquid material may have a configuration in which the graft Cg and the protective liquid Pl are held in a matrix made of a polymer material or the like. The polymer material constituting the matrix may be, for example, collagen and hyaluronic acid.

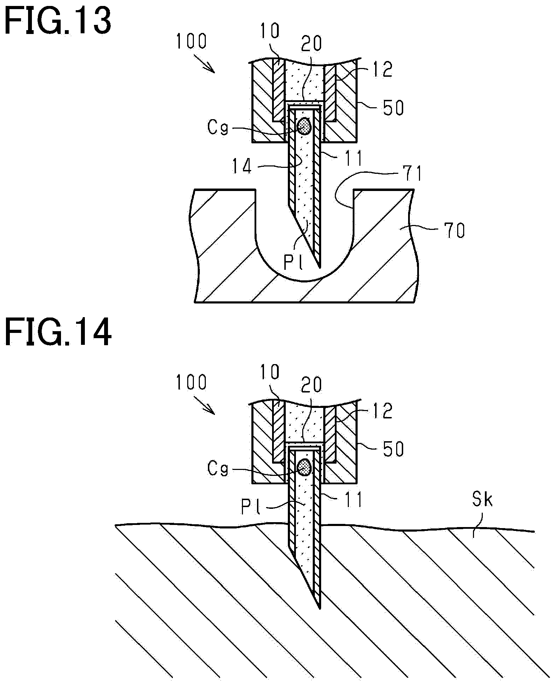

The cell transplantation device 100 aspirates the graft Cg into the needle 10 through the opening 13. More specifically, the tip of the needle 10 is directed toward the graft Cg stored together with the protective liquid Pl in the tray 70, and the graft Cg is aspirated together with the protective liquid Pl into the needle 10. For example, after the needle 10 is aligned with the recess 71 as shown in Fig. 11, the tip of the needle 10 is inserted into the recess 71 as shown in Fig. 12. Then, as the suction-ejection unit 60 is actuated as shown in Fig. 13, the graft Cg is aspirated together with the protective liquid Pl into the internal flow channel 14 through the opening 13.

The graft Cg that has entered the internal flow channel 14 through the opening 13 flows in the first tube 11 along with a flow of the protective liquid Pl toward the filter unit 20. Since the filter unit 20 allows the protective liquid Pl to pass through, the protective liquid Pl flows from the first tube 11 into the second tube 12 along with an aspiration flow, and further flows toward the proximal end of the needle 10. On the other hand, since the filter unit 20 blocks the graft Cg, the graft Cg is retained in a region between the filter unit 20 and the tip of the needle 10, that is, within the first tube 11. Thus, the graft Cg is collected into the first tube 11, and suction is stopped.

Subsequently, as shown in Fig. 14, the needle 10 is inserted into a transplantation site such as a skin Sk in the living body, and the tip of the first tube 11 is advanced into the skin Sk so that the opening 13 is positioned at the transplant target region. When an object to be transplanted is a group of cells that contributes to hair growth or hair restoration, the target region is at least one of an intradermal site and subcutaneous site.

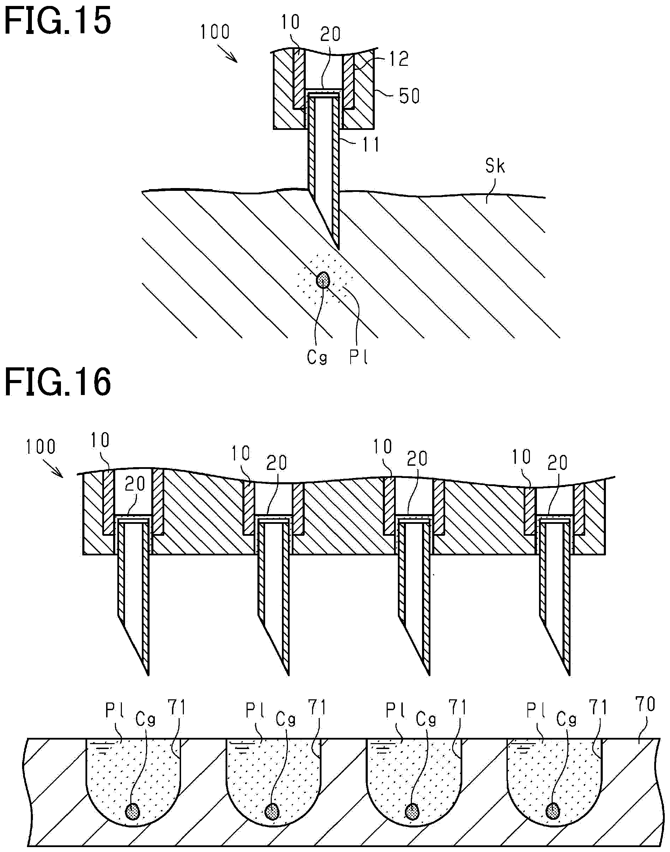

As shown in Fig. 15, as the suction-ejection unit 60 is actuated, the graft Cg that has been collected into the first tube 11 is ejected together with the protective liquid Pl through the opening 13. Accordingly, the graft Cg is placed at the target region. Then, the needle 10 is removed from the skin Sk.

Thus, transplantation of the graft Cg to the target region is completed.

According to the above transplantation method using the cell transplantation device 100, in which the grafts Cg can be collected from the tray 70 and placed in the transplant target region continuously using a single device, smooth transplantation of cells can be performed.

In placement of the graft Cg in the target region, if the amount of the protective liquid Pl ejected together with the graft Cg from the needle 10 excessively increases, the transplantation site may swell and the transplantation is hindered. For example, in use of the cell transplantation device 100 having a plurality of needles 10, the opening 13 of some of the needles 10 may be closed by the swollen transplantation site, which interferes with ejection of the graft Cg. Further, in removal of the needle 10 from the transplantation site, the protective liquid PI, together with the graft Cg, may flow back onto the surface of the transplantation site.

On the other hand, according to the cell transplantation device 100 of the present embodiment, in which the filter unit 20 is disposed at an intermediate position in the internal flow channel 14, the graft Cg is retained in a region between the filter unit 20 and the tip of the needle 10, that is, within the first tube 11 having the opening 13, once the graft Cg is collected in the needle 10. Accordingly, compared with the case where no filter unit 20 is disposed, the graft Cg can be retained at a position near the opening 13. Then, the needle 10 is advanced to the transplantation site while retaining the graft Cg at a position near the opening 13, and the graft Cg is ejected through the opening 13. Accordingly, it is possible to prevent the amount of the protective liquid Pl ejected together with the graft Cg from excessively increasing.

In particular, since the filter unit 20 allows the protective liquid Pl to pass through, the graft Cg is retained at a position near the opening 13 and the amount of the protective liquid Pl ejected together with the graft Cg is maintained at a predetermined amount regardless of the amount of the protective liquid Pl that has been aspirated together with the graft Cg and the period of suction. Therefore, even when the amount of the protective liquid Pl aspirated and the period of suction are increased in order to more successfully collect the grafts Cg, it is possible to prevent the amount of the protective liquid Pl ejected together with the graft Cg from increasing compared with the configuration in which no filter unit 20 is disposed. Accordingly, collection and ejection of the grafts Cg can be more successfully performed, which further improves the efficiency of transplantation.

Fig. 16 illustrates a step in a transplantation method using a cell transplantation device 100 having a plurality of needles 10.

Each filter unit 20 is disposed in a respective needle 10. The positional relationship among the needle 10, the filter unit 20, and the support 50 in the extending direction of the needle 10 in the cell transplantation device 100 having a plurality of needles 10 is the same as that in the cell transplantation device 100 having a single needle 10.

In collection of the grafts Cg using the cell transplantation device 100 having a plurality of needles 10, the cell transplantation device 100 is aligned with the tray 70 so that the respective needles 10 are directed to the corresponding grafts Cg. Accordingly, the grafts Cg can be collected into the corresponding needles 10 in a single operation. Further, a plurality of grafts Cg can be ejected from the respective needles 10 into the target region in a single operation. Accordingly, the efficiency of transplantation is further improved.

As described above, according to the cell transplantation device of the first embodiment, in which the grafts Cg can be collected from the tray 70 and placed in the transplant target region continuously using a single device, smooth transplantation of cells can be performed. Furthermore, according to the cell transplantation device of the first embodiment, the following effects can be achieved.

(1-1) Since the filter unit 20 is disposed at an intermediate position in the internal flow channel 14, the graft Cg can be retained near the opening 13 regardless of the amount of the protective liquid Pl that has been aspirated together with the graft Cg and the period of suction. Accordingly, even when the amount of the protective liquid Pl aspirated and the period of suction increase, it is possible to prevent the amount of the protective liquid Pl ejected together with the graft Cg from excessively increasing, and suitably perform collection and ejection of the grafts Cg. Therefore, the efficiency of transplantation is improved.

(1-2) The needle 10 includes the first tube 11 and the second tube 12, and the filter unit 20 is located near the connection between the first tube 11 and the second tube 12. Therefore, when the graft Cg is collected into the needle 10 through the opening 13, the graft Cg can be retained inside the first tube 11. Further, since the flow channel cross-sectional area of the second tube 12 is larger than the flow channel cross-sectional area of the first tube 11, the protective liquid Pl can easily flow toward the proximal end of the needle 10 via the filter unit 20 as it is aspirated together with the graft Cg.

(1-3) When the filter unit 20 has a mesh form in which a plurality of fibrous members 21 intersect each other, the filter unit 20 can be easily assembled to the needle 10 by preparing a small piece of a mesh in advance in which the fibrous members 21 intersect each other.

(1-4) When the fibrous member 21 is made of a fiber having a nominal water retention value of 7.0% or more, the fibrous member 21, which easily retains water, can contain liquid in advance to increase the flexibility of the fibrous member 21. Accordingly, the impact that the graft Cg receives when it comes into contact with the filter unit 20 decreases so that the graft Cg can be less affected by the impact.

(1-5) The ratio of the shielding area S to the flow channel cross-sectional area of the first tube 11 is 20% or more and 85% or less. With this configuration, an effect of preventing the graft Cg from passing through the filter unit 20 can be sufficiently achieved. On the other hand, since the protective liquid Pl aspirated together with the graft Cg into the needle 10 can easily pass through the filter unit 20, a pressure loss of the protective liquid Pl can be reduced. Accordingly, collection and ejection of the liquid containing the graft Cg can be smoothly performed.

(1-6) The fibrous member 21 is disposed to straddle over the proximal end of the first tube 11, and an end portion of the fibrous member 21 is fixed to an outer peripheral surface of the first tube 11. With this configuration, the filter unit 20 can be assembled to the needle 10 by joining the first tube 11 to the second tube 12. Therefore, positioning of the filter unit 20 can be easily performed compared with the case where drilling or other processing of the first tube 11 or the second tube 12 is required for assembly of the filter unit 20.

(1-7) When the distance La between the filter unit 20 and the end face on the proximal end of the first tube 11 in the extending direction of the needle 10 is 100 µm or less, the entire group of cells contributing to hair growth or hair restoration included in the graft Cg is prevented from entering the second tube 12. Accordingly, in ejection of the group of cells, the group of cells is prevented from being caught by the edge of the first tube 11 at the connection between the first tube 11 and the second tube 12.

(1-8) When the cell transplantation device 100 includes a plurality of needles 10 and filter units 20 each disposed in a respective needle 10, it is possible to collect the grafts Cg into the corresponding needles 10 in a single operation and eject the grafts Cg from the respective needles 10 in a single operation. Accordingly, the efficiency of transplantation of the cells is further improved.

(1-9) The graft Cg includes an aggregation of cells having a diameter of 100 µm or more and 1000 µm or less as a group of cells. Further, the graft Cg includes a cell aggregate containing skin-derived stem cells as a group of cells. When transplantation of such a group of cells is performed for hair growth or hair restoration, a large number of groups of cells are typically required to be transplanted. Using the cell transplantation device 100 for transplantation of the group of cells greatly improves the efficiency of cell transplantation, which is a great advantage for smooth cell transplantation.

### (Second Embodiment)

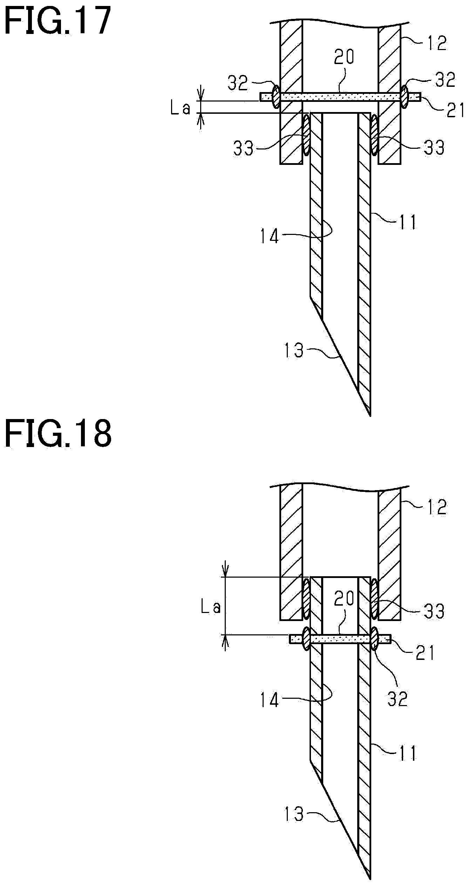

With reference to Figs. 17 to 19, a cell transplantation device of a second embodiment will be described. The following description focuses on differences between the second embodiment and the first embodiment, and configurations which are the same as that of the first embodiment will be referred to by the same reference numbers and the description thereof will be omitted.

In the cell transplantation device of the second embodiment, assembly of the needle 10 and the filter unit 20 differs from that of the first embodiment, while the structure and function of the support 50 and the suction-ejection unit 60 are the same as those of the first embodiment. Therefore, the following drawings only illustrate the needle 10 and the filter unit 20 to describe their configurations. The cell transplantation device of the second embodiment may include a single needle 10 or a plurality of needles 10. Each filter unit 20 is disposed in a respective needle 10.

As shown in Fig. 17, in the second embodiment, the fibrous member 21 penetrates the side wall of the needle 10, intersecting the internal flow channel 14, to form the filter unit 20. The filter unit 20 is located on the same side of the position where the flow channel cross-sectional area of the needle 10 varies as that on which the proximal end of the needle 10 is located, that is, between the position where the first tube 11 and the second tube 12 are connected to each other and the proximal end of the needle 10. As the filter unit 20 is located in the second tube 12 and intersects the internal flow channel 14, the fibrous member 21 penetrates the second tube 12 in a direction perpendicular to the extending direction of the needle 10. Both end portions of the fibrous member 21 are fixed to the outer peripheral surface of the second tube 12 via an adhesive portion 32.

Further, the proximal end of the first tube 11 is inserted in the distal end of the second tube 12 while the fibrous member 21 is not sandwiched between the first tube 11 and the second tube 12 at the connection therebetween. The outer peripheral surface of the first tube 11 is fixed to the inner peripheral surface of the second tube 12 via an adhesive portion 33.

The filter unit 20 allows the passage of a protective liquid, and prevents a graft from entering the second tube 12 from the first tube 11. The number and arrangement of the fibrous members 21 constituting the filter unit 20 are not specifically limited. The passage performance of the filter unit 20 can be regulated by adjusting the number, diameter, and arrangement of the fibrous members 21.

In order to reliably prevent the graft from entering the second tube 12, the distance La between the filter unit 20 and the end face of the first tube 11 in the extending direction of the needle 10 in the second embodiment is also preferably not larger than the diameter of the graft. When an object to be transplanted is a group of cells that contributes to hair growth or hair restoration, the above distance La is preferably 100 µm or less.

A transplantation method using the cell transplantation device of the second embodiment is performed in the same manner as in the first embodiment. That is, first, a graft is aspirated together with the protective liquid into the internal flow channel 14 through the opening 13 of the needle 10. Since the filter unit 20 allows the protective liquid to pass through but blocks the graft, the graft is retained between the filter unit 20 and the tip of the needle 10 in the first tube 11. Then, the needle 10 is inserted into the transplantation site, and the graft is ejected through the opening 13. Thus, the graft Cg is placed in the transplant target region in the living body.

Since the filter unit 20 in the second embodiment is also disposed at an intermediate position in the internal flow channel 14, the graft can be retained near the opening 13 regardless of the amount of the protective liquid and the period of suction. Accordingly, even when the amount of the protective liquid aspirated and the period of suction increase, it is possible to prevent the amount of the protective liquid ejected together with the graft from excessively increasing, and suitably perform collection and ejection of the grafts. Therefore, the efficiency of transplantation is improved.

As described above, according to the cell transplantation device of the second embodiment, in which the grafts can be collected from the tray and placed in the transplant target region continuously using a single device, smooth transplantation of cells can be performed. Furthermore, according to the cell transplantation device of the second embodiment, the same effects as (1-1), (1-2), (1-4), (1-5), (1-7) to (1-9) in the first embodiment can be achieved.

### [Modifications of Second Embodiment]

Modifications of the second embodiment will be described.

As shown in Fig. 18, the filter unit 20 may be located on the same side of the position where the flow channel cross-sectional area of the needle 10 varies as that on which the tip of the needle 10 is located. That is, the filter unit 20 may be located between the tip of the needle 10 and the connection between the first tube 11 and the second tube 12, and intersect the internal flow channel 14 in the first tube 11. The fibrous member 21 penetrates the first tube 11 in a direction perpendicular to the extending direction of the needle 10, and both end portions of the fibrous member 21 are fixed to the outer peripheral surface of the first tube 11 via the adhesive portion 32.

With this configuration as well, the filter unit 20 prevents the graft from entering the second tube 12 from the first tube 11, and retains the graft between the filter unit 20 and the tip of the needle 10. In this case, the distance La between the filter unit 20 and the end face on the proximal end of the first tube 11 in the extending direction of the needle 10 is also preferably not larger than the diameter of the graft. When an object to be transplanted is a group of cells that contributes to hair growth or hair restoration, the above distance La is preferably 100 µm or less. This prevents the filter unit 20 from being located too close to the tip of the needle 10 and ensures sufficient space to retain the graft.

According to the above modification, the same effects as (1-1), (1-2), (1-4), (1-5), (1-8) and (1-9) in the first embodiment can be achieved.

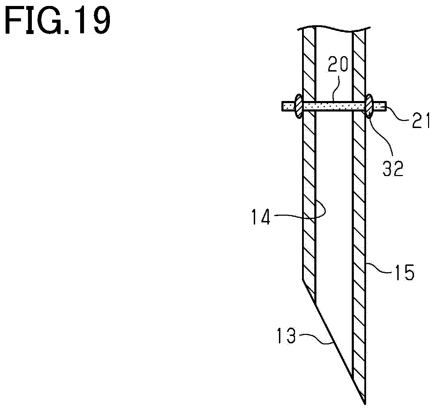

Furthermore, as shown in Fig. 19, a needle 15 may be formed of a single tube. The internal flow channel 14 may have a constant flow channel cross-sectional area. The filter unit 20 intersects the internal flow channel 14 at an intermediate position in the internal flow channel 14. The fibrous member 21 penetrates the needle 15 in a direction perpendicular to the extending direction of the needle 15, and both end portions of the fibrous member 21 are fixed to the outer peripheral surface of the needle 15 via the adhesive portion 32.

In this configuration as well, compared with the case where no filter unit 20 is disposed, the graft can be retained at a position near the opening 13. Accordingly, it is possible to prevent the amount of the protective liquid ejected together with the graft from excessively increasing, and suitably perform collection and ejection of the grafts. Therefore, the efficiency of transplantation is improved.

According to the above modification, the same effects as (1-1), (1-4), (1-5), (1-8) and (1-9) in the first embodiment can be achieved.

### [Modifications of First and Second Embodiments]

The above first and second embodiments and the modifications thereof can be modified and implemented as described below.

- The fibrous member 21 can be fixed to the needle 10 in a manner different from those in the above embodiments and modifications as long as the filter unit 20 is disposed at an intermediate position in the internal flow channel 14.
- The flow channel cross-sectional area of the first tube 11 may not necessarily be constant, and the flow channel cross-sectional area of the second tube 12 may not necessarily be constant. Regardless of the flow channel cross-sectional areas of the first tube 11 and the second tube 12, the filter unit 20 can be assembled to the needle 10 while the first tube 11 is joined to the second tube 12 as long as the fibrous member 21 constituting the filter unit 20 is arranged to straddle over the proximal end of the first tube 11. Accordingly, the filter unit 20 can be easily formed.

Furthermore, when the needle 10 includes a first unit located between the filter unit 20 and the tip of the needle 10 and a second unit located between the filter unit 20 and the proximal end of the needle 10, the first unit having a relatively smaller flow channel cross-sectional area and the second unit having a relatively larger flow channel cross-sectional area, the following effects can be obtained. That is, compared with a configuration in which a portion between the filter unit 20 and the proximal end of the needle 10 throughout the entire length has a flow channel cross-sectional area smaller than that of a portion between the filter unit 20 and the tip of the needle 10, the protective liquid can easily flow toward the proximal end of the needle 10 via the filter unit 20 when the graft is aspirated together with the protective liquid into the needle 10.

The first unit is located on the same side of the filter unit 20 as that on which the tip of the needle 10 is located, and the second unit is located on the same side of the filter unit 20 as that on which the proximal end of the needle 10 is located. In plan view in an extending direction of the needle 10, a portion of the proximal end of the first unit where the fibrous member 21 overlaps the internal flow channel 14 has an area of 20% or more and 85% or less of the flow channel cross-sectional area of the first unit. According to this configuration, the same effect as (1-5) in the first embodiment can be achieved.

- A tube constituting the needle 10 or 15 is not limited to a structure having a hollow cylindrical shape as long as it has openings at the tip and the proximal end, which communicate with each other via a space inside the tube. The outer shape of the above tube may be, but is not limited to, a cylindrical, conical, or prismatic shape. Further, a shape of the space defined in the above tube may be, but is not limited to, a cylindrical, conical, or prismatic shape. When the needle 10 is composed of a plurality of tubes, that is, the first tube 11 and the second tube 12, a space in the first tube 11 and a space in the second tube 12 may communicate with each other to form the internal flow channel 14.

### [Supplementary Notes]

The measure for solving the foregoing problems embraces the following technical ideas derived from the first and second embodiments and the modifications thereof.

### (Item 1)

A cell transplantation device configured to place a graft containing cells into a target region in a living body, the cell transplantation device including:
a needle extending in one direction, the needle having a flow channel defined therein and an opening at a tip of the needle; and
a filter unit made of a fibrous member, the filter unit being disposed at an intermediate position in the flow channel and configured to retain the graft between the filter unit and the opening when a liquid material containing the graft enters the flow channel through the opening.

### (Item 2)

The cell transplantation device according to the item 1, wherein the fibrous member is made of a fiber having a nominal water retention value of 7.0% or more.

### (Item 3)

The cell transplantation device according to the item 1 or 2, wherein
the needle includes
a first unit located on a same side of the filter unit as that on which the tip of the needle is located and
a second unit located on a same side of the filter unit as that on which a proximal end of the needle is located, and
the second unit has a flow channel cross-sectional area which is larger than a flow channel cross-sectional area of the first unit.

### (Item 4)

The cell transplantation device according to the item 1 or 2, wherein
the needle includes
a first tube including the tip and
a second tube connected to the proximal end of the first tube, and
the filter unit is located near a position where the first tube and the second tube are connected to each other.

### (Item 5)

The cell transplantation device according to the item 4, wherein,
in plan view in an extending direction of the needle,
a portion where the fibrous member overlaps an opening on the proximal end of the first tube has an area of 20% or more and 85% or less of an area of the opening on the proximal end.

### (Item 6)

The cell transplantation device according to the item 4 or 5, wherein
the fibrous member is disposed to straddle over the proximal end of the first tube, and
an end portion of the fibrous member is fixed to an outer peripheral surface of the first tube.

### (Item 7)

The cell transplantation device according to any one of the items 4 to 6, wherein a distance between the filter unit and an end face of the proximal end of the first tube in the extending direction of the needle is 100 µm or less.

### (Item 8)

The cell transplantation device according to any one of the items 1 to 7, including a plurality of the needles, and a plurality of the filter units each disposed in a respective needle.

### (Item 9)

The cell transplantation device according to any one of the items 1 to 8, wherein the cell transplantation device is used to place the graft containing a cell aggregate having a diameter of 100 µm or more and 1,000 µm or less into the target region.

### (Third Embodiment)

In transplantation, a graft stored in a container such as a culture vessel is transferred to a transplant target region in the living body. If the cells become dried in the step of transferring the graft to the target region, the cell membrane or protein structure may change and the function of the group of cells may be deactivated.

An object of a third embodiment is to provide a cell transplantation device that enables smooth transplantation of cells while preventing the cells from becoming dried during transfer to transplant target region.

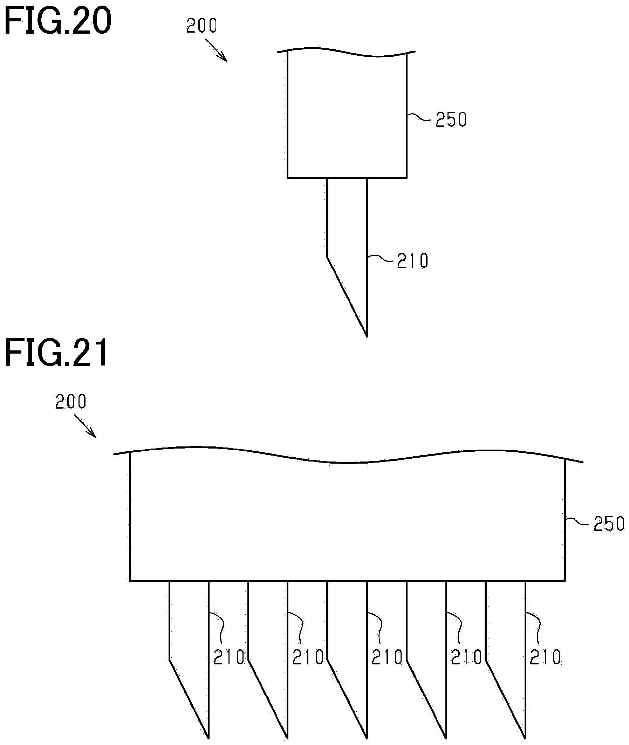

With reference to Figs. 20 to 31, a cell transplantation device of the third embodiment will be described. The same elements as those in the first embodiment are denoted by the same reference signs, and the description thereof is omitted.

### [Overall Configuration of Cell Transplantation Device]

As shown in Fig. 20, a cell transplantation device 200 includes a needle 210, and a support 250 that supports the needle 210. The needle 210 has a tubular shape extending in one direction, in other words, a hollow needle shape. The outer shape of the needle 210 is not specifically limited as long as it has a tip that can puncture a transplant target site in a living body. For example, the tip of the needle 210 may be sharpened by truncating a cylindrical shape obliquely relative to the extending direction.

The support 250 may be formed as a separate member from the needle 210 or may be formed integrally with the needle 210. The outer shape of the support 250 is not specifically limited.

When the support 250 is a member formed separately from the needle 210, the support 250 supports the needle 210, surrounding the outer periphery of a portion of the needle 210 which is continuous from the tip of the needle 210. For example, the needle 210 extends through a hole formed in the support 250 whereby it is assembled to the support 250. The tip and a region near the tip of the needle 210 protrude from the support 250 in the extending direction of the needle 210.

When the support 250 is formed integrally with the needle 210, the needle 210 protrudes from a surface of the support 250.

Regardless of whether the needle 210 and the support 250 are separate members or integrally formed, a portion of the needle 210 protruding from the support 250 includes at least a portion that penetrates a target region in the living body at the time of transplantation. When the target region is an intradermal or subcutaneous site, a portion of the needle 210 protruding from the support 250 preferably has a length of, for example, 200 µm or more and 6 mm or less.

Materials for forming the needle 210 are not specifically limited. For example, the needle 210 may be made of silicon, metal materials such as stainless steel, titanium, cobalt-chromium alloy, and magnesium alloy, or may be made of polymer materials such as commodity plastics, medical grade plastics, and plastics for cosmetic products. Examples of the polymer materials include polyethylene, polypropylene, polystyrene, polyamide, polycarbonate, cyclic polyolefin, polylactic acid, polyglycolic acid, polycaprolactone, acrylic, urethane resin, aromatic polyether ketone, epoxy resin, and copolymer materials of these resins. Further, the needle 210 may be made of ionizing radiation curable materials such as polyacrylic, and thermosetting materials such as urethane and epoxy. Materials for forming the support 250 are also not specifically limited, and the support 250 may be made of, for example, materials described above as the materials for the needle 210. The needle 210 and the support 250 may be made of the same materials or different materials. Further, the needle 210 and the support 250 may also be made of a combination of two or more materials.

The number of needles 210 of the cell transplantation device 200 is not specifically limited. The cell transplantation device 200 may include a single needle 210 as shown in Fig. 20 or a plurality of needles 210 as shown in Fig. 21. When the cell transplantation device 200 includes a plurality of needles 210, the arrangement of the plurality of needles 210 is not specifically limited. The plurality of needles 210 may be regularly arranged or irregularly arranged. Further, when the cell transplantation device 200 includes a plurality of needles 210, it is preferred that all the needles 210 are collectively supported by a single support 250. That is, the plurality of needles 210 are preferably supported by a single support 250 to provide the cell transplantation device 200 having the integrally formed plurality of needles 210.

In transplantation of a group of cells that contributes to hair growth or hair restoration, the arrangement of the group of cells in a direction across the surface of the skin, that is, the arrangement of hairs that grow at positions where the group of cells are transplanted is substantially determined according to the arrangement of the plurality of needles 210. For example, in transplantation of aggregates of cells such as hair follicle germs that contribute to hair growth or hair restoration, the density of the needles 210 per unit area is preferably 1/cm² or more and 400/cm² or less, and more preferably 20/cm² or more and 100/cm² or less. When the density of the needles 210 is no less than the above lower limit, the density of hairs that grow from the hair follicle germs will provide sufficient hair density. When the density of the needles 210 is no more than the above upper limit, nutrients are easily distributed to the groups of cells that are positioned in the skin at the same density as that of the needles 210. Thus, formation of hair follicles and hairs are suitably performed.

Further, in transplantation of groups of cells that contribute to hair growth or hair restoration, an interval which is a center-to-center distance between the adjacent needles 210 is preferably 1 mm or more and 4 mm or less. When the interval is no less than the above lower limit, the cell transplantation device 200 can be easily produced. When the interval is no more than the above upper limit, the density of hairs that grows from the transplanted groups of cells will provide sufficient hair density.

### [Detailed Configuration of Needle]

The needle 210 has an internal flow channel having an opening at the tip of the needle 210, and a region from an intermediate position in the flow channel to the opening serves as a storage portion that stores a liquid material containing a graft and its protective liquid. As specific examples of the structure of the storage portion, the following description will be given of a first example, in which a storage portion is formed by providing a fibrous member, and a second example, in which a storage portion is formed by changing the flow channel cross-sectional area of the internal flow channel.

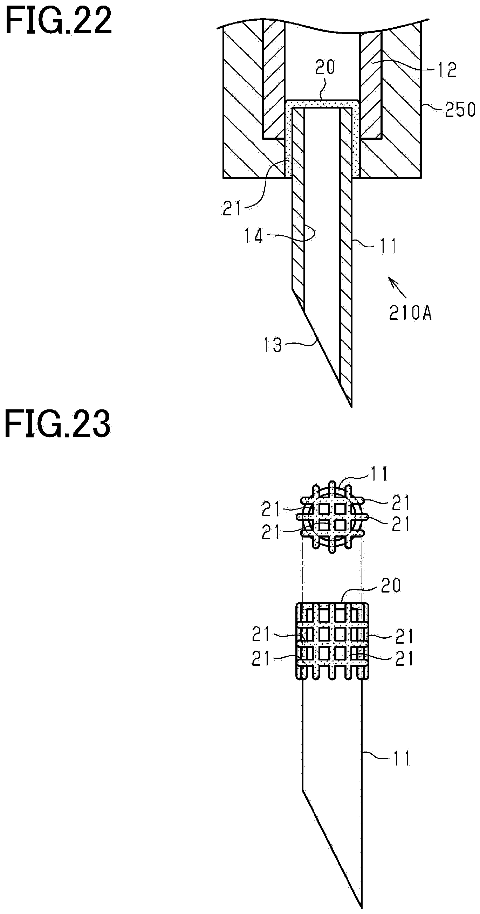

As shown in Fig. 22, a needle 210A, which is the needle 210 of the first example, is a member separate from the support 250. Although Fig. 22 shows a configuration of the cell transplantation device 200 having a single needle 210A, the cell transplantation device 200 may also include a plurality of needles 210A as described above.

The needle 210A of the first example has the same configuration as that of the needle 10 in the first embodiment. That is, the needle 210A includes a first tube 11 having a tip of the needle 210A and a second tube 12 having a flow channel cross-sectional area larger than that of the first tube 11. The tip of the first tube 11 has an opening 13. The second tube 12 is connected to a proximal end of the first tube 11. An internal flow channel 14 defined in the needle 210A extends in the extending direction of the needle 210A, and communicates with the opening 13.

The cell transplantation device 200 includes a filter unit 20 made of a fibrous member 21 at a position where the flow channel cross-sectional area of the needle 210A varies, that is, a position in the second tube 12 adjacent to the proximal end of the first tube 11. The filter unit 20 and the fibrous member 21 have the same configurations as those in the first embodiment. The filter unit 20 intersects the internal flow channel 14 at an intermediate position in the internal flow channel 14. The filter unit 20 allows a protective liquid to pass through from the tip of the needle 210A toward the proximal end, and prevents the passage of a graft.

Fig. 23 illustrates an example of the filter unit 20. In the figure, the proximal end of the first tube 11 on which the filter unit 20 is disposed is illustrated, viewed in a direction parallel to the extending direction of the needle 210A and a direction perpendicular to the extending direction of the needle 210A. In the example shown in Fig. 23, the filter unit 20 has a mesh form in which a plurality of fibrous members 21 intersect each other, and covers the proximal end of the first tube 11.

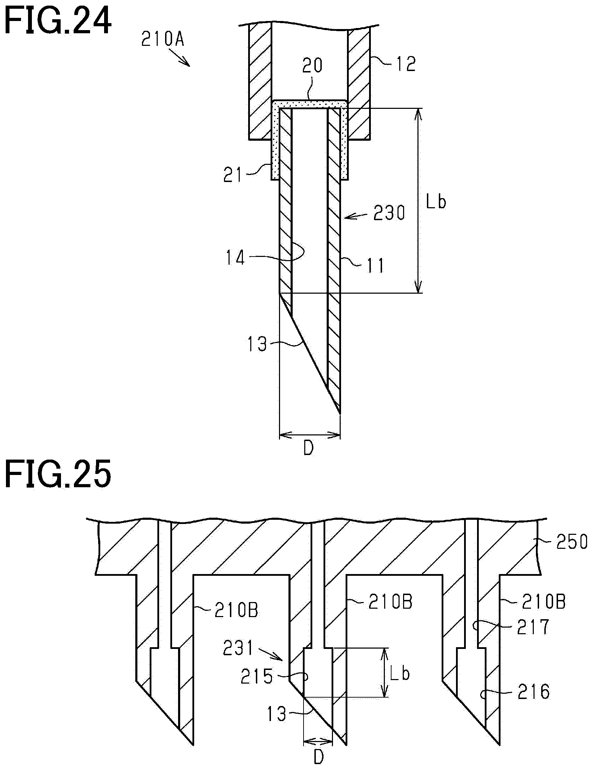

As shown in Fig. 24, a portion surrounded by the side wall from the filter unit 20 to the opening 13 serves as a storage portion 230 that stores a graft and its protective liquid. When the first tube 11 has a hollow cylindrical shape, the inner peripheral surface of the side wall extends in a cylindrical shape, defining a cylindrical space as the storage portion 230.

The storage portion has a length Lb in the extending direction of the needle 210A, which is larger than or equal to a maximum width D of the storage portion 230 in a direction perpendicular to the extending direction of the needle 210A. When the first tube 11 has a hollow cylindrical shape with a constant inner diameter, the maximum width D is the inner diameter, that is, a diameter of a circular region surrounded by the side wall of the first tube 11 in a cross-section perpendicular to the extending direction of the needle 210A.

As shown in Fig. 25, a needle 210B, which is the needle 210 of the second example, is formed integrally with the support 250. Although Fig. 25 shows a configuration of the cell transplantation device 200 having a plurality needles 210B, the cell transplantation device 200 may also include a single needle 210B as described above.

The needle 210B has an internal flow channel 215 defined therein. The internal flow channel 215 extends in the extending direction of the needle 210B, and communicates with the opening 13 at the tip. Further, the internal flow channel 215 penetrating the needle 210B extends to the inside of the support 250.

The internal flow channel 215 has a large-diameter portion 216 and a small-diameter portion 217, which has a flow channel cross-sectional area smaller than that of the large-diameter portion 216. The large-diameter portion 216 is located on the same side of the small-diameter portion 217 as that on which the tip of the needle 210B is located, and the opening 13 is located on the distal side of the large-diameter portion 216. The flow channel cross-sectional area of the large-diameter portion 216 is an area of a region surrounded by the side wall of the large-diameter portion 216 in a cross-section perpendicular to the extending direction of the needle 210B, and the flow channel cross-sectional area of the small-diameter portion 217 is an area of a region surrounded by the side wall of the small-diameter portion 217 in a cross-section perpendicular to the extending direction of the needle 210B.

For example, the large-diameter portion 216 and the small-diameter portion 217 are each formed in a hollow cylindrical shape having a constant inner diameter, and the inner diameter of the small-diameter portion 217 is smaller than the inner diameter of the large-diameter portion 216. The inner diameter of the large-diameter portion 216 has a size that allows the graft to pass through the large-diameter portion 216, and the inner diameter of the small-diameter portion 217 has a size that does not allow the graft to pass through the small-diameter portion 217. For example, the inner diameter of the large-diameter portion 216 is larger than the size assumed as the maximum diameter of the graft, and the inner diameter of the small-diameter portion 217 is smaller than the size assumed as the minimum diameter of the graft.

With this configuration, the graft entering the internal flow channel 215 through the opening 13 flows in the large-diameter portion 216 along with a flow of the protective liquid, and is retained in the large-diameter portion 216 without entering the small-diameter portion 217. Thus, a portion surrounded by the side wall from the position where the flow channel cross-sectional area varies to the opening 13 serves as a storage portion 231 that stores a graft and its protective liquid. When the large-diameter portion 216 has a hollow cylindrical shape, the inner peripheral surface of the side wall extends in a cylindrical shape, defining a cylindrical space as the storage portion 231.

The storage portion 231 of the needle 210B has a length Lb in the extending direction of the needle 210B, which is larger than or equal to a maximum width D of the storage portion 231 in a direction perpendicular to the extending direction of the needle 210B. When the large-diameter portion 216 has a hollow cylindrical shape with a constant inner diameter, the maximum width D is the inner diameter, that is, a diameter of a circular region surrounded by the side wall of the large-diameter portion 216 in a cross-section perpendicular to the extending direction of the needle 210B.

In the following description, a configuration of the storage portion common to the first example and the second example will be described.

The maximum width D is set according to the size of the graft to be stored. When the maximum width D is sufficiently larger than the diameter of the graft, the graft can smoothly enter the storage portion 230 or 231, reducing the occurrence of friction between the graft and the side wall of the storage portion 230 or 231. Accordingly decrease in the function of the group of cells can be suppressed.

Specifically, when the graft is a group of cells that contributes to hair growth or hair restoration, which is a cell aggregate such as spheroids and germs, a diameter of a sphere that approximates the group of cells, which is the graft, that is, a diameter of a minimum circumscribed sphere of the group of cells is approximately from 0.1 mm to 1.0 mm.

The maximum width D is preferably 0.15 mm or more when the approximate diameter of the group of cells is 0.1 mm, and the maximum width D is preferably 1.05 mm or more when the approximate diameter of the group of cells is 1.0 mm.

When the maximum width D is too large, the width of the needle 210 increases, which decreases the ability of the needle 210 to puncture the living body. Further, in collection of the graft into the needle 210, the graft once stored in the storage portion 230 or 231 may migrate in the storage portion 230 or 231 and is likely to exit the storage portion 230 or 231. Therefore, the maximum width D is preferably 2.0 mm or less.

That is, when the graft is a group of cells that contributes to hair growth or hair restoration, the maximum width D is preferably 0.15 mm or more and 2.0 mm or less, and more preferably 0.3 mm or more and 1.5 mm or less.

As described above, the length Lb of the storage portions 230 and 231 is larger than or equal to the maximum width D. With this configuration, the graft and the protective liquid can be stored in the storage portion 230 or 231 in a state in which the graft is surrounded by the protective liquid in the storage portion 230 or 231, and a space between the graft and the tip of the storage portion 230 or 231 is filled with the protective liquid.

In order to prevent the graft from being in contact with the side wall in the storage portion 230 or 231 during collection and ejection of the graft, the length Lb is preferably 15 mm or less.

A volume of the storage portions 230 and 231 is set to be larger than the volume of the graft to be stored.

When the approximate diameter of the group of cells is 0.1 mm, the volume of the group of cells is approximately 0.0005 µL. In this case, when the protective liquid is a water-based liquid such as a physiological saline or a culture medium, approximately 0.0009 µL evaporates in 30 seconds. Accordingly, the volume of the storage portions 230 and 231 is preferably 0.001 µL or more. Further, when the approximate diameter of the group of cells is 1.0 mm, the volume of the group of cells is approximately 0.523 µL. With an increase in the volume of the group of cells, the group of cells tends to migrate slowly and thus the time required for collection and ejection of the graft tends to increase. Accordingly, the volume of the protective liquid that can be stored in the storage portions 230 and 231 preferably increases as the volume of the group of cells increases. Therefore, when the approximate diameter of the group of cells is 1.0 mm, the volume of the storage portions 230 and 231 is preferably 0.75 µL or more. In particular, in a case where the cell transplantation device 200 is manually operated, in which the operation time required for the period from collection to ejection of the graft tends to increase, the volume of the storage portions 230 and 231 is preferably 1 µL.

On the other hand, when the volume of the storage portions 230 and 231 is too large, the time required for collection of the graft and the protective liquid into the storage portions 230 and 231 increases. Further, in collection of the graft into the needle 210, the graft once stored in the storage portion 230 or 231 is likely to exit the storage portion 230 or 231. Therefore, the volume of the storage portions 230 and 231 is preferably 1 µL or less.

That is, when the graft is a cell aggregate that contributes to hair growth or hair restoration, the volume of the storage portions 230 and 231 is preferably 0.001 µL or more and 1 µL or less.

Further, in the condition where the graft is stored in the storage portion 230 or 231 together with a sufficient amount of protective liquid that does not evaporate during transfer of the graft, it is possible to reduce damage or deformation of the graft due to contact between the graft and the inner peripheral surface of the side wall surrounding the storage portion 230 or 231, and more smoothly eject the graft, when the graft is held at a position closer to the opening 13 of the needle 210. Further, the inner peripheral surface of the side wall of the storage portions 230 and 231 having a cylindrical shape is preferred since the contact area between the graft and the inner peripheral surface decreases and damage or deformation of the graft is reduced.

The inner peripheral surface of the side wall surrounding the storage portions 230 and 231 is preferably subjected to a coating treatment for suppressing evaporation of the protective liquid. In other words, the above-mentioned inner peripheral surface preferably has a surface layer for suppressing evaporation of the protective liquid.

The material of the surface layer formed by a coating treatment is preferably a material having compatibility with a living body and a graft, and more preferably a material that is not likely to be degraded by contact with the needle 210 and the protective liquid. Specifically, the material of the surface layer may be alcohols such as glycol or glycerin, ethers, esters, oils such as vegetable oil and silicone, silane coupling agents, or mixtures thereof.

The surface layer has, for example, a function as a surfactant or a function of simply sealing the interface as an oil film to thereby prevent the protective liquid from evaporating. The material of the surface layer may be water-soluble or water-insoluble depending on the properties of the protective liquid. Further, the surface layer may exhibit a function of preventing the evaporation of the protective liquid in a state in which the surface layer is fixed to the inner peripheral surface or eluted in the protective liquid.

### [Transplantation Method]

A method of transplanting a graft using the cell transplantation device 200 will be described below. Although the figures illustrate the cell transplantation device 200 having the needle 210A of the first example, the same procedure of transplantation and the function of the storage portion apply to the cell transplantation device 200 having the needle 210B of the second example.

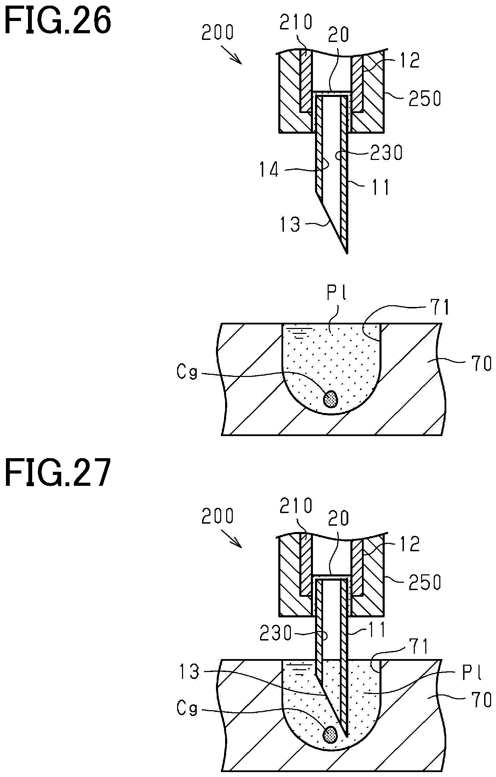

As shown in Fig. 26, a graft Cg together with a protective liquid Pl is held in a tray 70 such as a culture vessel before transplantation. The manner that the graft Cg is held in the tray 70 and the properties of the protective liquid Pl are the same as those in the first embodiment.

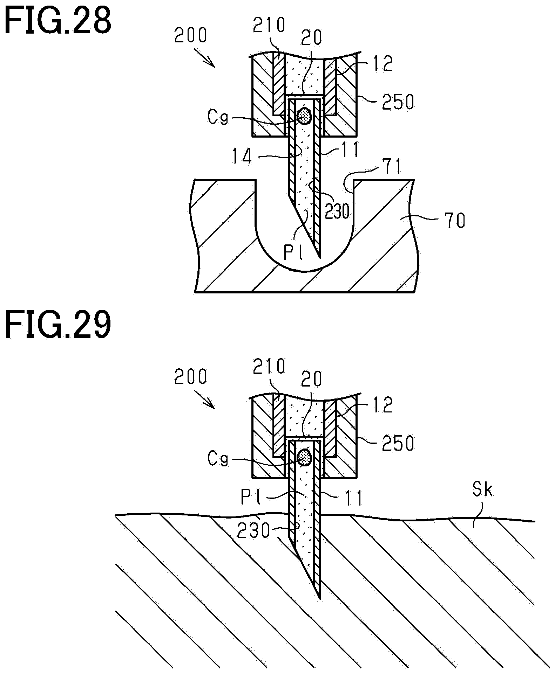

The cell transplantation device 200 aspirates the graft Cg into the needle 210 through the opening 13. More specifically, the tip of the needle 210 is directed toward the graft Cg stored together with the protective liquid Pl in the tray 70, and the graft Cg is aspirated together with the protective liquid Pl into the needle 210. For example, after the needle 210 is aligned with the recess 71 as shown in Fig. 26, the tip of the needle 210 is inserted into the recess 71 as shown in Fig. 27. Then, for example, a mechanism connected to the needle 210 sucks in air from the internal flow channel 14 to aspirate the graft Cg together with the protective liquid Pl into the internal flow channel 14 through the opening 13 as shown in Fig. 28.

The graft Cg that has entered the internal flow channel 14 through the opening 13 flows in the internal flow channel 14 along with a flow of the protective liquid Pl toward the proximal end of the needle 210. Since the proximal end of the storage portion 230 allows the protective liquid Pl to pass through, the protective liquid Pl flows in the storage portion 230 and further toward the proximal end of the needle 210 along with an aspiration flow. On the other hand, since the proximal end of the storage portion 230 blocks the graft Cg, the graft Cg is retained in the storage portion 230. As the suction is stopped, the graft Cg is stored together with the protective liquid Pl in the storage portion 230.

Then, the cell transplantation device 200, in which the graft Cg and the protective liquid Pl are stored in the storage portion 230, is transferred to the transplant target region in the living body. In the present embodiment, the length Lb of the storage portion 230 is larger than or equal to the maximum width D of the storage portion 230, and the maximum width D is larger than the diameter of the graft. Accordingly, the graft Cg and the protective liquid Pl can be stored in the storage portion 230 in a state in which the graft Cg is surrounded by the protective liquid Pl in the storage portion 230, and a space between the graft Cg and the tip of the storage portion 230 is filled with the protective liquid Pl. While the protective liquid Pl gradually evaporates with time, it is possible to prevent the cells from becoming dried during transfer to the transplant target region since the space between the graft Cg and the opening 13 is filled with the protective liquid Pl.

Since the needle 210 is a fine structure and the amount of the protective liquid Pl stored in the storage portion 230 is small, the liquid level in the storage portion 230 tends to be easily displaced toward the proximal end of the storage portion 230 as the protective liquid Pl evaporates during transfer even within a short period of time. Therefore, when it is assumed that it takes a long time to transfer the graft, a large length Lb is preferred.

When the cell transplantation device 200, in which the graft Cg and the protective liquid Pl are stored, is transferred to a target region such as the skin in the living body, the needle 210 is inserted into the skin Sk as shown in Fig. 29. Accordingly, the tip of the needle 210 is advanced into the skin Sk so that the opening 13 is positioned at the transplant target region. When an object to be transplanted is a group of cells that contributes to hair growth or hair restoration, the target region is at least one of an intradermal site and subcutaneous site.

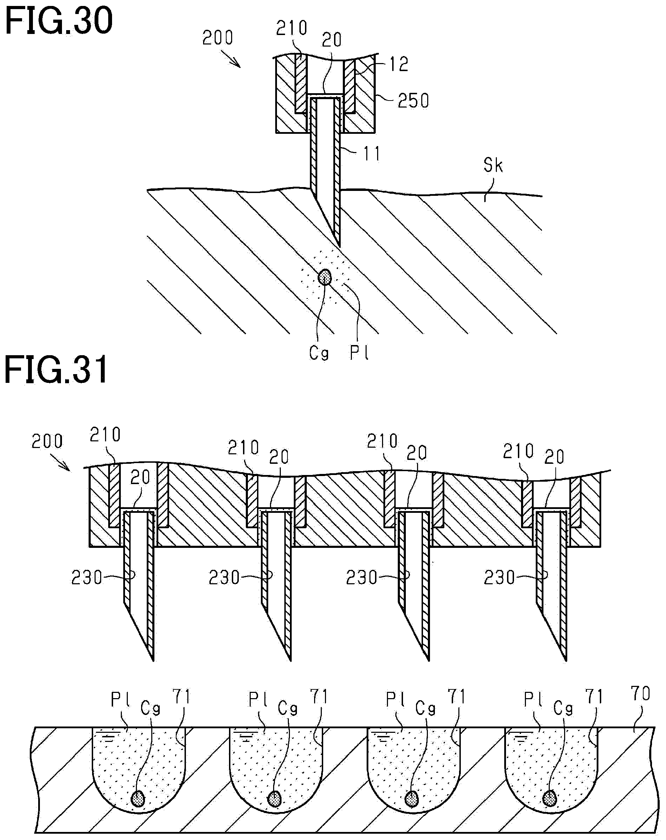

As shown in Fig. 30, for example, a mechanism connected to the needle 210 injects air into the internal flow channel 14 to eject the graft Cg together with the protective liquid Pl from the storage portion 230 through the opening 13. Accordingly, the graft Cg is placed at the target region. Then, the needle 210 is removed from the skin Sk.

Thus, transplantation of the graft Cg to the target region is completed. According to the above transplantation method using the cell transplantation device 200, in which the grafts Cg can be collected from the tray 70 and placed in the transplant target region continuously using a single device, smooth transplantation of cells can be performed. In addition, even when transplantation is performed into a highly elastic tissue such as skin, the needle 210 can be inserted into the tissue while following the elasticity of the tissue, and thus the graft Cg can be accurately positioned at the target region.

Fig. 31 illustrates a step in a transplantation method using the cell transplantation device 200 having a plurality of needles 210.

In collection of the grafts Cg using the cell transplantation device 200 having a plurality of needles 210, the cell transplantation device 200 is aligned with the tray 70 so that the respective needles 210 are directed to the corresponding grafts Cg. Accordingly, the grafts Cg can be collected into the corresponding needles 210 in a single operation. Preferably, each graft Cg is collected into a respective needle 210. Further, a plurality of grafts Cg can be ejected from the respective needles 210 into the target region in a single operation. Accordingly, the efficiency of transplantation is further improved.

As described above, according to the cell transplantation device of the third embodiment, in which the grafts Cg can be collected from the tray 70 and placed in the transplant target region continuously using a single device, smooth transplantation of cells can be performed. Furthermore, according to the cell transplantation device of the third embodiment, the following effects can be achieved.

(3-1) Since the length Lb of the storage portions 230 and 231 is larger than or equal to the maximum width D, the graft Cg can be stored in the storage portion 230 or 231 in a state in which the graft Cg is surrounded by the protective liquid Pl in the storage portion 230 or 231, and a space between the graft Cg and the tip of the storage portion 230 or 231 is filled with the protective liquid Pl. Therefore, it is possible to prevent the cells from drying out during transfer to the transplant target region.

(3-2) When the inner peripheral surface of the side wall of the storage portions 230 and 231 has a cylindrical shape, damage or deformation of the graft due to contact between the graft Cg and the inner peripheral surface can be reduced. Further, since a group of cells as the graft Cg has a shape approximate to a sphere, the shape of the storage portions 230 and 231 can be suitable to store the group of cells having such a shape while being surrounded by the protective liquid Pl.

(3-3) When the inner peripheral surface of the side wall of the storage portions 230 and 231 has a surface layer that suppresses the evaporation of the protective liquid PI, it is possible to further prevent the cells from becoming dried during transfer to the transplant target region.

(3-4) When the graft is a cell aggregate that contributes to hair growth or hair restoration, the storage portions 230 and 231 having a volume of 0.001 µL or more and 1 µL or less can store a sufficient amount of the protective liquid Pl to prevent the cells from becoming dried. Further, in collection of the graft, the graft once stored in the storage portion 230 or 231 can be prevented from exiting the storage portion 230 or 231 since the storage portions 230 and 231 are not excessively large in size.

(3-5) When the cell transplantation device 200 includes a plurality of needles 210, it is possible to collect the grafts Cg into the corresponding needles 210 in a single operation and eject the grafts Cg from the respective needles 210 in a single operation. Accordingly, the efficiency of transplantation of the cells is further improved. Further, the needles 210 can prevent the cells from becoming dried during transfer to the transplant target region. Therefore, it is possible to prevent occurrence of non-uniformity in the state of cells after transfer among the needles 210.

(3-6) When the cell transplantation device 200 is used to place the graft Cg including cells having stem cell properties (such as stem cells and progenitor cells) into the target region, it is possible to prevent the cells having stem cell properties, which contribute to the ability of producing tissues, from becoming dried during transfer to the target region. Accordingly, the cells having stem cell properties can maintain their functions to differentiate into cells that express various functions, and further produce biologically active substances such as cytokines so that they can function in a well-balanced manner. As a result, tissues can be smoothly produced after transplantation.

### [Modifications]

The third embodiment can be modified and implemented as described below.
- The inner peripheral surface of the side wall surrounding the storage portions 230 and 231 is not limited to a circular cylindrical shape, but may also be a polygonal or elliptical cylindrical shape. That is, in the cross-section perpendicular to the extending direction of the needle 210, the region surrounded by the side wall may not necessarily have a circular shape. In this case, the maximum width of the region surrounded by the side wall in the above cross-section is the maximum width D.
- All the configurations described in the first embodiment, the second embodiment, and the modifications thereof can be applied to the needle 210A and the filter unit 20 of the first example. In other words, a region surrounded by the side wall of the internal flow channel from an intermediate position, where the filter unit 20 made of the fibrous member 21 is disposed, to the opening 13 can retain the graft as a storage portion. In the case where the flow channel cross-sectional area of the internal flow channel varies in the storage portion such that the storage portion has a nonconstant diameter in the extending direction of the needle 210, the largest diameter is the maximum width D.
- The needle 210B of the second example may be a member separately formed from the support 250. Further, the flow channel cross-sectional area of the internal flow channel 215 may vary to have three or more levels. In other words, a region surrounded by the side wall of the internal flow channel from an intermediate position, where the flow channel cross-sectional area varies, to the opening 13, can retain the graft as a storage portion. In the case where the flow channel cross-sectional area of the internal flow channel varies in the storage portion such that the storage portion has a nonconstant diameter in the extending direction of the needle 210, the largest diameter is the maximum width D.
- As examples of the storage portion, the above embodiment has described the first example, in which the storage portion is formed by providing the fibrous member, and the second example, in which the storage portion is formed by changing the flow channel cross-sectional area of the internal flow channel. Without being limited to these, the proximal end of the storage portion may have a structure that allows the protective liquid to pass through but blocks the graft. In other words, the storage portion may be configured such that a region surrounded by the side wall of the internal flow channel from an intermediate position to the opening 13 can store the liquid material entering through the opening 13 while allowing the graft contained in the liquid material to be retained in the region.

### [Supplementary Notes]

The measure for solving the foregoing problems embraces the following items as technical ideas derived from the third embodiment and the modification thereof.

### (Item 11)

A cell transplantation device configured to place a graft containing cells into a target region in a living body, the cell transplantation device including:
a needle extending in one direction, the needle having a flow channel defined therein and an opening at a tip of the needle, wherein
a region surrounded by a side wall of the flow channel from an intermediate position in the flow channel to the opening serves as a storage portion that stores a liquid material entering through the opening while allowing the graft contained in the liquid material to be retained in the region, and a length of the storage portion in the extending direction of the needle is larger than a maximum width of the storage portion in a direction perpendicular to the extending direction of the needle.

### (Item 12)

The cell transplantation device according to the item 11, wherein the inner peripheral surface of the side wall in the storage portion has a cylindrical shape.

### (Item 13)

The cell transplantation device according to the item 11 or 12, wherein the cell transplantation device is used to place the graft containing cells having stem cell properties into the target region.

### (Item 14)

The cell transplantation device according to any one of the items 11 to 13, the cell transplantation device including a plurality of needles.

### (Item 15)

The cell transplantation device according to any one of the items 11 to 14, wherein the inner peripheral surface of the side wall in the storage portion has a surface layer that suppresses evaporation of liquid contained in the liquid material.

### (Fourth Embodiment)

In a conventional transplantation method, in which an incision is made in the transplantation site with a scalpel, and then groups of cells in a culture vessel are picked up one by one with tweezers or the like and transferred to a target region, it is required to exchange the tools used and frequently repeat operations of transferring the groups of cells. In particular, when transplantation is performed for hair regeneration, a large number of groups of cells are required to be transplanted, which requires great effort. The excessive load required for the transplantation may pose a significant issue in the widespread use of the above transplantation technique.

An object of a fourth embodiment is to provide a cell transplantation device that enables smooth transplantation of cells while further improving the efficiency of transplantation.

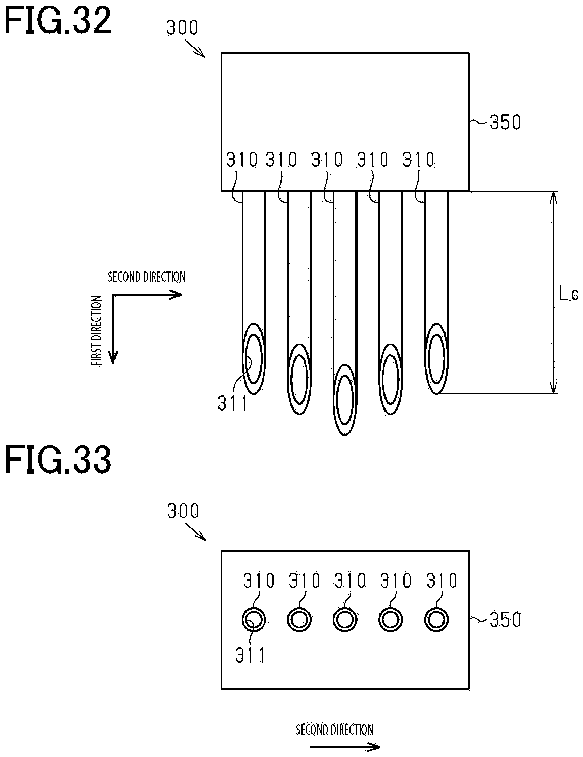

With reference to Figs. 32 to 36, a cell transplantation device of the fourth embodiment will be described. The same elements as those in the first embodiment are denoted by the same reference signs, and the description thereof is omitted.

### [Cell Transplantation Device]

As shown in Fig. 32, a cell transplantation device 300 includes a plurality of transplantation needles 310, and a support 350 that supports the transplantation needles 310. The transplantation needle 310 has a tubular shape extending in one direction, and has an opening 311 at a tip region. In other words, the transplantation needle 310 has a hollow needle shape. The outer shape of the transplantation needle 310 is not specifically limited as long as it has the tip region that can puncture a transplant target site in a living body. For example, the tip region of the transplantation needle 310 may be sharpened by truncating a cylindrical shape obliquely relative to the extending direction.

The plurality of transplantation needles 310 extend in a common direction, and are supported by one support 350. The support 350 may be formed as a separate member from the transplantation needles 310 or may be formed integrally with the transplantation needles 310. The outer shape of the support 350 is not specifically limited.

When the support 350 is a member formed separately from the transplantation needles 310, the support 350 supports the transplantation needles 310, surrounding the outer periphery of a portion of the transplantation needles 310 which is continuous from the tip region of the transplantation needles 310. For example, the transplantation needles 310 may extend through holes formed in the support 350 whereby they are assembled to the support 350. The tip region and a region near the tip region of the transplantation needles 310 protrude from the support 350 in the extending direction of the transplantation needles 310.

When the support 350 is formed integrally with the transplantation needles 310, the transplantation needles 310 protrude from a surface of the support 350.

The plurality of transplantation needles 310 are arranged in a second direction perpendicular to a first direction, which is an extending direction of the transplantation needle 310. Preferably, the openings 311 of the plurality of transplantation needles 310 are oriented in the same direction. The number of transplantation needles 310 is not specifically limited, and for example, may be approximately 5 to 10 when groups of cells that contribute to hair growth or hair restoration are transplanted.

The transplantation needles 310 include the transplantation needles 310 having tips whose positions are not aligned with each other in the first direction. The tip of the transplantation needle 310 is a point located at the most distal position in the first direction in the tip region of the transplantation needle 310. The tips of the plurality of transplantation needles 310 are arranged on a virtual plane different from a plane perpendicular to the first direction. The virtual plane may be a curved plane or a plane formed by bending a plane one or more times.

In the above configuration, when the surface of the support 350 is perpendicular to the first direction, lengths Lc of the portions of the transplantation needles 310 protruding from the surface of the support 350 are not uniform among the plurality of transplantation needles 310.

Specifically, from the transplantation needle 310 located at the center in the second direction toward the transplantation needle 310 located at the end in the second direction, the length Lc gradually decreases. In other words, among the plurality of transplantation needles 310, the transplantation needle 310 located at the center in the second direction has a largest length protruding from the support 350, and the transplantation needles 310 located closer to both ends in the second direction have a smaller length.

The portion of the transplantation needles 310 protruding from the support 350 include at least a portion that penetrates a target region in the living body at the time of transplantation. When the target region is an intradermal or subcutaneous site, the length Lc of the transplantation needle 310 is, for example, preferably 0.1 mm or more and 10 mm or less.

Further, when the target region is an intradermal or subcutaneous site, a difference between the largest length Lc and the smallest length Lc among the plurality of transplantation needles 310, that is, a distance between the tip of the transplantation needle 310 which is longest in the first direction and the tip of the transplantation needle 310 which is shortest in the first direction is preferably selected from the range of 0.1 mm or more and 10.0 mm or less. The above difference can be set according to the type of the living body to be transplanted or the site to be transplanted. It is preferred to increase the difference as the skin is more stretchable. For example, for a transplantation site having a hard skin such as a swine skin, the above difference is set to 0.1 mm, and for a transplantation site having a stretchable skin such as a mouse skin, the above difference is set to 10.0 mm.

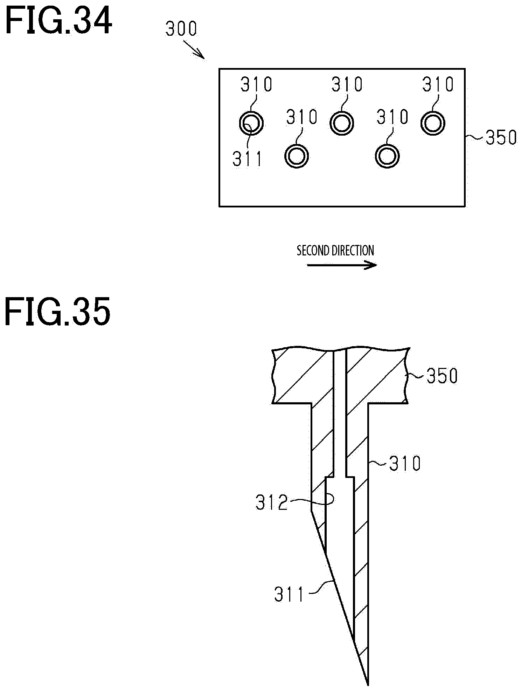

As viewed in a direction parallel to the first direction, the plurality of transplantation needles 310 may be arrayed in one row as shown in Fig. 33, or two rows as shown in Fig. 34. The plurality of transplantation needles 310 may be arranged at positions not aligned with each other in the second direction. According to a configuration in which the transplantation needles 310 are arrayed in two rows, when the transplantation needles 310 are inserted into the transplantation site, the transplantation site is pulled in a plurality of directions by the plurality of transplantation needles 310 that press the transplantation site. Accordingly, the surface of the transplantation site can be easily stretched. This facilitates puncturing of the transplantation site by the transplantation needles 310.

When the transplantation needles 310 are arrayed in two rows, it is preferred that the transplantation needles 310 are alternately located in these rows in the second direction. With this configuration, distances between the adjacent transplantation needles 310 are prevented from being non-uniform among the plurality of transplantation needles 310. Accordingly, for example, when the plurality of transplantation needles 310 are advanced in the living body, a pressing force tends to be uniformly applied to the transplantation needles 310. Further, the distance between the adjacent transplantation needles 310 in each row may or may not be equal to that in the other row.

When the plurality of transplantation needles 310 are arrayed in two rows, the positions of the tips of the transplantation needles 310 in the first direction may be constant or may not be constant in each row.

In transplantation of a group of cells that contributes to hair growth or hair restoration, the arrangement of the group of cells in a direction across the surface of the skin, that is, the arrangement of hairs that grow at positions where the group of cells are transplanted is substantially determined according to the arrangement of the plurality of transplantation needles 310. For example, in transplantation of aggregates of cells such as hair follicle germs that contribute to hair growth or hair restoration, the density of the transplantation needles 310 per unit area is preferably 1/cm² or more and 400/cm² or less, and more preferably 20/cm² or more and 100/cm² or less. When the density of the transplantation needles 310 is no less than the above lower limit, the density of hairs that grow from the hair follicle germs will provide sufficient hair density. When the density of the transplantation needles 310 is no more than the above upper limit, nutrients are easily distributed to the groups of cells that are positioned in the skin at the same density as that of the transplantation needles 310. Thus, formation of hair follicles and hairs are suitably performed.

Further, in transplantation of groups of cells that contribute to hair growth or hair restoration, an interval which is a center-to-center distance between two closest needles 310 is preferably 1 mm or more and 4 mm or less. When the interval is no less than the above lower limit, the cell transplantation device 300 can be easily produced. When the interval is no more than the above upper limit, the density of hairs that grows from the transplanted groups of cells will provide sufficient hair density.

When the graft is a group of cells that contributes to hair growth or hair restoration, which is a cell aggregate such as spheroids and germs, a diameter of a sphere that approximates the group of cells, which is the graft, that is, a diameter of a minimum circumscribed sphere of the group of cells is approximately from 0.1 mm to 1.0 mm. In this case, the opening 311 preferably has an opening area of 5,000 µm² or more and 1,200,000 µm² or less. The shape of the opening 311 is not specifically limited, and may be, for example, a circular shape or an elliptical shape. When the transplantation needle 310 has a hollow cylindrical shape, the inner diameter of the transplantation needle 310 at the opening 311 is preferably 80 µm or more and 1,200 µm or less. Since the opening 311 is not too small for the graft as long as the opening area and the inner diameter are no less than the above lower limit, an opening 311 having a sufficient size for the graft smoothly flowing in and out of the opening 311 can be achieved. In addition, since the opening 311 is not too large relative to the graft as long as the opening area and the inner diameter are no more than the above upper limit, an opening 311 that allows grafts to flow in and out one by one can be achieved. The size of the opening 311 can be appropriately selected from a size that allows the graft to pass through the inner flow channel of the transplantation needle 310 while having a size that is sufficient to prevent the graft from being in contact with the inner flow channel.

As shown in Fig. 35, the transplantation needle 310 includes an internal flow channel 312 defined therein, which is the flow channel that communicates with the opening 311. The internal flow channel 312 may have any structure as long as it allows the liquid material containing the graft and its protective liquid to enter through the opening 311.

It is preferred that the internal flow channel 312 is provided with a structure that blocks the graft at an internal position in the internal flow channel 312 so that the graft contained in the liquid material does not flow toward the proximal end of the transplantation needle 310. For example, the internal flow channel 312 may have the flow channel cross-sectional area that changes at an intermediate position in the internal flow channel 312 so that the graft does not flow toward the proximal end of the transplantation needle 310 via the position where the flow channel cross-sectional area changes. Fig. 35 illustrates an example in which the flow channel cross-sectional area of the internal flow channel 312 varies. Alternatively, a structure such as a filter or a mesh formed of a fibrous member may be provided to intersect the internal flow channel 312 at an intermediate position in the internal flow channel 312 so that the graft does not flow toward the proximal end of the transplantation needle 310 via the structure.

Due to such a structure being provided, a region of the transplantation needle 310 from the intermediate position in the internal flow channel 312 to the opening 311 serves as a storage portion that stores the graft. Since the graft is retained in the transplantation needle 310 in a region close to the tip of the transplantation needle 310, transplantation can be smoothly performed.

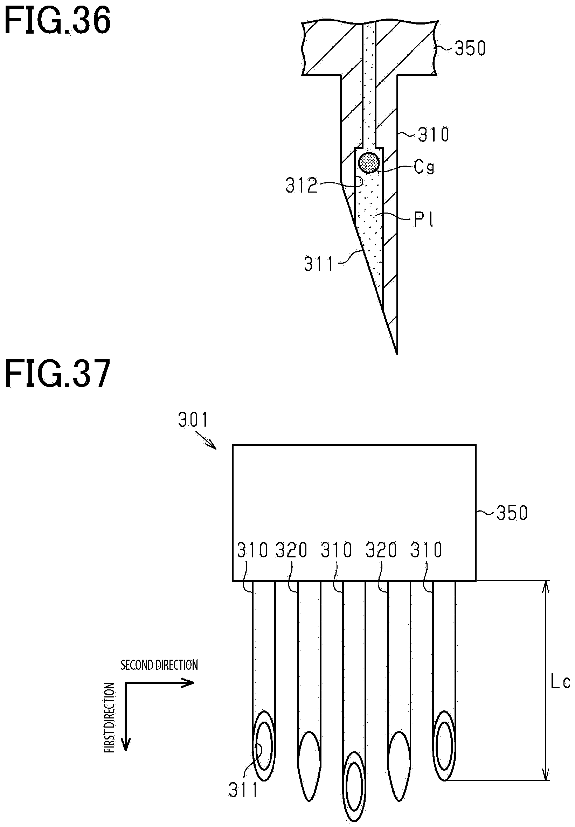

Fig. 36 illustrates an example in which the graft Cg is stored in the transplantation needle 310. The graft Cg is aspirated together with the protective liquid Pl into the internal flow channel 312 through the opening 311. In this process, the opening 311 functions as an inlet port through which the graft Cg is taken into the transplantation needle 310.

For example, the graft Cg and the protective liquid Pl are held in a container such as a culture vessel, and collected from the container into the transplantation needle 310 through the opening 311. In collection of the graft Cg, for example, a mechanism connected to the transplantation needle 310 applies suction to the inside of the internal flow channel 312 to aspirate the protective liquid Pl and the graft Cg into the internal flow channel 312 through the opening 311. The properties of the protective liquid Pl are the same as those in the first embodiment.

In collection of the grafts Cg, the cell transplantation device 300 is aligned with the container in which the grafts Cg are held so that the respective transplantation needles 310 are directed to the corresponding grafts Cg. Accordingly, the grafts Cg can be collected into the corresponding transplantation needles 310 in a single operation. Preferably, each graft Cg is collected into a respective transplantation needle 310.

When the cell transplantation device 300, in which the graft Cg and the protective liquid Pl are stored, is transferred to a transplantation site such as the skin of the living body, the transplantation needle 310 is inserted into the transplantation site. Accordingly, the opening 311 is positioned at the transplant target region. When an object to be transplanted is a group of cells that contributes to hair growth or hair restoration, the target region is at least one of an intradermal site and subcutaneous site. Then, the graft Cg together with the protective liquid Pl is ejected from the transplantation needle 310 through the opening 311. Accordingly, the graft Cg is placed at the target region. In this process, the opening 311 functions as an outlet port through which the graft Cg is ejected toward the target region. In ejection of the graft Cg, pressure may be applied to the inside of the internal flow channel 312. For example, a mechanism connected to the transplantation needle 310 injects air into the internal flow channel 312 to apply pressure to the inside of the internal flow channel 312.

As the grafts Cg are ejected from the respective transplantation needles 310, the plurality of grafts Cg can be placed at the target region in a single operation. Then, the transplantation needles 310 are removed from the transplantation site.

Materials for forming the transplantation needle 310 are not specifically limited. For example, the transplantation needle 310 may be made of silicon, metal materials such as stainless steel, titanium, cobalt-chromium alloy, and magnesium alloy, or may be made of polymer materials such as commodity plastics, medical grade plastics, and plastics for cosmetic products. Examples of the polymer materials include polyethylene, polypropylene, polystyrene, polyamide, polycarbonate, cyclic polyolefin, polylactic acid, polyglycolic acid, polycaprolactone, acrylic, urethane resin, aromatic polyether ketone, epoxy resin, and copolymer materials of these resins. Further, the transplantation needle 310 may be made of ionizing radiation curable materials such as polyacrylic, and thermosetting materials such as urethane and epoxy. Materials for forming the support 350 are also not specifically limited, and the support 350 may be made of, for example, materials described above as the materials for the transplantation needle 310. The transplantation needle 310 and the support 350 may be made of the same materials or different materials. Further, the transplantation needle 310 and the support 350 may also be made of a combination of two or more materials.

### [Advantageous Effects]

According to the transplantation method using the cell transplantation device 300, in which the grafts Cg can be collected from the container and placed in the transplant target region continuously using a single device, smooth transplantation of cells can be performed. Further, since the cell transplantation device 300 includes the plurality of transplantation needles 310, it is possible to collect and place the grafts Cg in a single operation. Therefore, the efficiency of transplantation is further improved.

Since a tissue such as a skin or an organ of a living body has elasticity, it is stretched when pressed with the transplantation needles 310. The degree of stretch is different depending on the position relative to the plurality of transplantation needles 310. When the positions of the tips of the plurality of transplantations needles 310 are aligned in the first direction, the lengths inserted into the transplantation site may be non-uniform among the transplantation needles 310, which makes it difficult to place the respective grafts Cg at desired depths. On the other hand, according to the cell transplantation device 300 of the present embodiment, in which the plurality of transplantation needles 310 include the transplantation needles 310 having tips whose positions are not aligned with each other in the first direction, the positions of the tips of the transplantation needles 310 can be set according to the target that undergoes transplantation. Accordingly, the transplantation needles 310 can be easily inserted into the target at desired lengths. Therefore, each of the grafts Cg can be easily placed at a desired depth.

Specifically, the inventors have found that, in transplantation performed to the skin, the degree of stretch of the skin is largest at the position corresponding to the transplantation needle 310 at the center in the second direction, and smaller at the position corresponding to the transplantation needle 310 closer to the end. When the transplantation needle 310 is pressed against a portion of the skin that is highly stretchable, the surface of the skin stretches and is displaced in a direction away from the transplantation needle 310, which makes it difficult to deeply puncture the skin by the transplantation needle 310.

Therefore, in a configuration in which the transplantation needle 310 at the center in the second direction has a largest protruding length and the transplantation needle 310 located closer to the end has a smaller protruding length, it is possible to easily ensure the insertion length of the transplantation needle 310 even when the transplantation needle 310 is inserted into a stretchable portion of the skin. As a result, the lengths of the transplantation needles 310 inserted into the skin can be prevented from being non-uniform among the plurality of transplantation needle 310, and each of the grafts Cg can be easily placed at a desired depth.

As described above, according to the cell transplantation device of the fourth embodiment, in which the grafts Cg can be collected from the container and placed in the transplant target region continuously using a single device, smooth transplantation of cells can be performed. Furthermore, according to the cell transplantation device of the fourth embodiment, the following effects can be achieved.

(4-1) Since the cell transplantation device 300 includes the plurality of transplantation needles 310, a plurality of grafts Cg can be collectively transplanted, improving the efficiency of transplantation.

(4-2) The plurality of transplantation needles 310 include at least one transplantation needle 310 having a tip whose position is not aligned with that of others in the first direction. Accordingly, even when the transplantation site is stretchable, the positions of the tips of the transplantation needles 310 can be set according to the stretchability so that the grafts Cg can be placed at desired depths.

(4-3) Among the plurality of transplantation needles 310, the tip of the transplantation needle 310 located at the center in the second direction protrudes more than the tips of the other transplantation needles 310. Accordingly, in transplantation of the grafts Cg into the skin, the lengths of the plurality of transplantation needles 310 inserted into the skin can be prevented from being non-uniform. As a result, each of the grafts Cg can be easily placed at a desired depth.

(4-4) When the plurality of transplantation needles 310 are arrayed in one row or two rows in the second direction, the positions of the tips of the transplantation needles 310 can be designed according to the stretchability of the transplantation site so that the grafts Cg can be placed at desired depths. Therefore, a cell transplantation device 300 that is able to place a plurality of grafts Cg at desired depths can be appropriately achieved.

### (Fifth Embodiment)

With reference to Fig. 37, a cell transplantation device of a fifth embodiment will be described. As in the fourth embodiment, an object of the fifth embodiment is to provide a cell transplantation device that enables smooth transplantation of cells while further improving the efficiency of transplantation. The following description focuses on differences between the fifth embodiment and the fourth embodiment, and configurations which are the same as that of the fourth embodiment will be referred to by the same reference numbers and the description thereof will be omitted.

While all the needles included in the cell transplantation device 300 of the fourth embodiment are transplantation needles 310, a plurality of needles included in a cell transplantation device 301 of the fifth embodiment, as shown in Fig. 37, include a plurality of transplantation needles 310 and one or more non-transplantation needles 320.

The non-transplantation needle 320, having no opening as an inlet port and outlet port for a graft, does not take in a graft. For example, the non-transplantation needle 320 may be a solid structure having a rod-like shape.

The non-transplantation needle 320 extends in the same direction as the transplantation needle 310, that is, in the first direction. The outer shape of the non-transplantation needle 320 is not specifically limited as long as it has the tip region that can puncture a transplantation site. The non-transplantation needle 320 may be formed of the same material as that of the transplantation needle 310, or a different material from that of the transplantation needle 310.

When the materials of the transplantation needle 310 and the non-transplantation needle 320 are different from each other, for example, a metal material may be used for the transplantation needle 310 and a polymer material may be used for the non-transplantation needle 320 from among the materials described as the materials for the transplantation needle 310 of the fourth embodiment. Alternatively, a polymer material may be used for the transplantation needle 310 and a metal material may be used for the non-transplantation needle 320. Due to the different materials used for the transplantation needle 310 and the non-transplantation needle 320, the transplantation needle 310 and the non-transplantation needle 320 may be, for example, different in the degree of friction against the living body, and thus may be different in ease of insertion into the living body. Specifically, for example, using a roughly polished metal material or a low density resin material, which forms a microtexture on the surface, can increase the friction between the living body and the needle.

The non-transplantation needle 320 has a configuration that can more easily puncture the transplantation site than the transplantation needle 310 does. In other words, the non-transplantation needle 320 has an ability to puncture the transplantation site better than the transplantation needle 310. For example, the tip region of the non-transplantation needle 320 is more pointed than that of the transplantation needle 310. Alternatively, the non-transplantation needle 320 may have a puncture ability higher than that of the transplantation needle 310 due to the difference between the material of the non-transplantation needle 320 and the material of the transplantation needle 310.

The non-transplantation needle 320, which is not required to have a structure for storing the graft, has less restriction in terms of the shape and material than the transplantation needle 310. Therefore, providing the non-transplantation needle 320 with a puncture ability higher than that of the transplantation needle 310 is easier than increasing the puncture ability of the transplantation needle 310.

As in the fourth embodiment, the plurality of needles including the transplantation needles 310 and the non-transplantation needles 320 are arrayed in one row or two rows, and their positions are not aligned with each other in the second direction. When the plurality of needles are arrayed in two rows, the rows may include a row in which only the transplantation needles 310 are arrayed and a row in which only the non-transplantation needles 320 are arrayed, or may include a row in which both the transplantation needles 310 and the non-transplantation needles 320 are arrayed.

Preferably, the non-transplantation needles 320 are uniformly distributed among the transplantation needles 310. For example, the transplantation needles 310 and the non-transplantation needles 320 may be alternately positioned in the second direction. Alternatively, one or more non-transplantation needles 320 may be positioned between the consecutively transplantation needles 310 in the second direction. Further alternatively, a predetermined number of transplantation needles 310 and a predetermined number of non-transplantation needles 320 may be alternately positioned in the second direction.

In the fifth embodiment, the positions of the tips of the plurality of needles including the transplantation needles 310 and the non-transplantation needles 320 are not aligned with each other in the first direction. The tip of the transplantation needle 320 is a point located at the most distal position in the first direction in the tip region of the transplantation needle 320. Regardless of whether the needle is a transplantation needle 310 or a non-transplantation needle 320, the length Lc of a portion protruding from the surface of the support 350 gradually decreases from the needle located at the center in the second direction toward the needle located at the end in the second direction.

Depending on the number and arrangement of the transplantation needles 310 and the non-transplantation needles 320, the positions of the tips of the plurality of transplantation needles 310 may be aligned or may not be aligned in the first direction. Similarly, the positions of the tips of the plurality of non-transplantation needles 320 may be aligned or may not be aligned in the first direction.

In transplantation of groups of cells that contribute to hair growth or hair restoration, the density and interval of the plurality of needles may be set in the same range as that in the fourth embodiment regardless of whether the needle is a transplantation needle 310 or a non-transplantation needle 320.

### [Advantageous Effects]

According to the cell transplantation device 301 of the fifth embodiment, in which the plurality of needles include needles having tips whose positions are not aligned with each other in the first direction, the positions of the tips of the needles can be set so that the needles can be easily inserted into the transplantation site at desired lengths. That is, since the transplantation needles 310 tend to be inserted into the transplantation site at desired lengths, the graft can be easily placed at desired depth compared with the configuration in which no non-transplantation needle 320 is included and the positions of the tips of the plurality of transplantation needles 310 are aligned.

In a configuration in which the transplantation needle 310 at the center in the second direction has a largest protruding length and the transplantation needle 310 located closer to the end has a smaller protruding length, in transplantation performed to the skin, it is possible to easily ensure the insertion length of the transplantation needle 310 even when the transplantation needle 310 is inserted into a stretchable portion of the skin. As a result, the lengths of the transplantation needles 310 inserted into the skin can be prevented from being non-uniform among the plurality of transplantation needle 310, and each of the grafts can be easily placed at a desired depth.

Since the non-transplantation needle 320 has a puncture ability higher than that of the transplantation needle 310, the non-transplantation needle 320 tends to puncture the transplantation site before the transplantation needle 310 does even when the transplantation site is stretchable. Therefore, since the non-transplantation needle 320 punctures the transplantation site before the transplantation needle 310 does, the non-transplantation needle 320, which is pressed against the transplantation site, prevents the transplantation site from being stretched. Accordingly, the transplantation needles 310 located adjacent to the non-transplantation needle 320 can easily puncture the transplantation site.

As described above, according to the cell transplantation device of the fifth embodiment, in which the grafts can be collected from the container and placed in the transplant target region continuously using a single device, smooth transplantation of cells can be performed. Furthermore, according to the cell transplantation device of the fifth embodiment, the following effects can be achieved in addition to the effect of (4-1) in the fourth embodiment and the effect obtained by replacing the transplantation needles 310 in (4-2) to (4-4) with the needles including the transplantation needles 310 and the non-transplantation needles 320.

(4-5) Since the plurality of needles in the cell transplantation device 301 includes the non-transplantation needles 320, the non-transplantation needles 320 can prevent the transplantation site from being stretched so that the transplantation needles 310 can easily puncture the transplantation site.

(4-6) Since the tip region of the non-transplantation needles 320 is more pointed than that of the transplantation needles 310, the non-transplantation needles 320 tend to more easily puncture the transplantation site than the transplantation needles 310 do. Therefore, since the non-transplantation needles 320 puncture the transplantation site before the transplantation needles 310 do, the non-transplantation needles 320 can prevent the transplantation site from being stretched so that the transplantation needles 310 located adjacent to the non-transplantation needles 320 can easily puncture the transplantation site.

(4-7) In a configuration in which one or more transplantation needles 310 and one or more non-transplantation needles 320 are alternately positioned in the second direction, it is possible to prevent the non-transplantation needles 320 from being non-uniformly distributed among the transplantation needles 310. Therefore, the transplantation needles 310 can easily puncture a wide area of the transplantation site.

(4-8) When the transplantation needle 310 and the non-transplantation needle 320 are made of materials different from each other, the transplantation needle 310 and the non-transplantation needle 320 can be made of a material suitable for each function. For example, the transplantation needle 310 and the non-transplantation needle 320 may have different puncture abilities, or may be different in the degree of friction against the living body.

### [Modifications]

The fourth and fifth embodiments can be modified and implemented as described below. - In both the fourth and fifth embodiments, the protruding length of the plurality of needles in the first direction is not necessarily set to decrease from the needle located at the center in the second direction toward the needle located at the end, and may also be set according to the tendency of stretchability of the transplantation site. For example, the protruding length of the plurality of needles in the first direction may be set to increase from the needle located at the center in the second direction toward the needle located at the end. Further, the plurality of needles may include needles whose tip positions in the first direction coincide with each other, and may include, for example, two or more needles with a tip having a largest protruding length. The stretchability of the transplantation site often varies continuously in a region that undergoes transplantation. For this reason, in order to set the protruding lengths of the plurality of needles according to the tendency of stretchability of the transplantation site, the number of needles whose tip positions in the first direction coincide with each other is preferably 2 or less, and the number of needles with a tip having a largest protruding length is also preferably 2 or less.

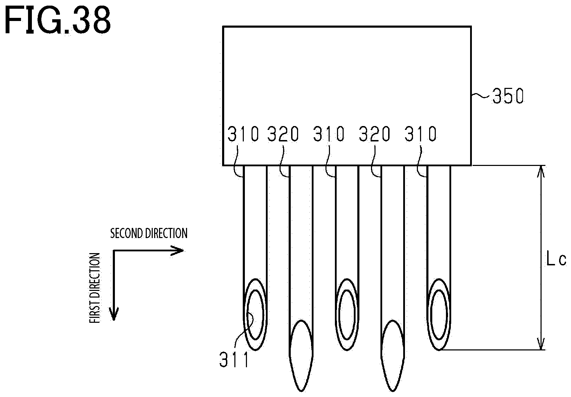

- The needle may be displaceable in the first direction relative to the support 350, and the tip position of each needle may also be displaceable. With this configuration, the protruding length of the plurality of needles can be easily adjusted according to the stretchability of the transplantation site.
- When the plurality of needles include the transplantation needles 310 and the non-transplantation needles 320, the tip of the non-transplantation needles 320 may protrude more than the tip of the transplantation needles 310 in the first direction as shown in Fig. 38. With this configuration, since the non-transplantation needles 320 puncture the transplantation site before the transplantation needles 310 do, the non-transplantation needles 320 can prevent the transplantation site from being stretched so that the transplantation needles 310 located adjacent to the non-transplantation needles 320 can easily puncture the transplantation site. In this case, since the tip of the non-transplantation needles 320 protrude more than the tip of the transplantation needles 310, the non-transplantation needles 320 can more easily puncture the transplantation site. Accordingly, the puncture ability of the non-transplantation needles 320 due to the shape or material thereof may be the same as that of the transplantation needles 310. Further, the positions of the tips of the plurality of transplantation needles 310 may be aligned or may not be aligned in the first direction.
- As long as the transplantation needle 310 includes the opening 311 in the tip region, the opening 311 functioning as an inlet port and an outlet port for the graft, the other structures may be different from the above embodiments. For example, the transplantation needle 310 may have a plurality of openings 311, or the internal flow channel 312 may extend in a direction inclined to the first direction. Furthermore, the outer shape of the transplantation needle 310 may be, but is not limited to, a cylindrical, conical, or prismatic shape. Moreover, a shape of the space defined in the transplantation needle 310 may be, but is not limited to, a cylindrical, conical, or prismatic shape.
- As long as the non-transplantation needle 320 includes no opening 311 that functions as an inlet port and an outlet port for the graft, the other structures may be different from the above embodiments. For example, the non-transplantation needle 320 may have an opening having a function different from that of the above opening 311 on the side face, or may have a flow channel defined therein. Furthermore, the outer shape of the non-transplantation needle 320 may be, but is not limited to, a cylindrical, conical, or prismatic shape.
- The arrangement of a plurality of needles is not limited to the array of one row or two rows arranged in the second direction, and may be arranged in any array.

### [Supplementary Notes]

The measure for solving the foregoing problems embraces the following items as technical ideas derived from the fourth and fifth embodiments and the modifications thereof.

### (Item 21)

A cell transplantation device configured to place a graft containing cells into a target region in a living body, the cell transplantation device including:
a plurality of needles extending in a first direction, wherein
the plurality of needles include the needle having a tip whose position is not aligned with that of others in the first direction, and a plurality of transplantation needles each having an opening in a tip region, the opening being provided as an inlet port through which the graft is taken into the needle and an outlet port through which the graft is ejected toward the target region.

### (Item 22)

The cell transplantation device according to the item 21, wherein the plurality of needles are arrayed in one row or two rows in a second direction perpendicular to the first direction.

### (Item 23)

The cell transplantation device according to the item 21 or 22, wherein the plurality of needles include a non-transplantation needle having no opening.

### (Item 24)

The cell transplantation device according to the item 23, wherein a tip region of the non-transplantation needle is more pointed than a tip region of the transplantation needle.

### (Item 25)

The cell transplantation device according to the item 23 or 24, wherein
the plurality of needles are arranged at positions not aligned with each other in a second direction perpendicular to the first direction, and
one or more of the transplantation needles and one or more of the non-transplantation needles are alternately positioned in the second direction.

### (Item 26)

The cell transplantation device according to any one of the items 23 to 25, wherein a tip of the non-transplantation needle protrudes more than a tip of the transplantation needle.

### (Item 27)

The cell transplantation device according to any one of the items 23 to 26, wherein the transplantation needle and the non-transplantation needle are made of materials different from each other.

### (Sixth Embodiment)

In transplantation, a graft stored in a container such as a culture vessel is transferred to a transplant target region in the living body. Since the graft is micro-sized, it is difficult to determine whether the graft is appropriately held by a tool or a device for transferring the graft only by visual inspection. When the graft is not appropriately held during transplantation, or inspection of the grafts as to whether they are appropriately held or not requires an effort, the efficiency of transplantation decreases.

A sixth embodiment is an embodiment of a cell transplantation system which includes a cell transplantation device, and an object of the sixth embodiment is to provide a cell transplantation system that enables smooth transplantation of cells while improving the efficiency in collecting cells from a container to place the cells into a target region.

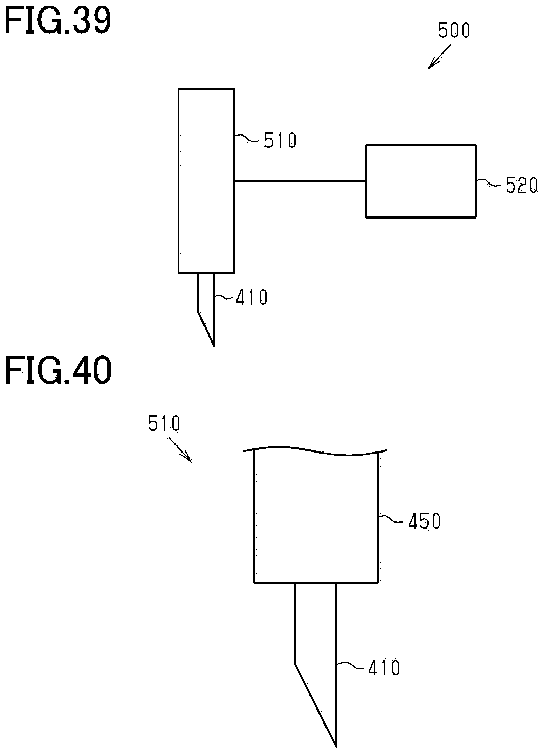

With reference to Figs. 39 to 51, a cell transplantation device of the sixth embodiment will be described.

### [Cell Transplantation System]

With reference to Fig. 39, an overall configuration of a cell transplantation system will be described. A cell transplantation system 500 includes a cell transplantation device 510 and a detector 520.

The cell transplantation device 510 includes a needle 410 having a tubular shape, which is configured to store a graft for transferring the graft to a transplant target region in a living body.

The detector 520 is electrically connected to the cell transplantation device 510, and detects when the graft is held by the needle 410 in response to an electric signal from the cell transplantation device 510. The detector 520 includes a circuit that processes the above electric signal.

### [Cell Transplantation Device]

With reference to Figs. 40 to 43, a detailed configuration of the cell transplantation device 510 will be described.

As shown in Fig. 40, the cell transplantation device 510 includes the needle 410, and a support 450 that supports the needle 410. The needle 410 has a tubular shape extending in one direction, in other words, a hollow needle shape. The outer shape of the needle 410 is not specifically limited as long as it has a tip that can puncture a transplant target site such as a skin in a living body. For example, the tip of the needle 410 may be sharpened by truncating a cylindrical shape obliquely relative to the extending direction.

The support 450 may be formed as a separate member from the needle 410 or may be formed integrally with the needle 410. The outer shape of the support 450 is not specifically limited.

When the support 450 is a member formed separately from the needle 410, the support 450 supports the needle 410, surrounding the outer periphery of a portion of the needle 410 which is continuous from the tip of the needle 410. For example, the needle 410 extends through a hole formed in the support 450 whereby it is assembled to the support 450. The tip and a region near the tip of the needle 410 protrude from the support 450 in the extending direction of the needle 410.

When the support 450 is formed integrally with the needle 410, the needle 410 protrudes from a surface of the support 450.

Regardless of whether the needle 410 and the support 450 are separate members or integrally formed, a portion of the needle 410 protruding from the support 450 includes at least a portion that penetrates a transplant target region in the living body.

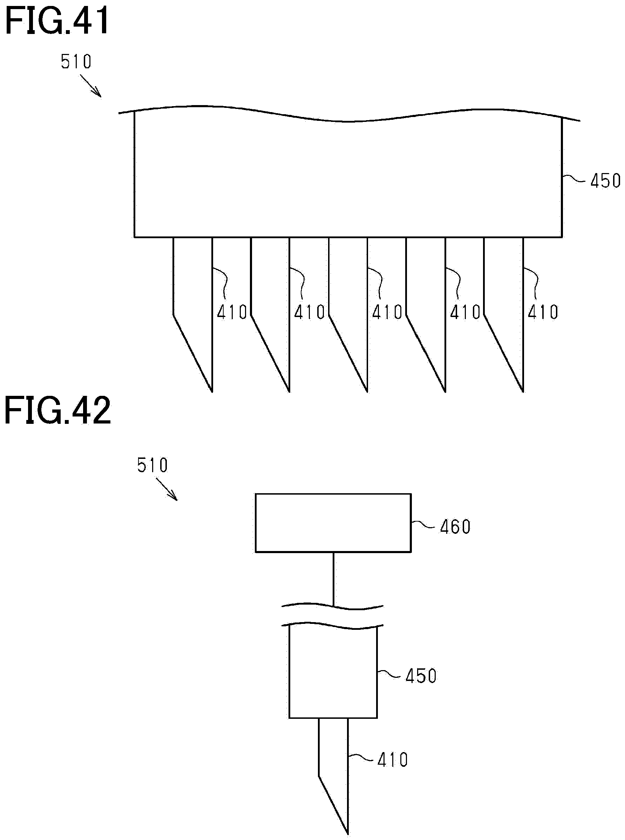

The number of needles 410 of the cell transplantation device 510 is not specifically limited. The cell transplantation device 510 may include a single needle 410 as shown in Fig. 40 or a plurality of needles 410 as shown in Fig. 41. When the cell transplantation device 510 includes a plurality of needles 410, the arrangement of the plurality of needles 410 is not specifically limited. The plurality of needles 410 may be regularly arranged or irregularly arranged. Further, when the cell transplantation device 510 includes a plurality of needles 410, it is preferred that all the needles 410 are collectively supported by a single support 450. That is, the plurality of needles 410 are preferably supported by a single support 450 to provide the cell transplantation device 510 having the integrally formed plurality of needles 410.

As shown in Fig. 42, the cell transplantation device 510 may include a suction-ejection unit 460 that assists in collecting and ejecting a graft into and from the needle 410. The suction-ejection unit 460 is configured to apply suction to a graft located outside the needle 410 so that the graft is taken into the needle 410 through an opening at the tip of the needle 410, and apply pressure to a graft located inside the needle 410 so that the graft is ejected from the needle 410 through the opening of the needle 410.

The suction-ejection unit 460 is not specifically limited as long as it has a mechanism capable of suction and ejection of a graft, and is connected to the needle 410 in a manner capable of such suction and ejection. For example, the suction-ejection unit 460 may be configured to aspirate a graft together with its protective liquid by sucking in air from the needle 410, and eject the graft together with the protective liquid by applying pressure to the inside of the needle 410.

Although Fig. 42 shows a configuration of the cell transplantation device 510 having a single needle 410, the cell transplantation device 510 having a plurality of needles 410 may also have the suction-ejection unit 460. The suction-ejection unit 460 may collectively aspirate the grafts into the plurality of needles 410 or independently aspirate the grafts into the individual needles 410. Similarly, the suction-ejection unit 460 may collectively eject the grafts from the plurality of needles 410 or independently eject the grafts from the individual needles 410.

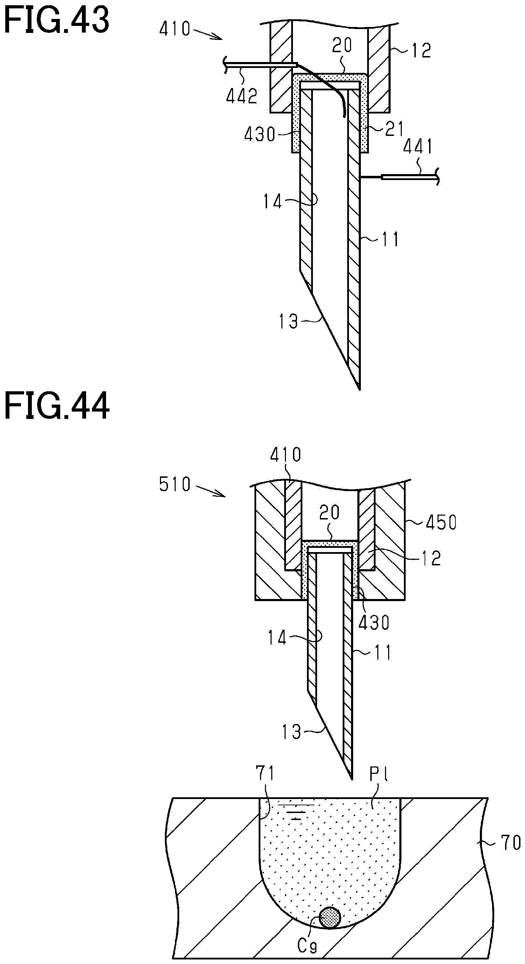

Next, a detailed structure of the needle 410 will be described. As shown in Fig. 43, the needle 410 has the internal flow channel 14 that communicates with the opening 13 at the tip, and a portion surrounding the region from an intermediate position in the internal flow channel 14 to the opening 13 serves as a holding portion 430 that holds a graft. The holding portion 430 prevents the graft from flowing from the holding portion 430 toward the proximal end of the needle 410, and stores a liquid material containing the graft and the protective liquid. As specific examples of the structure of the holding portion 430, the following description will be given of an example, in which the holding portion 430 is formed by providing a fibrous member. In this case, the needle 410 has the same configuration as that of the needle 10 in the first embodiment. Although the following drawings show a configuration of the cell transplantation device 510 having a single needle 410, the cell transplantation device 510 may also include a plurality of needles 410 as described above.

The needle 410 includes the first tube 11 having a tip of the needle 410 and a second tube 12 having a flow channel cross-sectional area larger than that of the first tube 11. In this case, the needle 410 is a separate member from the support 450. The tip of the first tube 11 has an opening 13. The second tube 12 is connected to a proximal end of the first tube 11. The internal flow channel 14 defined in the needle 410 extends in the extending direction of the needle 410, and communicates with the opening 13.

The needle 410 includes the filter unit 20 made of the fibrous member 21 at a position where the flow channel cross-sectional area of the needle 410 varies. The filter unit 20 and the fibrous member 21 have the same configurations as those in the first embodiment. The filter unit 20 intersects the internal flow channel 14 at an intermediate position in the internal flow channel 14. The filter unit 20 allows a protective liquid to pass through from the tip of the needle 410 toward the proximal end, and prevents the passage of a graft. For example, the filter unit 20 has a mesh form in which a plurality of fibrous members 21 intersect each other, and covers the proximal end of the first tube 11. A portion surrounding the region from an intermediate position in the internal flow channel 14 to the opening 13 serves as a holding portion 430 that retains a graft in this region. Further, in the extending direction of the needle 410, a length of the holding portion 430, that is, a length from the proximal end of the opening 13 to the filter unit 20 may be larger than the diameter of the graft.

In transplantation of aggregations of cells such as cell aggregates, which are groups of cells that contribute to hair growth or hair restoration, the number of cells included in a group of cells is approximately from 1 × 10³cells to 4 × 10⁴cells. In this case, the diameter of a sphere that approximates a group of cells, that is, the diameter of a minimum circumscribed sphere of a group of cells, is approximately from 100 µm to 1000 µm.

Accordingly, the length of the holding portion 430 is preferably 1 mm or more. Further, in ejection of the graft from the opening 13, if the amount of the protective liquid ejected together with the graft excessively increases, the transplant target region may be overfilled with the protective liquid and the placement of the graft may be hindered. From this viewpoint, the length of the holding portion 430 is preferably 50 mm or less.

The cell transplantation device 510 includes a first conductor 441 and a second conductor 442. The first conductor 441 and the second conductor 442 are each electrically connected to the detector 520. The first conductor 441 and the second conductor 442 are each embodied as, for example, a conductive wire covered with an insulating sheath, and have a conductive end portion exposed from the insulating sheath.

The end portion of the first conductor 441 is located outside the needle 410 and in contact with an outer peripheral surface of the first tube 11 that constitutes the holding portion 430 before and after the graft is held in the holding portion 430. The end portion of the second conductor 442 is located inside and near the proximal end of the first tube 11, that is, near the filter unit 20. The end portion of the second conductor 442 is separated from an inner peripheral surface of the first tube 11 before the graft is held in the holding portion 430, and is pressed against the inner peripheral surface of the first tube 11 by the graft when the graft is held in the holding portion 430. The second conductor 442 extends from the inside of the second tube 12 to the inside of the first tube 11 via the filter unit 20, for example, obliquely intersecting the internal flow channel 14. For example, the second conductor 442 penetrates through the needle 410 or is lead out from the proximal end of the needle 410 to the outside of the needle 410, and is connected to the detector 520.

In the first tube 11 constituting the holding portion 430, at least a portion that is in contact with the end portion of the first conductor 441 and a portion that is in contact with the end portion of the second conductor 442 when the graft is held are made of an electrically conductive material and continuous with each other to form an electrically conductive portion. For example, the entire first tube 11 may be made of metal so that the entire first tube 11 forms an electrically conductive portion, or a portion near the proximal end of the first tube 11 may be made of metal to form an electrically conductive portion while the remaining portion may be made of resin. Materials of the second tube 12 are not specifically limited.

### [Cell Transplantation System]

Operation of the cell transplantation system 500 will be described in connection with a method of transplanting a graft using the cell transplantation device 510. The method of transplanting a graft includes a step of collecting a graft into the cell transplantation device 510, and a step of placing the graft into a transplant target region in the living body. In some of the drawings described below, the first conductor 441 and the second conductor 442 are omitted.

As shown in Fig. 44, the graft Cg together with the protective liquid Pl is stored in the tray 70 such as a culture vessel before it is collected into the cell transplantation device 510. The manner that the graft Cg is held in the tray 70 and the properties of the protective liquid Pl are the same as those in the first embodiment.

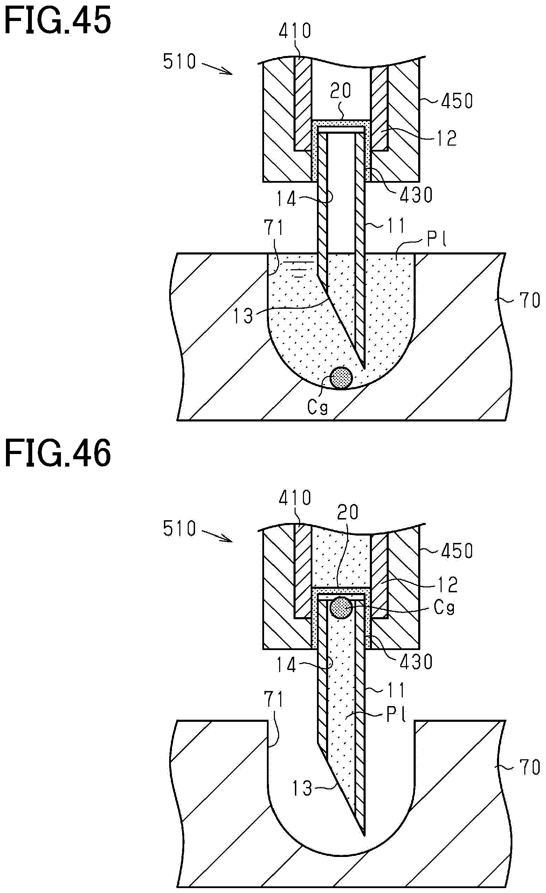

In the collection step, the cell transplantation device 510 aspirates the graft Cg into the needle 410 through the opening 13. More specifically, the tip of the needle 410 is directed toward the graft Cg stored together with the protective liquid Pl in the tray 70, and the graft Cg is aspirated together with the protective liquid Pl into the holding portion 430. For example, after the needle 410 is aligned with the recess 71 as shown in Fig. 44, the tip of the needle 410 is inserted into the recess 71 as shown in Fig. 45. Then, as the suction-ejection unit 460 is actuated as shown in Fig. 46, the graft Cg is aspirated together with the protective liquid Pl into the internal flow channel 14 through the opening 13.

The graft Cg that has entered the internal flow channel 14 through the opening 13 flows in the first tube 11 along with a flow of the protective liquid Pl toward the filter unit 20. Since the filter unit 20 allows the protective liquid Pl to pass through, the protective liquid Pl flows from the first tube 11 into the second tube 12 along with an aspiration flow, and further flows toward the proximal end of the needle 410. On the other hand, since the filter unit 20 blocks the graft Cg, the graft Cg is retained between the filter unit 20 and the opening 13. Thus, the graft Cg is held in the holding portion 430 and stored in the needle 410.

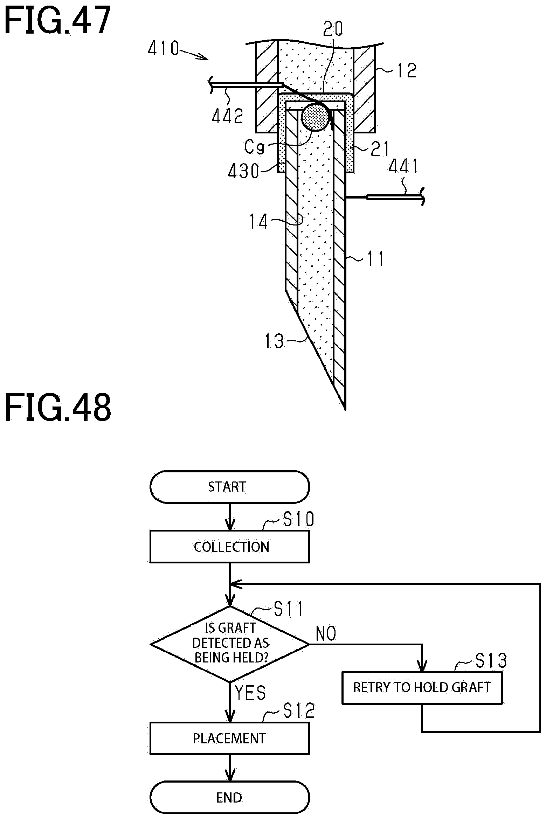

Fig. 47 illustrates the needle 410 in which the graft Cg is held in the holding portion 430. As shown in Fig. 47, the graft Cg that has entered the needle 410 flows along with a flow of the protective liquid Pl toward the filter unit 20. In this process, the graft Cg abuts against the second conductor 442 and displaces the second conductor 442. As a result, the end portion of the second conductor 442 comes into contact with the inner peripheral surface of the first tube 11 to cause the first conductor 441 and the second conductor 442 to be electrically connected to each other. The second conductor 442, which extends from the inside of the second tube 12 to the inside of the first tube 11 via the filter unit 20, obliquely intersecting the internal flow channel 14, facilitates the graft Cg to push the second conductor 442 and displace the second conductor 442 in the process of flowing toward the filter unit 20.

The detector 520 detects an electrical change in the holding portion 430, which is a change in electrical conduction indicating whether the first conductor 441 and the second conductor 442 are electrically connected or not, to detect when the graft Cg is held. For example, the detector 520 applies a voltage between the end portions of the first conductor 441 and the second conductor 442 connected to the detector 520, and detects that the first conductor 441 and the second conductor 442 are electrically connected when it detects an electric current flowing in the conductors 441 and 442. The detection of electrical conduction between the first conductor 441 and the second conductor 442 indicates that the graft Cg is held.

Fig. 48 is a flowchart illustrating a procedure performed by the cell transplantation system 500, focusing on a collection step. The collection step is performed in step S10 after the cell transplantation device 510 is positioned directed toward the tray 70. The collection step, which is a step of collecting the graft Cg into the needle 410, includes, for example, aspirating the graft Cg by actuating the suction-ejection unit 460.

Then, in step S11, it is determined whether the graft Cg is detected as being held in the holding portion 430. If the graft Cg is detected as being held (yes in step S11), the process proceeds to step S12, in which a placement step is performed. If the graft Cg is not detected as being held in a predetermined time after the collection step (no in step S11), a retry step is performed in step S13. As a reason why the graft Cg fails to be held in the holding portion 430, that is, the graft Cg fails to enter the needle 410, it is presumed that the graft Cg may be adhered to the outer peripheral surface of the needle 410 near the tip, or the graft Cg may be left in the tray 70. The retry step, which is a step for dealing with such a situation, includes, for example, vibrating the needle 410 or the tray 70, ejecting the protective liquid Pl from the needle 410 toward the tray 70, and applying suction again to the inside of the needle 410. After the retry step, the steps from step S11 onward are repeated.

The procedure shown in Fig. 48 may also be repeatedly performed according to an instruction from a control unit included in the cell transplantation system 500. For example, the control unit may include the detector 520, and, based on the detection result from the detector 520, transmit a control signal to the cell transplantation device 510 to perform a retry step when the graft Cg is not detected as being held.

The control unit may include, for example, hardware elements used in a computer, such as a CPU, a RAM, and a ROM, and software. The control unit may not necessarily execute all of the various processes by software. For example, the control unit may include an application-specific integrated circuit (ASIC), which is hardware specifically designed to execute at least part of the various processes. The control unit may include one or more dedicated hardware circuits such as ASICs, a microcomputer including one or more processors that operate according to the software which is a computer program, or a combination thereof.

Alternatively, the procedure shown in Fig. 48 may also be performed by a user operating the cell transplantation device 510. For example, when the detector 520 detects that the graft Cg is held, a display lamp or the like is lighted to notify the detection to the user. Upon receiving the notification, the user performs a placement step. Alternatively, if not receiving the notification, the user performs a retry step.

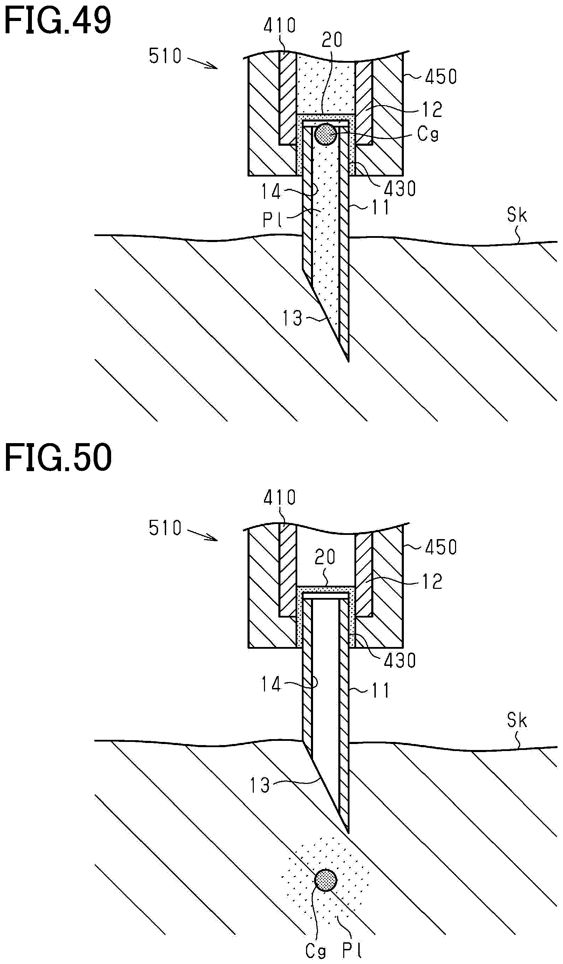

In the placement step, the cell transplantation device 510 is transferred to a transplant target region in the living body, and as shown in Fig. 49, the needle 410 is inserted into the target site, for example, skin Sk. Thus, the tip of the first tube 11 is advanced into the target site so that the opening 13 is positioned at the transplant target region. When an object to be transplanted is a group of cells that contributes to hair growth or hair restoration, the target region is at least one of an intradermal site and subcutaneous site.

As shown in Fig. 50, as the suction-ejection unit 460 is actuated, the graft Cg that has been collected into the first tube 11 is ejected together with the protective liquid Pl through the opening 13. Accordingly, the graft Cg is placed at the target region. Then, the needle 410 is removed from the target site.

Thus, transplantation of the graft Cg to the target region is completed.

According to the above transplantation method using the cell transplantation device 510, in which the grafts Cg can be collected from the tray 70 and placed in the transplant target region continuously using a single device, smooth transplantation of cells can be performed.

Since the detector 520 can detect when the graft Cg is held in the needle 410, the holding state of the graft Cg, that is, whether the graft Cg is held or not is easily recognized compared with a case of visual inspection, and the accuracy in the inspection of the holding state can be improved. Accordingly, it is possible to improve the efficiency in collection of the graft Cg from the tray 70, and more smoothly perform cell transplantation using the cell transplantation device 510.

Specifically, it is difficult to recognize the holding state of the graft Cg by visual inspection since the graft Cg and the needle 410 are micro-sized. In addition, when the needle 410 is opaque or an external tool such as the tray 70 interferes with observation, visual inspection of the graft Cg may be difficult. Further, when the cell transplantation device 510 includes the plurality of needles 410, it is a burden for the user to inspect the holding state of the graft Cg for each of the needles 410. In particular, in transplantation of groups of cells that contribute to hair growth or hair restoration, a large number of grafts Cg are typically required to be transplanted. Accordingly, inspection of the holding state of the grafts Cg requires a great effort.

On the other hand, according to the cell transplantation system 500 of the present embodiment, transplantation can be performed based on the detection by the detector 520 as to whether the graft Cg is held. Accordingly, the efficiency in transplantation of the grafts Cg can be greatly improved.

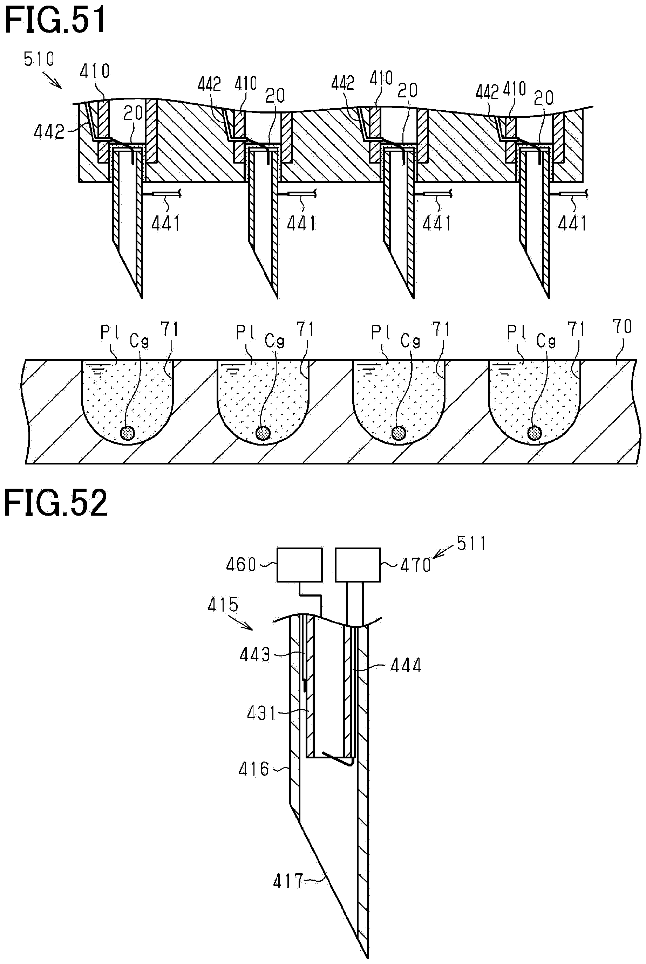

Fig. 51 illustrates a step in a transplantation method using the cell transplantation device 510 having a plurality of needles 410. The plurality of needles 410 each have the holding portion 430. The first conductor 441 and the second conductor 442 are provided for each needle 410, and the detector 520 detects when the graft Cg is held for each needle 410.

In the collection step, the cell transplantation device 510 is aligned with the tray 70 so that the respective needles 410 are directed to the corresponding grafts Cg. Accordingly, the grafts Cg can be collected into the corresponding needles 410 in a single operation. Further, in the placement step, a plurality of grafts Cg can be ejected from the respective needles 410 into the target region in a single operation. Accordingly, the efficiency of transplantation is further improved.

When the flow shown in Fig. 48 is applied to the cell transplantation system 500 in which the cell transplantation device 510 includes the plurality of needles 410, the placement step is performed, for example, after the graft Cg is detected as being held for all the needles 410. In this case, the retry step is repeated until the graft Cg is detected as being held for all the needles 410.

Alternatively, the placement step may also be performed after the graft Cg is detected as being held for at least a predetermined number of needles 410. In transplantation of group of cells that contribute to hair growth or hair restoration, a large number of grafts Cg are transplanted in a predetermined range. Therefore, even if some of the plurality of needles 410 do not hold the graft Cg and do not eject the graft Cg into the target region, the influence on the effect of the transplantation may be negligible. In this case, all the needles 410 do not necessarily hold the graft Cg, and transplantation can be performed when at least the predetermined number of needles 410 hold the graft Cg. Accordingly, the time required before the placement step can be reduced.

In the sixth embodiment, the holding portion 430 may have a configuration different from that described above as long as it is configured to retain the graft Cg in a region surrounded by the holding portion 430 from an intermediate position in the internal flow channel 14 of the needle 410 to the opening 13. Further, the above configuration may have the first conductor 441 located outside the needle 410 and in contact with the holding portion 430, and the second conductor 442 located inside the needle 410 and configured to be in contact with the holding portion 430 when the graft Cg is held in the holding portion 430.

All the configurations described in the first embodiment, the second embodiment, and the modifications thereof can be applied to, for example, the needle 410 and the filter unit 20. Further, in the needle 410 integrally formed with the support 450, the holding portion may be formed by providing the filter unit 20 in the internal flow channel. Alternatively, instead of providing the filter unit 20 made of a fibrous member 21, the holding portion that holds the graft Cg may also be formed by providing a sheet-shaped filter or changing the flow channel cross-sectional area of the internal flow channel 14.

As described above, according to the cell transplantation system of the sixth embodiment, in which the grafts Cg can be collected from the tray 70 and placed in the transplant target region continuously using a single device, smooth transplantation of cells can be performed. Furthermore, according to the cell transplantation system of the sixth embodiment, the following effects can be achieved.

(6-1) Since the detector 520 detects when the graft Cg is held in the needle 410, it is possible to improve the efficiency in collection of the graft Cg into the cell transplantation device 510, and more smoothly perform cell transplantation using the cell transplantation device 510.

(6-2) The needle 410 includes the holding portion 430 that prevents the graft Cg flowing toward the proximal end of the needle 410, and the detector 520 detects when the graft Cg is held in the holding portion 430. With this configuration, the holding portion 430 holds the graft Cg, whereby the graft Cg is prevented from flowing deeper in the needle 410. Accordingly, transplantation is smoothly performed. Since the detector 520 detects when the graft Cg is held in holding portion 430, it accurately detects when the graft Cg is held in the needle 410.

(6-3) In the needle 410, a portion surrounding a region from an intermediate position in the internal flow channel 14 to the opening 13 serves as the holding portion 430 that allows the graft Cg contained in the liquid material entering through the opening 13 to be retained in the region. Accordingly, the graft Cg can be accurately held near the tip of the needle 410.

(6-4) The detector 520 detects when the graft Cg is held in the needle 410 as an electrical change in the needle 410 due to holding the graft. Therefore, it is possible to accurately detect when the graft Cg is held in the needle 410.

Specifically, the first conductor 441 is in contact with the electrically conductive portion of the holding portion 430, and the second conductor 442 is in contact with the electrically conductive portion of the holding portion 430 when the graft Cg is held in the holding portion 430. The detector 520 detects a change in electrical conduction between the first conductor 441 and the second conductor 442 to thereby detect when the graft Cg is held. Thus, a change in electrical conduction between the first conductor 441 and the second conductor 442 can be used to accurately detect when the graft Cg is held.

(6-5) The cell transplantation device 510 includes a plurality of needles 410, and the detector 520 detects when the graft Cg is held for each needle 410. When the cell transplantation device 510 includes the plurality of needles 410, visual inspection of the holding state of the grafts Cg for each of the needles 410 requires a great effort. Therefore, the configuration of the present embodiment can be applied to the cell transplantation system 500 in which the cell transplantation device 510 includes the plurality of needles 410 to improve the efficiency of transplantation.

(6-6) The graft Cg includes an aggregation of cells having a diameter of 100 µm or more and 1000 µm or less as a group of cells. Further, the graft Cg includes a cell aggregate containing skin-derived stem cells as a group of cells. When transplantation of such a group of cells is performed for hair growth or hair restoration, a large number of groups of cells are typically required to be transplanted. Using the cell transplantation device 510 for transplantation of the group of cells greatly improves the efficiency of cell transplantation, which is a great advantage for improved collection of the grafts.

### (Seventh Embodiment)

With reference to Figs. 52 to 56, a cell transplantation device of a seventh embodiment will be described. As in the sixth embodiment, an object of the seventh embodiment is to provide a cell transplantation system that enables smooth transplantation of cells while improving the efficiency in collecting cells from the container. The following description focuses on differences between the seventh embodiment and the sixth embodiment, and configurations which are the same as that of the sixth embodiment will be referred to by the same reference numbers and the description thereof will be omitted.

In the cell transplantation system of the seventh embodiment, the configuration of the holding portion of the cell transplantation device and the arrangement of the first conductor and the second conductor differ from those of the sixth embodiment, while the structure and function of the support 450 and the suction-ejection unit 460 and the configuration of the detector 520 are the same as those of the sixth embodiment.

### [Cell Transplantation Device]

As shown in Fig. 52, a cell transplantation device 511 of the seventh embodiment includes a needle 415, which is a separate member from the support 450. The needle 415 includes an outer cylinder 416 and a tubular holding portion 431 located inside the outer cylinder 416. The outer shape of the outer cylinder 416 is not specifically limited as long as it has a tip that can puncture a transplant target site such as a skin. For example, the outer cylinder 416 may extends in a hollow cylindrical shape, and the tip of the outer cylinder 416 may be sharpened by truncating a cylindrical shape obliquely relative to the extending direction.

The holding portion 431 extends inside the outer cylinder 416 in the extending direction of the outer cylinder 416. The shape of the holding portion 431 is not specifically limited as long as it has a tubular shape. For example, when the outer cylinder 416 extends in a hollow cylindrical shape, the holding portion 431 may also extend in a hollow cylindrical shape. The end face on the tip of the holding portion 431 is preferably a flat surface perpendicular to the extending direction of the holding portion 431.

The holding portion 431 is displaceable relative to the outer cylinder 416 in the extending direction of these tubes, that is, in the extending direction of the needle 415. Specifically, the holding portion 431 is displaceable relative to the outer cylinder 416 between a state in which the tip of the holding portion 431 does not protrude from the opening 417 at the tip of the outer cylinder 416 but is located inside the outer cylinder 416 and a state in which the tip of the holding portion 431 protrudes from the opening 417 and is located outside the outer cylinder 416.

The cell transplantation device 511 includes a displacement unit 470 having a structure and a mechanism for moving the outer cylinder 416 and the holding portion 431 relative to each other. As the displacement unit 470 is actuated, the holding portion 431 is displaced relative to the outer cylinder 416.

Further, the suction-ejection unit 460 can apply suction to the inside of the holding portion 431, and apply pneumatic pressure to the inside of the holding portion 431. The suction-ejection unit 460 is an example of a suction unit.

As the suction is applied to the inside of the holding portion 431, the graft is attracted to the holding portion 431 and held at the tip of the holding portion 431. The inner diameter of the holding portion 431 has a size that does not allow the entire graft to enter the holding portion 431. On the other hand, the inner diameter of the holding portion 431 preferably has a size sufficient to exert a suction force for aspirating the graft.

The cell transplantation device 511 includes a first conductor 443 and a second conductor 444. The first conductor 443 and the second conductor 444 are each electrically connected to the detector 520. The first conductor 443 and the second conductor 444 are each embodied as, for example, a conductive wire covered with an insulating sheath, and have a conductive end portion exposed from the insulating sheath.

The end portion of the first conductor 443 is in contact with the holding portion 431 before and after the graft is held by the holding portion 431. For example, the end portion of the first conductor 443 is located inside the outer cylinder 416 and outside the holding portion 431, and is in contact with the outer peripheral surface of the holding portion 431.

The end portion of the second conductor 444 is located near the tip of the holding portion 431. The end portion of the second conductor 444 is separated from the holding portion 431 before the graft is held by the holding portion 431, and is pressed against the holding portion 431 by the graft when the graft is held by the holding portion 431. For example, the second conductor 444 is located inside the outer cylinder 416 and outside the holding portion 431, and the end portion of the second conductor 444 protrudes inside the holding portion 431 through the opening at the tip of the holding portion 431.

For example, each of the first conductor 443 and the second conductor 444 penetrates through the needle 415 or is lead out from the proximal end of the needle 415 to the outside of the needle 415, and is connected to the detector 520.

In the holding portion 431, at least a portion that is in contact with the end portion of the first conductor 443 and a portion that is in contact with the end portion of the second conductor 444 when the graft is held are made of an electrically conductive material and continuous with each other to form an electrically conductive portion. For example, the entire holding portion 431 may be made of metal so that the entire holding portion 431 forms an electrically conductive portion, or a portion near the proximal end of the holding portion 431 may be made of metal to form an electrically conductive portion while the remaining portion may be made of resin. Materials of the outer cylinder 416 are not specifically limited.

### [Cell Transplantation System]

Operation of the cell transplantation system of the seventh embodiment will be described in connection with a method of transplanting a graft using the cell transplantation device 511. In some of the drawings described below, the first conductor 443 and the second conductor 444 are omitted.

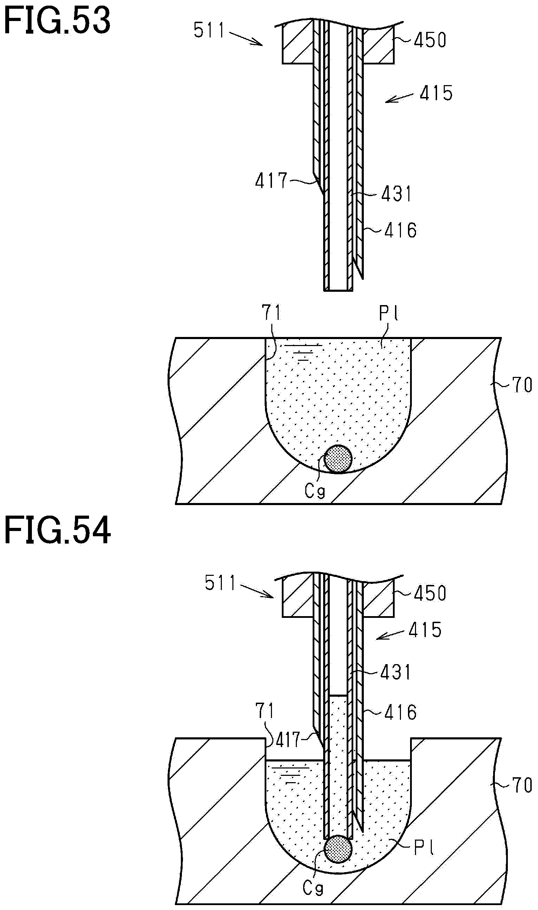

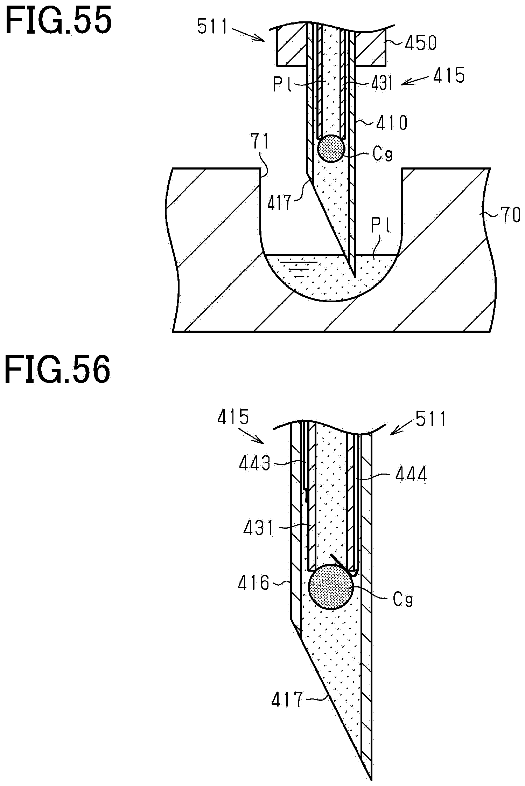

Fig. 53 shows the cell transplantation device 511 in the collection step, in which the tip of the holding portion 431 protrudes from the opening 417 at the tip of the outer cylinder 416. Then, the tip of the holding portion 431 is brought close to the graft Cg, and suction is applied to the inside of the holding portion 431. Accordingly, as shown in Fig. 54, the graft Cg is attracted to the tip of the holding portion 431 and held at the tip of the holding portion 431. Then, as shown in Fig. 55, as the holding portion 431 is displaced relative to the outer cylinder 416, the tip of the holding portion 431 is retracted in the outer cylinder 416 so that the graft Cg is collected into the needle 415. Since the graft Cg is collected together with the protective liquid PI, the graft Cg is surrounded by the protective liquid Pl in the needle 415. The graft Cg is held at the tip of the holding portion 431 and prevented from flowing toward the proximal end of the needle 415.

Fig. 56 illustrates the needle 415 in which the graft Cg is held by the holding portion 431. As shown in Fig. 56, the graft Cg is in contact with the tip of the holding portion 431 when held by the holding portion 431. When the graft Cg is held by the holding portion 431, the graft Cg abuts against the second conductor 444 and displaces the second conductor 444. As a result, the end portion of the second conductor 444 comes into contact with the holding portion 431 to cause the first conductor 443 and the second conductor 444 to be electrically connected to each other. Due to the end portion of the second conductor 444 protruding inside the holding portion 431 through the opening at the tip of the holding portion 431, the graft Cg, abutting against the tip of the holding portion 431, can be easily in contact with the tip of the holding portion 431 of the second conductor 444.

As in the sixth embodiment, the detector 520 detects an electrical change in the holding portion 431, which is a change in electrical conduction between the first conductor 443 and the second conductor 444, to thereby detect when the graft Cg is held. Further, in the seventh embodiment, the collection step and the placement step are performed in the same procedure as that of the flow shown in Fig. 48 in the sixth embodiment. In the seventh embodiment, it is possible to detect the timing when the graft Cg is held by the holding portion 431 before the graft Cg is stored in the needle 415. Therefore, the graft Cg may be stored in the needle 415 by displacing the holding portion 431 relative to the outer cylinder 416 after the graft Cg is detected as being held by the holding portion 431.

In a state in which the graft Cg is stored in the needle 415, a distance between the proximal end of the opening 417 and the tip of the holding portion 431 in the extending direction of the needle 415 may be larger than the diameter of the graft Cg.

The placement step is performed in the same manner as in the sixth embodiment. That is, while the needle 415 is inserted into the transplantation site in the living body, the suction-ejection unit 460 is actuated to eject the graft Cg together with the protective liquid Pl from the needle 415 through the opening 417. Accordingly, the graft Cg is placed at the target region. The needle 415 is then removed from the transplantation site, and transplantation of the graft Cg into the target region is completed.

Alternatively, after the needle 415 is inserted into the transplantation site, the outer cylinder 416 may be retracted relative to the holding portion 431 so that the graft Cg and the tip of the holding portion 431 protrude from the opening 417. Since the graft Cg and the tip of the holding portion 431 are positioned in a space where the tip of the needle 415 was located in the target region, the graft Cg can be smoothly ejected from the needle 415 and placed in the target region.

In the seventh embodiment, since the detector 520 detects when the graft Cg is held by the needle 415, the holding state of the graft Cg can be easily recognized compared with a case of visual inspection, and the accuracy in the inspection of the holding state can be improved. Accordingly, it is possible to improve the efficiency in collection of the graft Cg from the tray 70, and more smoothly perform transplantation of the graft Cg using the cell transplantation device 511.

Further, in the collection step, the graft Cg is held at the tip of the holding portion 431 with the tip of the holding portion 431 protruding from the opening 417 of the outer cylinder 416. Accordingly, the graft Cg can be held with a holding unit for holding the graft Cg being located closer to the graft Cg compared with the case where the graft Cg is held inside the needle 415. Therefore, it is possible to easily hold the graft Cg.

As in the sixth embodiment, the cell transplantation device 511 of the seventh embodiment may include a single needle 415 or a plurality of needles 415. When the cell transplantation device 511 includes a plurality of needles 415, the detector 520 detects when the graft Cg is held for each of the needles 415.

In the seventh embodiment, the holding portion 431 may not necessarily be displaceable relative to the outer cylinder 416, and may always be located inside the outer cylinder 416. That is, the graft Cg may be held at the tip of the holding portion 431 by suction while the tip of the holding portion 431 is located inside the outer cylinder 416. In this case, the cell transplantation device 511 does not include the displacement unit 470. Further, the outer cylinder 416 may be formed integrally with the support 450. In short, both the first conductor 443 and the second conductor 444 may be in contact with the holding portion 431 inside the needle 415 when the graft Cg is collected in needle 415.

As described above, according to the cell transplantation system of the seventh embodiment, in which the grafts Cg can be collected from the tray 70 and placed in the transplant target region continuously using a single device, smooth transplantation of cells can be performed. Further, according to the cell transplantation system of the seventh embodiment, the following effects can be achieved in addition to the effects of (6-1), (6-2), (6-4), (6-5), and (6-6) in the sixth embodiment.

(6-7) The needle 415 includes the outer cylinder 416 and the tubular holding portion 431 located inside the outer cylinder 416, and the graft Cg is held at the tip of the holding portion 431 when suction is applied to the inside of the holding portion 431. Accordingly, the graft Cg can be accurately held near the tip of the needle 415.

### (Eighth Embodiment)

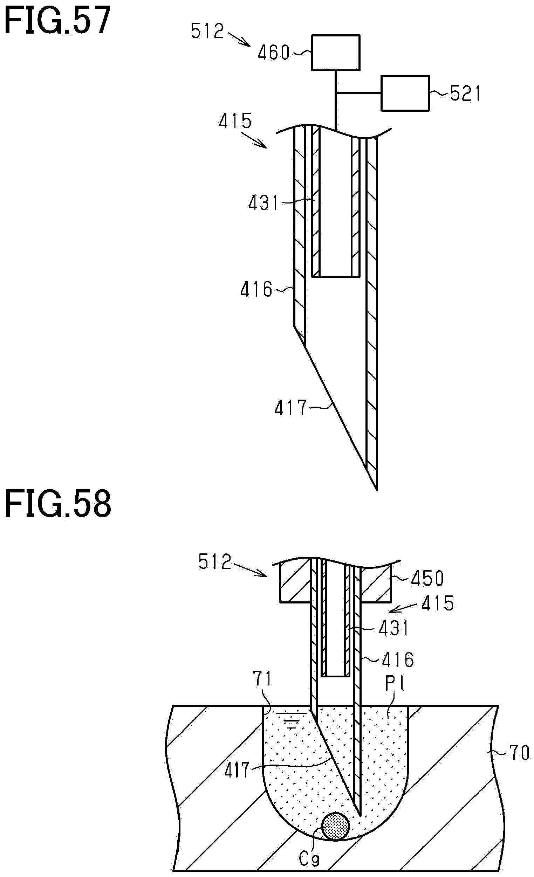

With reference to Figs. 57 to 59, a cell transplantation device of an eighth embodiment will be described. As in the sixth embodiment, an object of the eighth embodiment is to provide a cell transplantation system that enables smooth transplantation of cells while improving the efficiency in collecting cells from the container. The following description focuses on differences between the eighth embodiment and the sixth and seventh embodiments, and configurations which are the same as those of the sixth and seventh embodiments will be referred to by the same reference numbers and the description thereof will be omitted.

In the cell transplantation system of the eighth embodiment, a method by which the detector detects when the graft is held differs from that of the sixth and seventh embodiments, while the structure and function of the support 450 and the suction-ejection unit 460 are the same as those of the sixth embodiment.

### [Cell Transplantation Device]

As shown in Fig. 57, the needle 415 included in the cell transplantation device 512 of the eighth embodiment has the same configuration as that in the seventh embodiment. That is, the needle 415 includes the outer cylinder 416 and the holding portion 431. As in the seventh embodiment, the holding portion 431 may be displaceable relative to the outer cylinder 416 in the extending direction of the needle 415, or the holding portion 431 may always be located inside the outer cylinder 416 at a fixed position relative to the outer cylinder 416.

In the eighth embodiment, the cell transplantation device 512 does not include a first conductor and a second conductor. A detector 521 detects a change in pressure in the holding portion 431 to detect when the graft is held.

Specifically, the detector 521 is connected to a suction path from the suction-ejection unit 460 to the holding portion 431, and includes a sensor that detects a pressure corresponding to the pressure in the holding portion 431. The detector 521 detects when the holding portion 431 holds a graft according to the detection result from the sensor.

In the eighth embodiment, materials of the outer cylinder 416 and the holding portion 431 are not specifically limited, and the holding portion 431 may not necessarily include an electrically conductive portion.

### [Cell Transplantation System]

Operation of the cell transplantation system of the eighth embodiment will be described in connection with a method of transplanting a graft using the cell transplantation device 512. Further, as in the seventh embodiment, in the collection step of the eighth embodiment, the graft may be collected into the outer cylinder 416 by displacing the holding portion 431 after the graft is held by the holding portion 431 with the tip of the holding portion 431 protruding from the opening 417 of the outer cylinder 416, or the graft may be held and collected with the tip of the holding portion 431 being located inside the outer cylinder 416. The following description illustrates the case where the graft is held and collected with the tip of the holding portion 431 being located inside the outer cylinder 416.

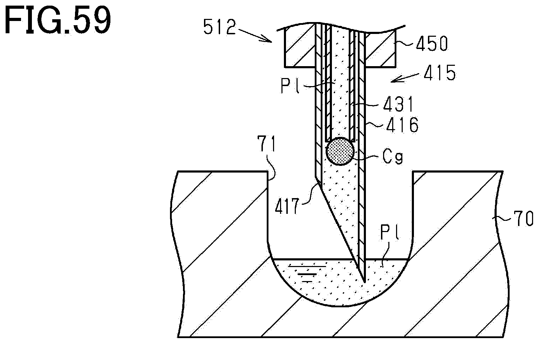

As shown in Fig. 58, in the collection step, the tip of the holding portion 431 is located inside the outer cylinder 416. When suction is applied to the inside of the holding portion 431 with the tip of the needle 415 being brought close to the graft Cg, the graft Cg enters the outer cylinder 416, and is attracted to the tip of the holding portion 431 and held at the tip of the holding portion 431 as shown in Fig. 59. Thus, the graft Cg is collected into the needle 415. Since the graft Cg is collected together with the protective liquid PI, the graft Cg is surrounded by the protective liquid Pl in the needle 415.

As shown in Fig. 59, the graft Cg is in contact with the tip of the holding portion 431 when held by the holding portion 431. Accordingly, when the graft Cg is held by the holding portion 431 while suction is applied to the inside of the holding portion 431, the opening at the tip of the holding portion 431 is partially or entirely closed, which increases the pressure in the holding portion 431.

The detector 521 detects a pressure change in the holding portion 431 to detect when the graft Cg is held. Detecting an increase in pressure in the holding portion 431 indicates that the graft Cg is held.

In the eighth embodiment, the collection step and the placement step are performed in the same procedure as that of the flow shown in Fig. 48 in the sixth embodiment. When the graft Cg is held with the tip of the holding portion 431 protruding from the opening 417 of the outer cylinder 416, it is possible to detect the timing when the graft Cg is held by the holding portion 431 before the graft Cg is stored in the needle 415. Therefore, the graft Cg may be stored in the needle 415 by displacing the holding portion 431 relative to the outer cylinder 416 after the graft Cg is detected as being held by the holding portion 431. The placement step is performed in the same manner as in the seventh embodiment.

In the eighth embodiment, since the detector 521 detects when the graft Cg is held by the needle 415, the holding state of the graft Cg can be easily recognized compared with a case of visual inspection, and the accuracy in the inspection of the holding state can be improved. Accordingly, it is possible to improve the efficiency in collection of the graft Cg from the tray 70, and more smoothly perform cell transplantation using the cell transplantation device 512.

As in the sixth embodiment, the cell transplantation device 512 of the eighth embodiment may include a single needle 415 or a plurality of needles 415. When the cell transplantation device 512 includes a plurality of needles 415, the detector 521 detects when the graft Cg is held for each of the needles 415.

As described above, according to the cell transplantation system of the eighth embodiment, in which the grafts Cg can be collected from the tray 70 and placed in the transplant target region continuously using a single device, smooth transplantation of cells can be performed. Further, according to the cell transplantation system of the eighth embodiment, the following effects can be achieved in addition to the effects of (6-1), (6-2), (6-5), and (6-6) in the sixth embodiment and the effect of (6-7) in the seventh embodiment.

(6-8) The detector 521 detects when the graft Cg is held in the needle 410 as a pressure change in the needle 410 due to the hold. Therefore, it is possible to accurately detect when the graft Cg is held in the needle 410.

Specifically, the detector 521 detects a change in pressure in the holding portion 431 due to the graft Cg being held by the holding portion 431 to thereby detect when the graft Cg is held. Thus, a change in pressure in the holding portion 431 can be used to accurately detect when the graft Cg is held.

### [Modifications]

The sixth, seventh, and eighth embodiments can be modified and implemented as described below.
- The holding portion may have a configuration different from that described in the above embodiments as long as it is configured to prevent the graft contained in the liquid material from flowing toward the proximal end of the needle.
- In the above embodiments, the detector detects when the graft is held by detecting an electrical change or a pressure change in the holding portion. The detection method is not limited to that described in the above embodiments as long as the detector can detect when the graft is held in the needle. For example, the detector may also detect when the graft is held in the needle by using a change in resistance in the circuit including the holding portion, a change in capacitance or an electromagnetic field in the needle, or an optical change such as a change in reflectance or transmittance of light in the needle.
- A tube or tubular member constituting the needle, that is, a member such as the first tube 11, the second tube 12, the outer cylinder 416, or the holding portion 431 is not limited to a structure having a hollow cylindrical shape as long as it has openings at the tip and the proximal end, which communicate with each other via a space inside the member. The outer shape of the above members may be, but is not limited to, a cylindrical, conical, or prismatic shape. Further, a shape of the space defined in the above members may be, but is not limited to, a cylindrical, conical, or prismatic shape.
- The needle may also have a tubular shape without having a holding portion. In this case, the graft is held by the needle when it enters the needle, and the detector can detect when the graft enters the needle as the graft being held.
- The detector may detect when the graft is held by the needle by observing the tray 70. For example, the cell transplantation system may include a camera that captures an image of the inside of the recess 71 of the tray 70, and determine whether the graft is present in the recess 71 by analyzing the image captured by the camera in the collection step. When it is determined that the graft is not present in the recess 71, the detector determines that the graft has been collected in the needle, that is, the graft is held in the needle. Thus, the graft is detected as being held.
- In addition to detecting when the graft is held in the collection step, the detector may also detect when the graft is released from the needle after the needle is inserted into the transplantation site in the placement step. Detecting when the graft is released can prevent failure of appropriate transplantation due to the graft being left in the needle.

### [Supplementary Notes]

The measure for solving the foregoing problems embraces the following items as technical ideas derived from the sixth, seventh, and eighth embodiments and the modifications thereof.

### (Item 31)

A cell transplantation system including: a cell transplantation device configured to place a graft containing cells into a target region in a living body,
the cell transplantation device including a needle extending in one direction, the needle being configured to collect the graft through an opening formed at a tip of the needle and store the graft in the needle; and
a detector that detects when the graft is held by the needle.

### (Item 32)

The cell transplantation system according to the item 31, wherein
the needle includes a holding portion configured to hold the graft, the holding portion being configured to prevent the graft contained in a liquid material from flowing toward the proximal end of the needle, and
the detector detects when the graft is held by the holding portion.

### (Item 33)

The cell transplantation system according to the item 32, wherein a portion surrounding a region from an intermediate position in a flow channel defined in the needle to the opening serves as the holding portion that allows the graft contained in the liquid material entering through the opening to be retained in the region.

### (Item 34)

The cell transplantation system according to the item 32, wherein
the needle includes an outer cylinder having the opening,
the holding portion has a tubular shape located inside the outer cylinder,
the cell transplantation device includes a suction unit that applies suction to the inside of the holding portion, and
the holding portion is configured to hold the graft at a tip of the holding portion by the suction.

### (Item 35)

The cell transplantation system according to the item 34, wherein the detector detects a change in pressure in the holding portion due to the graft being held by the holding portion to thereby detect when the graft is held.

### (Item 36)

The cell transplantation system according to any one of the items 31 to 35, wherein
the cell transplantation device includes a plurality of needles, and
the detector detects when the graft is held for each of the needles.

### (Item 37)

The cell transplantation system according to any one of the items 31 to 36, wherein the cell transplantation device is used to place the graft containing a cell aggregate having a diameter of 100 µm or more and 1,000 µm or less into the target region.

### (Item 38)

The cell transplantation system according to any one of the items 31 to 37, wherein the cell transplantation device is used to place the graft, which is a cell aggregate containing skin-derived stem cells, into the target region.

### (Ninth Embodiment)

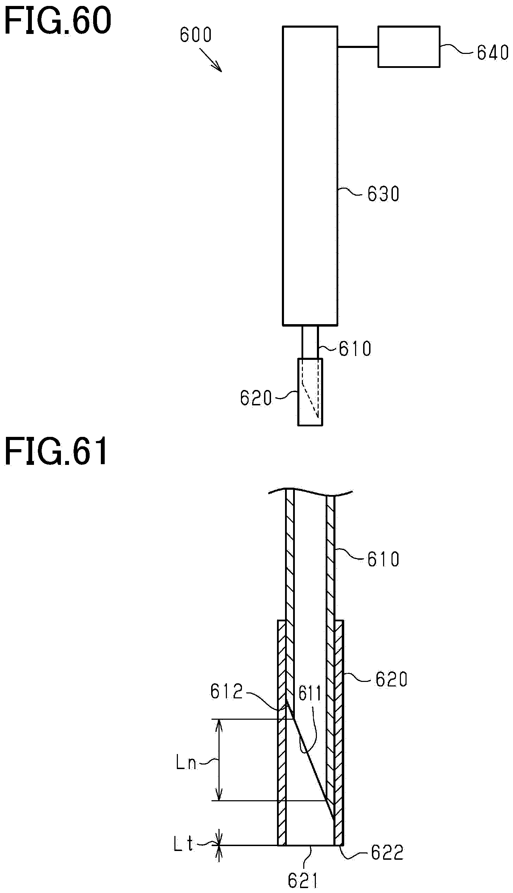

With reference to Figs. 60 to 73, a cell transplantation device of a ninth embodiment will be described. An object of the ninth embodiment is to provide a cell transplantation device that enables smooth transplantation of cells.

### [Cell Transplantation Device]

As shown in Fig. 60, a cell transplantation device 600 includes a needle 610 extending in one direction, an outer cylinder 620 located outside the needle 610, and a support 630 that supports the needle 610. The outer cylinder 620 is an example of a tubular member. Further, the cell transplantation device 600 includes a suction unit 640 that applies suction to the inside of the needle 610. The support 630 may also serve as the suction unit 640.

First, a detailed configuration of the needle 610 and the outer cylinder 620 will be described. As shown in Fig. 61, the needle 610 has a tubular shape, and an opening 611 at the tip. The tip of the needle 610 may be sharpened by truncating a tubular shape obliquely relative to the extending direction. The outer shape of the needle 610 is not specifically limited. For example, the needle 610 may extend in a hollow cylindrical shape, and the tip of the needle 610 may have a shape formed by truncating a cylindrical shape obliquely relative to the extending direction. An end face 612 on the tip of the needle 610, that is, the end face 612 that defines the opening 611, is beveled relative to an extending direction, which is a direction in which the needle 610 extends.

The outer cylinder 620 has a tubular shape fitted outside the needle 610. The outer cylinder 620 extends in the same direction as the needle 610, that is, in the extending direction. The tip of the needle 610 extends inside the outer cylinder 620 such that the outer peripheral surface of the needle 610 is in contact with the inner peripheral surface of the outer cylinder 620. The outer shape of the needle 610 is not specifically limited as long as the inner peripheral surface of the outer cylinder 620 has a shape extending along the outer peripheral surface of the needle 610. For example, when the needle 610 has a hollow cylindrical shape, the outer peripheral surface of the outer cylinder 620 may be circular or polygonal as long as the inner peripheral surface of the outer cylinder 620 is circular cylindrical.

An end face 622 on the tip of the outer cylinder 620, that is, the end face 622 that defines an opening 621 at the tip of the outer cylinder 620 is a flat surface perpendicular to the extending direction. With this configuration, a length Lt of the opening 621 of the outer cylinder 620 in the extending direction is 0, and the length Lt is smaller than a length Ln of the opening 611 of the needle 610 in the extending direction.

The outer cylinder 620 is configured to be slidable along the needle 610. Specifically, the outer cylinder 620 can be slid along the needle 610 to switch the state in which the opening 611 of the needle 610 is located inside the outer cylinder 620 to the state in which the opening 611 of the needle 610 is exposed from the outer cylinder 620. In other words, the outer cylinder 620 is configured to be displaced along the needle 610 from the state in which the tip of the outer cylinder 620 protrudes from the tip of the needle 610.

Further, the length of the outer cylinder 620 in the extending direction may be a length that allows the opening 611 of the needle 610 to be housed in the outer cylinder 620.

Materials of the needle 610 and the outer cylinder 620 are not specifically limited. The needle 610 and the outer cylinder 620 may be made of, for example, a metal or a resin. Examples of the resin material forming the outer cylinder 620 may include fluororesin and silicone. When the outer cylinder 620 is transparent, the graft can be easily observed when collected into the outer cylinder 620.

The outer cylinder 620 may be elastically deformable, and may be attached to the needle 610 using the elasticity. Alternatively, the outer cylinder 620 may be attached to the needle 610 by using a member for fixing the outer cylinder 620 to the needle 610. Further, the needle 610 or the outer cylinder 620 may have a structure such as a groove or a projection for engaging the outer cylinder 620 at a predetermined position to the needle 610.

At least one of the inner peripheral surface of the needle 610 and the inner peripheral surface of the outer cylinder 620 is preferably made of a material that reduces adhesion of the graft. For example, a material of the tubular structure of the needle 610 and the outer cylinder 620 may have properties that reduce adhesion of the graft, or an inner peripheral surface of the tubular structure may be subjected to coating to form a surface layer having properties that reduce adhesion of the graft. Examples of the material forming the surface layer include silicone and polyethylene glycol.

A space inside the needle 610 may have a size suitable for storing the graft. For example, when the graft is a group of cells that contributes to hair growth or hair restoration, which is a cell aggregate such as spheroids and germs, the space inside the needle 610 preferably has a size suitable to store one graft. Specifically, an inner diameter of the needle 610 is preferably larger than the assumed diameter of a graft. For example, a diameter of the sphere that approximates the group of cells, which is the cell aggregate, that is, a diameter of a minimum circumscribed sphere of the group of cells is approximately from 0.1 mm to 1.0 mm. The needle 610 may be, for example, a 25G injection needle. In this case, the inner diameter of the needle 610 is 0.4 mm, the outer diameter of the needle 610 is 0.5 mm, and a length of the end face 612 of the needle 610 from the distal end to the proximal end in the extending direction is 2.8 mm.

A smaller outer diameter of the needle 610 is preferred as long as the strength of the needle 610 is guaranteed. Since the side wall thickness of the needle 610 decreases as the outer diameter of the needle 610 decreases, the graft is not likely to be caught by the edge of the side wall when the graft is collected into the needle 610. Specifically, the thickness of the side wall of the needle 610 is preferably 50 µm or less.

The support 630 may be formed as a separate member from the needle 610 or may be formed integrally with the needle 610. When the support 630 is a member formed separately from the needle 610, the support 630 supports the needle 610, surrounding the outer periphery of a portion of the needle 610 which is continuous from the tip of the needle 610. For example, the needle 610 extends through a hole formed in the support 630 whereby it is assembled to the support 630. When the support 630 is formed integrally with the needle 610, the needle 610 protrudes from a surface of the support 630. The outer shape of the support 630 is not specifically limited.

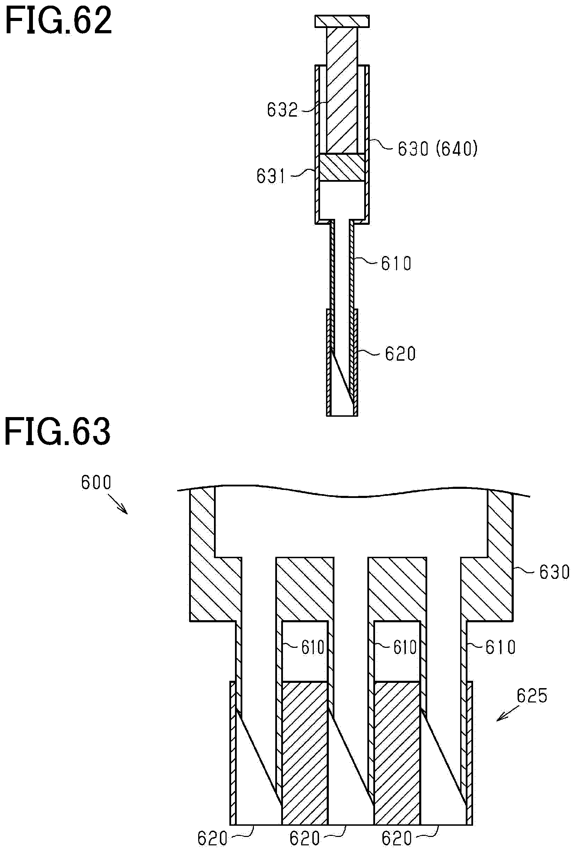

The suction unit 640 includes a mechanism that applies suction to the inside of the needle 610. The suction unit 640 may further have a function of applying pneumatic pressure to the inside of the needle 610. Fig. 62 illustrates an example of the cell transplantation device 600 which includes the support 630 that also serves as the suction unit 640 having a function of applying suction and pressure to the inside of the needle 610.

The support 630 shown in Fig. 62 has a syringe-like configuration, and includes a barrel 631 connected to the proximal end of the needle 610 and a plunger 632 located inside the barrel 631. The barrel 631 has a tubular shape having an inner diameter larger than that of the needle 610. The plunger 632 applies suction to the inside of the needle 610 when it is retracted in the barrel 631, and applies pressure to the inside of the needle 610 when it is pushed in the barrel 631.

The suction unit 640 is not limited to the above configuration, and, for example, may include a pump mechanism or the like connected to the needle 610, and apply suction and pressure by driving the mechanism.

The number of needles 610 of the cell transplantation device 600 is not specifically limited. The cell transplantation device 600 may include a single needle 610 as shown in Figs. 60 and 62 or a plurality of needles 610 as shown in Fig. 63. When the cell transplantation device 600 includes a plurality of needles 610, the arrangement of the plurality of needles 610 is not specifically limited. The plurality of needles 610 may be regularly arranged or irregularly arranged. Further, when the cell transplantation device 600 includes a plurality of needles 610, it is preferred that all the needles 610 are collectively supported by a single support 630. That is, the plurality of needles 610 are preferably supported by a single support 630 to provide the cell transplantation device 600 having the integrally formed plurality of needles 610. Further, suction may be individually performed on each of the needles 610, or collectively performed to the plurality of needles 610.

When the cell transplantation device 600 includes a plurality of needles 610, an outer cylinder 620 is provided for each of the plurality of needles 610. The outer cylinder 620 may be an independent tube for each needle 610, or the side walls of the adjacent outer cylinders 620 may be connected to each other to form a single member. When the side walls of the adjacent outer cylinders 620 are connected to each other, the side walls may be connected to each other at least in part in the extending direction. Fig. 63 illustrates an example in which a plurality of outer cylinders 620 are connected to each other to form a single member. In Fig. 63, the entire length in the extending direction of the side walls of the adjacent outer cylinders 620 are connected to each other.

In the above configuration, the cell transplantation device 600 is an example of a biological transfer device. When the cell transplantation device 600 includes a single needle 610, one outer cylinder 620 is a member for a biological transfer device, and when the cell transplantation device 600 includes a plurality of needles 610, a group of the outer cylinders 620 mounted on the respective needles 610 is a member for a biological transfer device. For example, as shown in Fig. 63, when the plurality of outer cylinders 620 are connected to each other to form a single member 625, which is a member for a biological transfer device.

### [Transplantation Method]

A method of transplanting a graft using the cell transplantation device 600 will be described below.

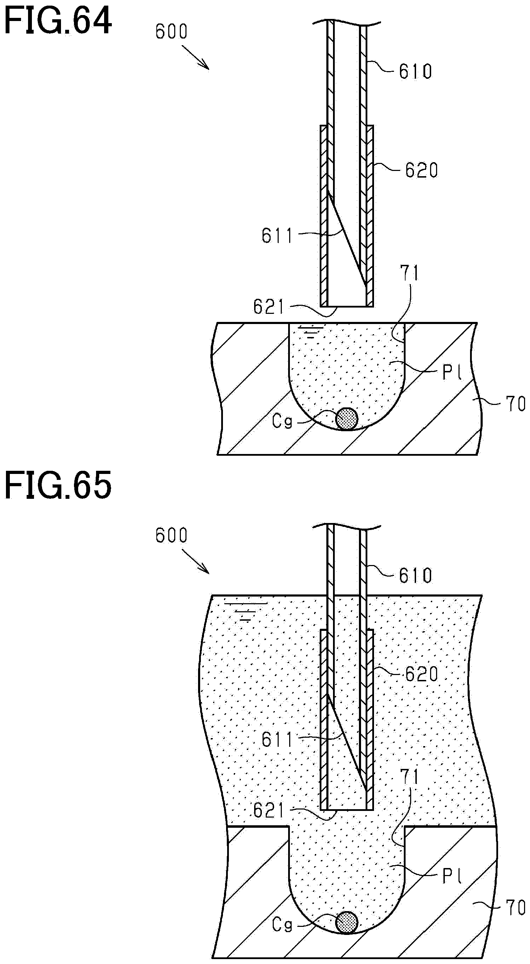

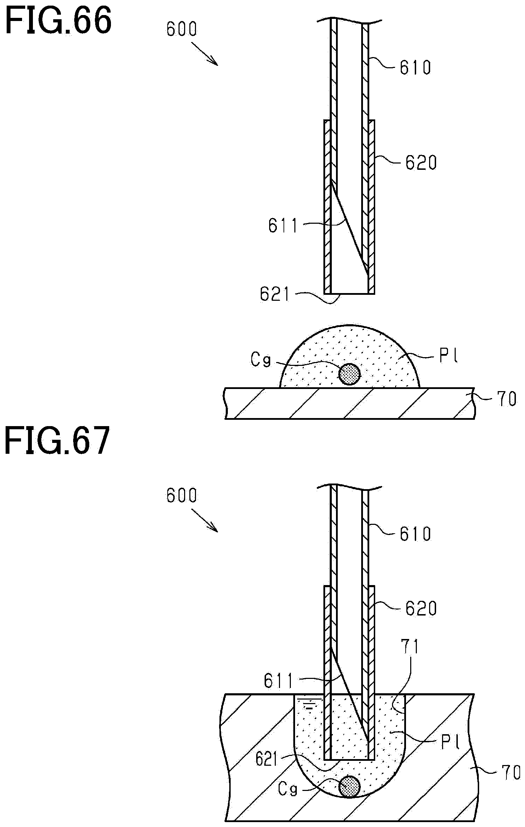

As shown in Fig. 64, a graft Cg together with a protective liquid Pl is held in a tray 70 such as a culture vessel before transplantation. The manner that the graft Cg is held in the tray 70 and the properties of the protective liquid Pl are the same as those in the first embodiment. For example, as shown in Fig. 64, the graft Cg and the protective liquid Pl are stored in a recess 71 of the tray 70, and the graft Cg is located near the bottom of the recess 71. Alternatively, as shown in Fig. 65, while the graft Cg is located near the bottom of the recess 71, the entire region of the tray 70 including the inside and the top of the recess 71 may be filled with the protective liquid Pl. Alternatively, as shown in Fig. 66, the graft Cg and the protective liquid Pl may be placed on a flat surface of the tray 70. When the protective liquid Pl has low fluidity, the grafts Cg separated from each other can be placed on the flat surface together with the protective liquid Pl.

In short, the tray 70 has a placement region, where the graft Cg is to be placed, and may hold the graft Cg in the placement region. The placement region is, for example, the recess 71 or a predetermined region on the flat surface. When the tray 70 is a culture vessel, and cells are cultured in the tray 70 to obtain the graft Cg, the tray 70 having the recess 71 is more suitable to culture the cells. Preferably, the tray 70 is made of a material having low adhesion to the cells.

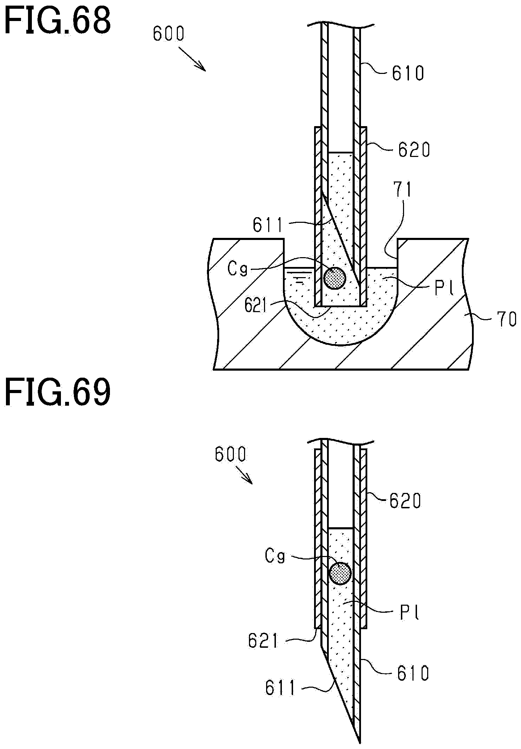

In collection of the graft Cg into the needle 610, in the cell transplantation device 600, the opening 611 at the tip of the needle 610 is located inside the outer cylinder 620. That is, the tip of the outer cylinder 620 is located at the tip of the cell transplantation device 600. Then, as shown in Fig. 67, the tip of the outer cylinder 620 is brought close to the graft Cg in the protective liquid PI, and suction is applied to the inside of the needle 610 by actuating the suction unit 640. Accordingly, as shown in Fig. 68, suction is also applied to the inside of the outer cylinder 620 to thereby collect the graft Cg together with the protective liquid Pl into the outer cylinder 620 through the opening 621 at the tip of the outer cylinder 620. Further, the graft Cg enters the needle 610 through the opening 611 of the needle 610 along with a flow of the protective liquid Pl due to the suction.

In order to collect the graft Cg into the needle 610, the graft Cg needs to be drawn to a tubular portion defined by the side wall of the needle 610, that is, a position over the proximal end of the opening 611.

In the tray 70, the graft Cg is settled in the protective liquid Pl. Accordingly, when the tip of the needle 610 having no outer cylinder 620 is brought close to the graft Cg, the tubular side wall portion is located at a position separated from the graft Cg by a distance corresponding to the length Ln of the opening 611 in the extending direction. As a consequence, even when suction is applied, the graft Cg is more likely to fail to enter the needle 610. This problem is more obvious as the length Ln of the opening 611 increases. For example, the needle 610 having a more pointed tip due to the end face 612 being more slanted has more difficulty in collecting the graft Cg into the needle 610.

In order to facilitate collection of the graft Cg, the graft Cg can be floated by vibrating the tray 70. However, this requires a step of applying vibration, which leads to a decrease in the efficiency of transplantation. Further, a large amount of protective liquid Pl may be collected into the needle 610 together with the graft Cg, which also leads to a decrease in the efficiency of transplantation.

On the other hand, according to the cell transplantation device 600 of the present embodiment, the tip of the outer cylinder 620 protrudes from the tip of the needle 610 in collection of the graft Cg into the needle 610. Since the outer cylinder 620 has a tubular side wall portion extending to the end face 622, the graft Cg can be collected by drawing the graft Cg into the tubular portion. Therefore, compared with the case where no outer cylinder 620 is provided, the graft Cg can be easily collected since a position into which the graft Cg needs to be drawn can be brought closer to the graft Cg.

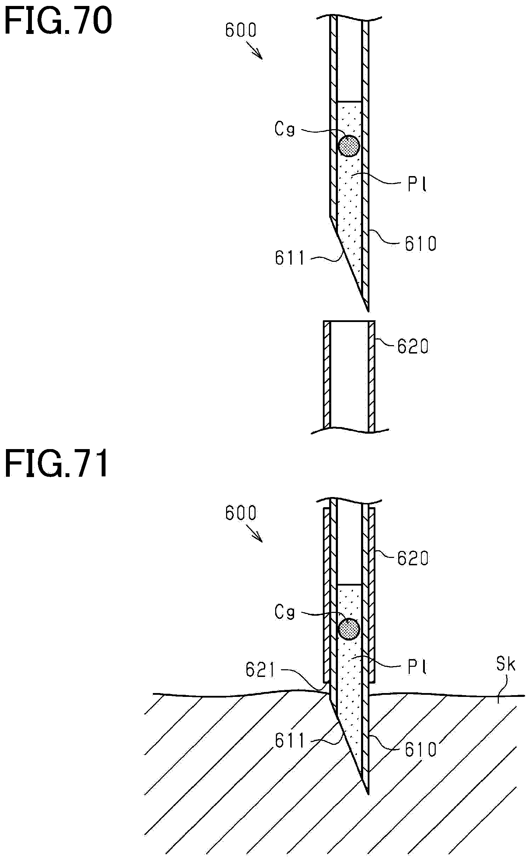

Once the graft Cg is collected into the needle 610, the outer cylinder 620 is displaced so that the opening 611 of the needle 610 is exposed from the outer cylinder 620. As shown in Fig. 69, the opening 611 of the needle 610 may be exposed by moving the outer cylinder 620 toward the proximal end of the needle 610, or, as shown in Fig. 70, the opening 611 of the needle 610 may be exposed by moving the outer cylinder 620 to a direction away from the proximal end of the needle 610 to remove it from the needle 610. A portion of the needle 610 exposed from the outer cylinder 620 includes at least a portion that penetrates the living body to place the graft Cg into a transplant target region. The outer cylinder 620 may be moved manually or may be moved by electric power by using a motor or the like.

In the present embodiment, since the tip of the needle 610 is exposed by sliding the outer cylinder 620 relative to the needle 610, a step of exposing the tip of the needle 610 can be easily performed. Accordingly, after the graft Cg is collected into the needle 610, the graft Cg can be quickly placed into the transplant target region, suppressing the deterioration of the graft Cg.

After the tip of the needle 610 is exposed, the cell transplantation device 600 is transferred to a transplantation site in the living body, for example, the skin Sk. Then, as shown in Fig. 71, the needle 610 is inserted into the transplantation site. Since the tip of the needle 610 is exposed and has a shape that can easily puncture the transplantation site, the cell transplantation device 600 can be smoothly advanced into the transplantation site. Accordingly, the tip of the needle 610 is advanced into the transplantation site so that the opening 611 is positioned at the transplant target region. When an object to be transplanted is a group of cells that contributes to hair growth or hair restoration, the target region is at least one of an intradermal site and subcutaneous site.

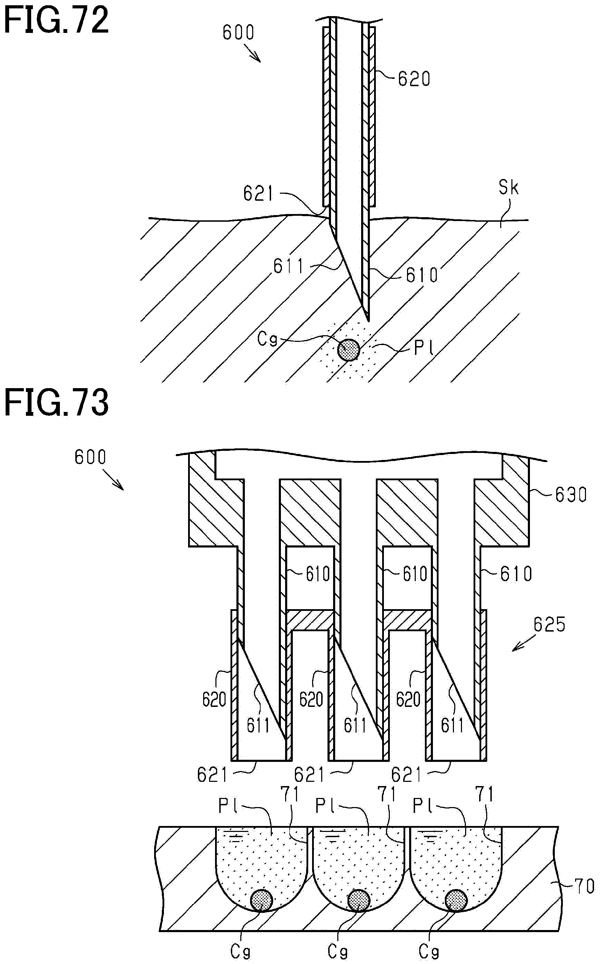

As shown in Fig. 72, as pressure is applied to, for example, the inside of the needle 610, the graft Cg that has been collected into the needle 610 is ejected together with the protective liquid Pl through the opening 611. Accordingly, the graft Cg is placed at the target region. Then, the needle 610 is removed from the transplantation site.

Thus, transplantation of the graft Cg to the target region is completed. According to the above transplantation method using the cell transplantation device 600, in which the grafts Cg can be collected from the tray 70 and placed in the transplant target region continuously using a single device, smooth transplantation of cells can be performed.

Fig. 73 illustrates a step in a transplantation method using the cell transplantation device 600 having a plurality of needles 610. In Fig. 73, the proximal ends of a plurality of outer cylinders 620 in the extending direction are connected to each other to form a single member 625, which is a member for a biological transfer device.

In collection of the grafts Cg using the cell transplantation device 600 having a plurality of needles 610, the cell transplantation device 600 is aligned with the tray 70 so that the respective needles 610 are directed to the corresponding grafts Cg. Accordingly, the grafts Cg can be collected into the corresponding needles 610 in a single operation. Then, the plurality of grafts Cg can be ejected from the respective needles 610 into the target region in a single operation. Accordingly, the efficiency of transplantation is further improved.

Specifically, the amount and arrangement of the placement regions in the tray 70 and the amount and arrangement of the needles 610 in the cell transplantation device 600, and the amount and arrangement of the outer cylinders 620 are set such that each needle 610 and each outer cylinder 620 correspond to a respective placement region. Accordingly, when the tray 70 and the cell transplantation device 600 face each other in the extending direction, each needle 610 and each outer cylinder 620 face a respective placement region. In other words, each needle 610 and each outer cylinder 620 are assigned to a respective placement region such that the placement region and the needle 610 correspond to each other one to one, and the placement region and the outer cylinder 620 correspond to each other one to one.

Since each needle 610 is assigned to a respective placement region, the amount of grafts Cg collected into each needle 610 can be adjusted by adjusting the amount of grafts Cg to be placed in each placement region. Accordingly, it is easy to collect a predetermined amount of grafts Cg into a plurality of needles 610. For example, each graft Cg is preferably collected into a respective needle 610.

Further, even when the tray 70 includes one placement region and the cell transplantation device 600 includes one needle 610 and one outer cylinder 620, it can also be regarded that each needle 610 and each outer cylinder 620 are assigned to a respective placement region when the placement region and the needle 610 are disposed to face each other.

In addition, when the placement regions of the amount and arrangement corresponding to the amount and arrangement of the needles 610 in the cell transplantation device 600 are regarded as one group, the tray 70 may include a plurality of groups of placement regions. In such a configuration, collection of the grafts Cg into the cell transplantation device 600 and placement of the grafts Cg into the target region are repeated, and collection of the grafts Cg is performed for each of the groups of placement regions. As described above, even when the tray 70 includes the plurality of groups of placement regions, it can also be regarded that each needle 610 and each outer cylinder 620 are assigned to a respective placement region.

In the above configuration, the cell transplantation device 600 and the tray 70 constitute a biological transfer unit, and the member for a biological transfer device and the tray 70 constitute a biological transfer kit.

### [Modifications]

Modifications of the cell transplantation device 600 of the ninth embodiment will be described below one by one.

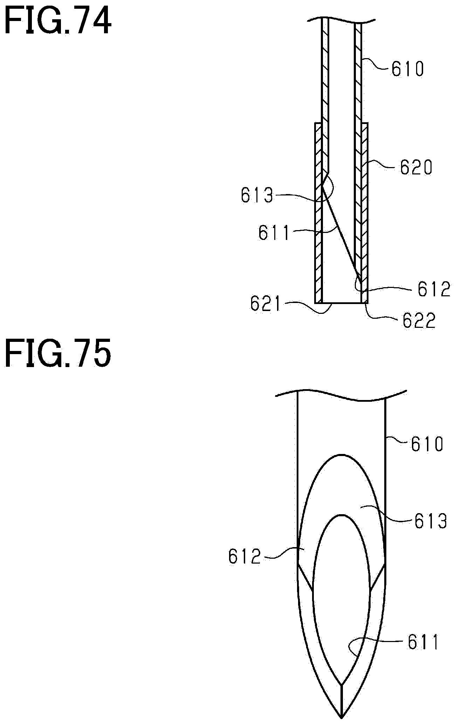

As shown in Fig. 74, the end face 612 on the tip of the needle 610 may have a slant portion 613 inclined radially inward toward the proximal end of the needle 610, in other words, inclined toward the space inside the needle 610. The slant portion 613 includes the proximal end of the end face 612. The proximal end of the end face 612 is a portion of the end face 612 including the proximal edge of the end face 612, which is a point closest to the proximal end of the needle 610 in the extending direction. The slant portion 613 is inclined from a plane parallel to the opening 611. Specifically, when a direction connecting the proximal end and the distal end of the end face 612 is defined as a gradient direction of the opening 611, and an inclination of the gradient direction from the extending direction is defined as positive, an inclination of the slant portion 613 from the extending direction is negative.

For example, as shown in Fig. 75, in the end face 612 viewed in a direction perpendicular to the extending direction, the slant portion 613 is a region extending from the proximal end of the end face 612 to the center between the distal end and the proximal end of the end face 612. Fig. 75 illustrates an example in which the end face 612 has an elliptical annular shape, and is composed of three faces. The range of the slant portion 613 is not specifically limited, and the slant portion 613 may include at least the proximal end of the end face 612.

If the proximal end of the end face 612 is parallel to the gradient direction, the proximal end of the end face 612 protrudes toward the opening 611. This may cause the graft entering the needle 610 to be caught by the edge of the proximal end of the end face 612. Due to the slant portion 613, provided at the proximal end of the end face 612, being inclined toward the space inside the needle 610, the graft can be prevented from being caught by the edge of the proximal end of the end face 612. Therefore, collection of the graft into the needle 610 can be smoothly performed.

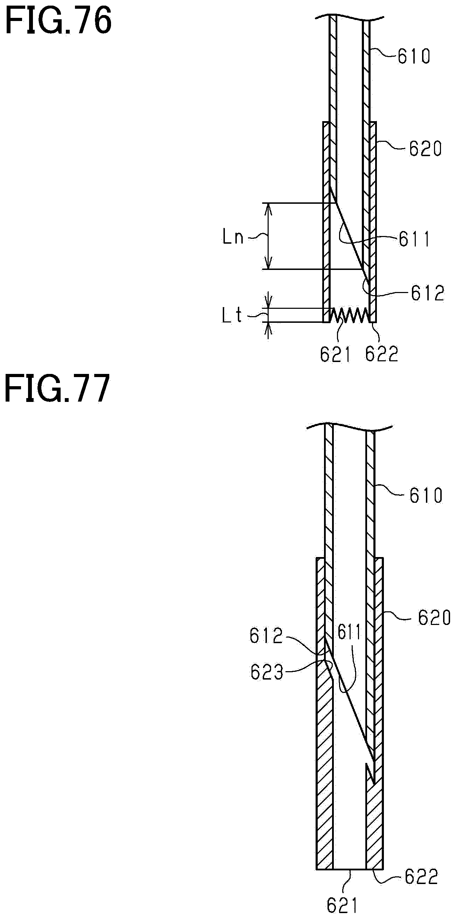

As shown in Fig. 76, the end face 622 on the tip of the outer cylinder 620 may not necessarily be flat, and may have irregularities on a surface perpendicular to the extending direction. The irregularities are formed in a circumferential direction of the outer cylinder 620. Due to the irregularities formed on the end face 622, when the end face 622 is pressed against a portion of the tray 70 where the graft is held in collection of the graft, gaps are formed between the surface of the tray 70 and recesses on the end face 622. Since the protective liquid can flow into the outer cylinder 620 through the gaps, suction can be continued. Therefore, collection of the graft can be smoothly performed.

The length Lt of the opening 621 of the outer cylinder 620 is a length from the distal end to the proximal end of the opening 621 in the extending direction, and even when the end face 622 has irregularities, the length Lt of the opening 621 is smaller than the length Ln of the opening 611 of the needle 610.

As described above, according to the cell transplantation device of the ninth embodiment, the following effects can be achieved.

(9-1) According to the cell transplantation device 600, since the grafts can be collected from the tray 70 and placed in the living body continuously using a single device, smooth transplantation of grafts can be performed.

(9-2) When the graft is collected with the tip of the outer cylinder 620 protruding from the tip of the needle 610, the tubular portion into which the graft is drawn in collection of the graft can be brought close to the graft. Accordingly, the graft can easily enter the tubular portion, so the graft can be smoothly collected into the needle 610. Therefore, transplantation can be more smoothly performed.

(9-3) In a configuration in which the end face 622 on the tip of the outer cylinder 620 is a flat surface perpendicular to the extending direction of the needle 610, the tubular portion into which the graft is drawn in collection of the graft can be brought closer to the graft. Therefore, the graft can be more smoothly collected.

(9-4) In a configuration in which the end face 622 on the tip of the outer cylinder 620 has irregularities on a surface perpendicular to the extending direction of the needle 610, suction can be continued even when the tip of the outer cylinder 620 is pressed against the tray 70 in collection of the graft. Therefore, collection of the graft can be smoothly performed.

(9-5) In a configuration in which the end face 612 on the tip of the needle 610 includes the slant portion 613, and the slant portion 613 includes the proximal end of the end face 612, the graft is prevented from being caught by the edge of the end face 612 in collection of the graft.

(9-6) In a configuration in which the inner peripheral surface of the needle 610 and the inner peripheral surface of the outer cylinder 620 are made of a material that reduces adhesion of the graft, the graft entering the outer cylinder 620 easily flows into the needle 610, and the graft is easily ejected from the needle 610 during placement of the graft into the target region. Therefore, transplantation can be more smoothly performed.

(9-7) The tip of the needle 610 is exposed from the outer cylinder 620 by sliding the outer cylinder 620 relative to the needle 610. Since the outer cylinder 620 can be easily slid to expose the tip of the needle 610, the graft can be quickly placed into the target region after the graft is collected into the needle 610. Therefore, deterioration of the graft can be suppressed.

### [Other Modifications]

The ninth embodiment and the modifications thereof can be modified and implemented as described below.
- The inner diameter of the outer cylinder 620 may not necessarily be constant. For example, the inner diameter of the tip of the outer cylinder 620 may gradually increase toward the end face 622 within a range at which a suction force can be maintained. With this configuration, the opening 621 can be increased in size so that the graft can easily enter the opening 621.

Alternatively, as shown in Fig. 77, the inner diameter of the outer cylinder 620 may vary such that the inner diameter of a portion of the outer cylinder 620 protruding from the end face 612 on the tip of the needle 610 in collection of the graft corresponds to the inner diameter of the needle 610, whereas the inner diameter of a portion surrounding the needle 610 in collection of the graft is larger than or equal to the outer diameter of the needle 610. In other words, the inner peripheral surface of the outer cylinder 620 has a stepped portion 23 that follows the end face 612 of the needle 610 when attached to the needle 610 in collection of the graft. In Fig. 77, the end face 612 of the needle 610 and the stepped portion 23 are illustrated as being separated from each other for ease of understanding. However, in collection of the graft, the end face 612 and the stepped portion 23 are in contact with each other. With this configuration, the diameter of the flow channel through which the graft flows is constant from the outer cylinder 620 to the needle 610 without having a stepped portion at the end face 612. Accordingly, the graft is prevented from being caught by the edge of the end face 612. The outer shape of the outer cylinder 620 may be constant, or may vary following the inner diameter.

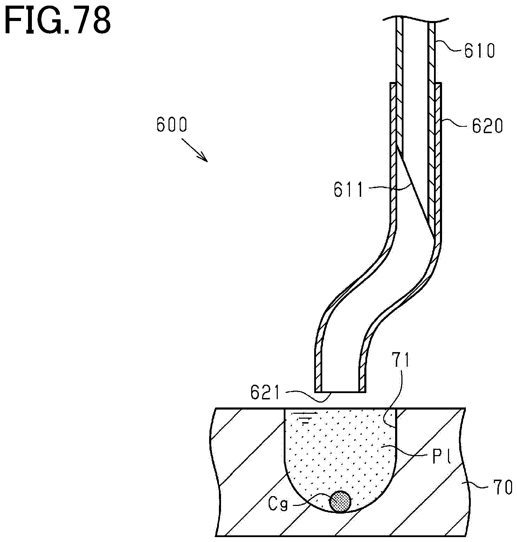

- The end face 622 of the outer cylinder 620 may be inclined from the extending direction, that is, the length Lt of the opening 621 in the extending direction may not necessarily be 0. As long as the length Lt of the opening 621 is smaller than the length Ln of the opening 611 of the needle 610, the tubular portion into which the graft is drawn in collection of the graft can be brought closer to the graft compared with the case where no outer cylinder 620 is provided.
- At least part of the outer cylinder 620 may be elastically deformable or plasticity deformable. Further, the outer cylinder 620 may have both the elastically deformable portion and the plasticity deformable portion. For example, as shown in Fig. 78, when a deformable portion is included in a portion of the outer cylinder 620 which protrudes from the tip of the needle 610 in collection of the graft, the outer cylinder 620 can be deformed so that the tip of the outer cylinder 620 can be brought close to the graft Cg. Therefore, since the degree of freedom of the positional relationship between the needle 610 and the graft in collection of the graft is improved, the graft can be easily collected.
- As long as the needle 610 includes the opening 611 at the tip, the opening 611 functioning as an inlet port and an outlet port for the graft, the other structures may be different from the above embodiments. For example, the space inside the needle 610 may extend in a direction inclined from the extending direction of the needle 610. Further, the tubular members such as the needle 610 and the outer cylinder 620 are not limited to a structure having a hollow cylindrical shape as long as they have openings at the tip and the proximal end, which communicate with each other via a space inside the tube. As long as the length Lt of the opening 621 of the outer cylinder 620 in the extending direction is smaller than the length Ln of the opening 611 of the needle 610, the outer shape of the tubular members and the shape of the space defined in the tubular members may be, but is not limited to, a cylindrical, conical, or prismatic shape. Further, the needle 610 may have a structure that retains the graft in a region from an intermediate position in the extending direction of the internal space to the tip. For example, the graft can be prevented from flowing from a predetermined position in the internal space toward the proximal end by changing the cross-sectional area of the internal space or providing a filter or a mesh-like structure.
- Instead of the outer cylinder 620, a tubular member may be disposed inside the needle 610, and the graft may be aspirated with the tip of the tubular member protruding from the tip of the needle 610, that is, the tip of the tubular member protruding from the opening 611. The graft is held at the tip of the tubular member, closing the opening at the tip, or may be drawn into the tubular member and held in the tubular member. Then, as the tubular member is displaced relative to the needle 610, the graft together with the tip of the tubular member is retracted into the needle 610. With this configuration as well, as long as the length of the opening on the tip of the tubular member in the extending direction is smaller than the length of the opening 611 of the needle 610, the tubular portion into which the graft is drawn in collection of the graft can be brought close to the graft.

### [Examples]

The cell transplantation device of the ninth embodiment will be described below by using specific examples.

### <Example 1>

A 96-well plate (manufactured by Sumitomo Bakelite Co., Ltd.: PrimeSurface) was provided. In each well of the plate, 200 µL of pure water, and a plastic bead with a 210 µm diameter as a model of a group of cells were placed. A cell transplantation device was provided, which includes a 25G injection needle as a needle and a transparent tube made of Teflon as an outer cylinder. The outer cylinder was slidable toward the proximal end of the needle.

A plastic bead settled in one well in the 96-well plate was collected by using the cell transplantation device. For collecting the bead, a syringe pump was used to apply suction at a rate of 1000 µL/min and a volume of 150 µL. Collection of the plastic bead was performed 10 times. In all of the 10 cases, the plastic bead was collected into the needle. After the outer cylinder was removed from the needle, collection of the plastic bead was performed in the same manner. In all of the 10 cases, the plastic bead was not collected into the needle.

### <Example 2>

A 96-well plate (manufactured by Sumitomo Bakelite Co., Ltd.: PrimeSurface) was provided. In each well of the plate, 200 µL of follicle dermal papilla cell growth medium (manufactured by PromoCell) was placed, and 4 × 10³ cells of human follicle dermal papilla cells (manufactured by PromoCell) were seeded. The cells were cultured under an atmosphere of 5%CO₂ and 37°C for 72 hours. It was found that a spheroid of approximately 200 µm diameter had been formed after the culture.

A spheroid settled in one well in the 96-well plate was collected by using the same cell transplantation device as that in Example 1. For collecting the spheroid, a syringe pump was used to apply suction at a rate of 500 µL/min and a volume of 150 µL. Collection of the spheroid was performed 10 times. In all of the 10 cases, the spheroid was collected into the needle. After the outer cylinder was removed from the needle, collection of the spheroid was performed in the same manner. In all of the 10 cases, the spheroid was not collected into the needle.

### <Example 3>

In the cell transplantation device used in Examples 1 and 2, the outer cylinder was displaced toward the proximal end of the needle to expose the tip of the needle. The length of the exposed tip of the needle was 5 mm. The needle was inserted into an excised nude mouse skin (manufactured by Charles River Laboratories Japan, Inc.), and the needle was advanced to the inside of the skin.

### [Supplementary Notes]

The measure for solving the foregoing problems embraces the following items as technical ideas derived from the ninth embodiment and the modification thereof.

### (Item 41)

A biological transfer device configured to place an object into a living body, the transfer device including:
a needle having a tubular shape extending in one direction, the needle being configured to collect the object into the needle from an opening located at a tip of the needle; and
a tubular member located outside or inside the needle, the tubular member being configured to be displaceable along an extending direction of the needle from a state in which a tip of the tubular member protrudes from the tip of the needle, wherein
the tubular member has an opening located at the tip in the extending direction of the needle, the opening having a length which is smaller than a length of the opening located at the tip of the needle.

### (Item 42)

The biological transfer device according to the item 41, wherein an end face on the tip of the tubular member is a flat surface perpendicular to the extending direction of the needle.

### (Item 43)

The biological transfer device according to the item 41, wherein an end face on the tip of the tubular member has irregularities on a surface perpendicular to the extending direction of the needle.

### (Item 44)

The biological transfer device according to any one of the items 41 to 43, wherein
an end face on the tip of the needle includes a slant portion inclined radially inward toward the proximal end of the needle, and
the slant portion includes the proximal end of the end face in the extending direction of the needle.

### (Item 45)

The biological transfer device according to any one of the items 41 to 44, wherein at least one of an inner peripheral surface of the needle and an inner peripheral surface of the tubular member is made of a material that reduces adhesion of the object.

### (Item 46)

The biological transfer device according to any one of the items 41 to 45, wherein the tubular member is located outside the needle and configured to be slidable along the outer peripheral surface of the needle.

### (Item 47)

A member for a biological transfer device, the member being configured to be attached to a device which includes a needle having a tubular shape extending in one direction and configured to collect an object into the needle from an opening located at a tip of the needle and place the object into a living body, the member including
a tubular member fitted outside the needle and configured to be slidable along an outer peripheral surface of the needle, wherein
the tubular member has an opening located at the tip in the extending direction of the needle, the opening having a length which is smaller than a length of the opening located at the tip of the needle.

### (Item 48)

A biological transfer unit including: a tray having one or more placement regions, which are regions where objects are to be placed; and
a biological transfer device configured to transfer the objects from the tray into a living body,
the biological transfer device including:
   a needle having a tubular shape extending in one direction, the needle being configured to collect the objects located in the placement regions into the needle from an opening located at a tip of the needle; and
   a tubular member located outside or inside the needle, the tubular member being configured to be displaceable along an extending direction of the needle from a state in which a tip of the tubular member protrudes from the tip of the needle, wherein
   the needle is assigned to each of the placement regions, and
   the tubular member has an opening located at the tip in the extending direction of the needle, the opening having a length which is smaller than a length of the opening located at the tip of the needle.

### (Item 49)

A biological transfer kit including: a tray having a plurality of placement regions, which are regions where objects are to be placed; and
a member configured to be attached to a device which includes a needle having a tubular shape extending in one direction and configured to collect the objects located in the placement regions into the needle from an opening located at a tip of the needle and transfer the objects from the tray into a living body, wherein
the member includes a plurality of tubular members fitted outside the needle and configured to be slidable along an outer peripheral surface of the needle, and each of the tubular members has an opening located at the tip in the extending direction of the needle, the opening having a length which is smaller than a length of the opening located at the tip of the needle, and
each of the tubular member is assigned to each of the placement regions.

### (Modifications)

The first to ninth embodiments and the modifications thereof can be modified and implemented as described below.
- A group of cells to be transplanted may not necessarily be a group of cells that contributes to hair growth or hair restoration, and may be a group of cells that exhibits desired effects when placed in a living body. For example, the group of cells to be transplanted may be a group of cells that exhibits effects in cosmetic use such as elimination of wrinkles or improvement in moisturizing state in the skin.
- The issue that smooth transfer of an object into a living body is difficult is not limited to cells, but also applies to a case where solid substances such as drugs are transferred and placed into a living body. That is, in a method in which an incision is made in a living body with a scalpel, and then objects are picked up one by one with tweezers or the like and transferred into a living body, it is required to exchange the tools used and frequently repeat operations of transferring the objects, which may hinder smooth transfer of the objects.

The cell transplantation device according to the first to ninth embodiments and the modifications thereof are merely examples of the biological transfer device, and the objects transferred into a living body by the biological transfer device are not limited to groups of cells, and may be solid substances such as drugs.

## Claims

1. A cell transplantation device configured to place a graft containing cells into a target region in a living body, the cell transplantation device comprising:
a needle extending in one direction, the needle having a flow channel defined therein and an opening at a tip of the needle; and
a filter unit made of a fibrous member, the filter unit being disposed at an intermediate position in the flow channel and configured to retain the graft between the filter unit and the opening when a liquid material containing the graft enters the flow channel through the opening.

2. The cell transplantation device according to claim 1, wherein the filter unit has a mesh form in which a plurality of fibrous members intersect each other.

3. The cell transplantation device according to claim 1 or 2, wherein
the needle includes
a first unit located on a same side of the filter unit as that on which the tip of the needle is located and
a second unit located on a same side of the filter unit as that on which a proximal end of the needle is located,
the second unit has a flow channel cross-sectional area which is larger than a flow channel cross-sectional area of the first unit, and,
in plan view in an extending direction of the needle, a portion of the proximal end of the first unit where the fibrous member overlaps the flow channel has an area of 20% or more and 85% or less of the flow channel cross-sectional area of the first unit.

4. The cell transplantation device according to any one of claims 1 to 3, wherein
the needle includes
a first tube including the tip and
a second tube connected to the proximal end of the first tube,
the filter unit is located near a position where the first tube and the second tube are connected to each other, and
the second tube has a flow channel cross-sectional area which is larger than a flow channel cross-sectional area of the first tube.

5. A cell transplantation device configured to place a graft containing cells into a target region in a living body, the cell transplantation device comprising:
a needle extending in one direction, the needle having a flow channel defined therein and an opening at a tip of the needle, wherein
a region surrounded by a side wall of the flow channel from an intermediate position in the flow channel to the opening serves as a storage portion that stores a liquid material entering through the opening while allowing the graft contained in the liquid material to be retained in the region, and
a length of the storage portion in the extending direction of the needle is larger than a maximum width of the storage portion in a direction perpendicular to the extending direction of the needle.

6. The cell transplantation device according to claim 5, wherein the storage portion has a volume of 0.001 µL or more and 1 µL or less.

7. A cell transplantation device configured to place a graft containing cells into a target region in a living body, the cell transplantation device comprising
a plurality of needles extending in a first direction, wherein
the plurality of needles include
at least one needle having a tip whose position is not aligned with that of others in the first direction, and
a plurality of transplantation needles each having an opening in a tip region, the opening being provided as an inlet port through which the graft is taken into the needle and an outlet port through which the graft is ejected toward the target region.

8. The cell transplantation device according to claim 7, wherein
the plurality of needles are arranged at positions not aligned with each other in a second direction perpendicular to the first direction, and, among the plurality of needles, a tip of the needle located at a center in the second direction protrudes more than a tip of any other of the needles.

9. The cell transplantation device according to any one of claims 1 to 8, wherein the cell transplantation device is used to place the graft, which is a cell aggregate containing skin-derived stem cells, into the target region.

10. A cell transplantation system comprising:
a cell transplantation device configured to place a graft containing cells into a target region in a living body, the cell transplantation device including a needle extending in one direction, the needle being configured to collect the graft through an opening formed at a tip of the needle and store the graft in the needle; and
a detector that detects when the graft is held by the needle.

11. The cell transplantation system according to claim 10, wherein
the detector detects when the graft is held by the needle as an electrical change or a pressure change in the needle due to holding the graft.

12. The cell transplantation system according to claim 10 or 11, wherein
the cell transplantation device includes a first conductor and a second conductor electrically connected to the detector,
the needle includes a holding portion configured to hold the graft, the holding portion being configured to prevent the graft contained in a liquid material from flowing toward a proximal end of the needle,
the holding portion includes an electrically conductive portion having electrical conductivity,
the first conductor is in contact with the electrically conductive portion of the holding portion,
the second conductor is in contact with the electrically conductive portion when the graft is held by the holding portion, and
the detector detects when the graft is held by detecting electrical conduction between the first conductor and the second conductor.
